# EUROPEAN PATENT APPLICATION

(11) **EP 4 696 680 A1**
(43) Date of publication of application: **18.02.2026**
(21) Application number: 24194788.6
(22) Date of filing: 15.08.2024
(51) Int. Cl.: C07D 255/02, C07B 59/00, C07D 257/08, C07D 403/12, C07F 1/08, C07F 5/00, A61K 51/04

(54) **DUAL-FUNCTIONAL TETRAZINE COMPOUNDS FOR VERSATILE RADIOLABELING WITH RADIOMETALS AND RADIOHALOGENS FOR DIAGNOSTIC AND THERAPEUTIC APPLICATIONS**

(71) Applicant: Tetrakit Technologies ApS, 2200 Copenhagen N (DK)
(72) Inventor: POULIE, Christian Bernard Matthijs, 2200 Copenhagen N (DK); MARTIN, Marcel, 2200 Copenhagen N (DK); BATTISTI, Umberto Maria, 2200 Copenhagen N (DK); VÁZQUEZ, Rocío García, 2200 Copenhagen N (DK)
(74) Representative: Budde Schou A/S

(57) **Abstract**

The present invention provides dual-functional tetrazine compounds labeling with either metals including radiometals or with halogens including radiohalogens suitable for therapeutic or diagnostic purposes. Alternatively, the tetrazines can be used as precursors for labeling with a radiohalogen when the compound comprises a leaving group. The tetrazines are specifically engineered to facilitate efficient and stable incorporation of various radionuclides. By integrating a chelator group for radiometals and a leaving group for radiohalogens, these tetrazines offer a flexible and interchangeable structure that facilitates the development of radiopharmaceuticals. The tetrazines are for use in pretargeting therapy, diagnostics, imaging and therenostics. Use of the precursors for labeling with radiohalogen and use of the tetrazines as reagent in tetrazine ligation is moreover disclosed.

## Description

### Field of the Invention

The present invention applies to the development of dual-functional tetrazine compounds designed for versatile radiolabeling with both radiometals and radiohalogens. These compounds are specifically engineered to facilitate efficient and stable incorporation of various radionuclides. By integrating a chelator group for radiometals and a leaving group for radiohalogens, these tetrazines offer a flexible and interchangeable structure that facilitates the development of radiopharmaceuticals. They enhance the practicality and versatility of radiopharmaceuticals used in diagnostic imaging and targeted radionuclide therapy, thus contributing to improved patient outcomes and expanding the capabilities of theranostic applications, particularly when certain radionuclides are not feasible or accessible at specific sites.

### Background of the Invention

Click chemistry involves reactions that are fast, simple, versatile, chemoselective, and give high product yields. These reactions find applications across various research fields, including materials science, polymer chemistry, and pharmaceutical sciences. In nuclear medicine, the potential of click chemistry has been particularly evident. Labeled targeting vectors based on click chemistry are extensively used for both imaging and therapeutic purposes. The type of radionuclide used in these vectors determines their application. A notable example of click chemistry in this field is tetrazine ligation. This specific and rapid reaction occurs between 1,2,4,5-tetrazines (Tz) and trans-cyclooctene (TCO), making it ideal for synthon-based labeling due to its high second-order rate constants and specificity. The reaction begins with an inverse electron-demand Diels-Alder reaction, followed by a retro-Diels-Alder reaction driven by the expulsion of nitrogen (N2). Tetrazine ligation is among the fastest known chemical ligations, with second-order rate constants reaching up to 10⁶ M⁻¹ s⁻¹ in acetonitrile at 25°C. This speed and specificity distinguish it from other known click reactions, such as the Staudinger ligation (0.003 M⁻¹ s⁻¹) and strain-promoted azide-alkyne cycloaddition (SPAAC) (0.1 M⁻¹ s⁻¹). Due to these qualities, tetrazine ligation is exceptionally well-suited for synthon-based labeling, enhancing the efficiency and applicability of radiopharmaceuticals in nuclear medicine. However, conventional Tz-TCO ligation lacks regio-specificity and results in complex isomeric mixtures, unsuitable for pharmaceutical applications due to variable pharmacokinetics and potential toxicological effects. A method described in WO2023/170174 utilizes tetrazine ligation click chemistry to produce a single isomeric compound, enhancing the practical use of these radiolabeled vectors.

Radiopharmaceuticals are gradually more used in theranostics, particularly in oncology, for diagnostic imaging and targeted radionuclide therapy. Targeted radionuclide therapy effectively treats cancer by confining the delivered dose to tumors, providing an advantage over external beam radiotherapy, especially for micrometastatic disease. Theranostics combines diagnostic imaging and targeted therapy, a powerful concept in personalized medicine aiding patient selection, dose-finding, and therapy response monitoring. A theranostic pair consists of two radionuclides interchangeable without altering the radiopharmaceutical's pharmacokinetics, enabling applications in both diagnostic imaging and radionuclide therapy.

The primary diagnostic imaging methods are PET (Positron Emission Tomography) and SPECT (Single Photon Emission Computed Tomography). PET, the gold standard in nuclear imaging, uses positron-emitting radionuclides such as fluorine-18 (¹⁸F) and gallium-68 (⁶⁸Ga) to produce high-resolution images and quantification. SPECT, more common in cardiology and bone scans, uses gamma-emitting radionuclides like iodine-123 (¹²³I) or technetium-99m (^{99m}Tc). Both methods rely on radiolabeled vectors targeting specific diseases for diagnosis or monitor the disease progression.

PET provides high-resolution and sensitivity, essential for detecting small metastases. Innovations like total-body PET have increased sensitivity and detection capabilities, further strengthening PET's position in modern clinical practice. PET uses radionuclides that emit positrons that annihilate with electrons to emit two gamma photons which are detected by the PET scanner enabling the imaging. Fluorine-18 is one of the most commonly used PET isotopes, with a half-life of approximately 110 minutes. This duration is long enough for complex synthesis and imaging procedures, yet short enough to minimize patient radiation exposure. It is produced in cyclotrons and used in various radiotracers, notably FDG, for metabolic imaging in oncology, neurology, and cardiology.

Another key PET isotope is Gallium-68, with a half-life of 68 minutes. It is produced from a germanium-68/gallium-68 generator, making it readily available in clinical settings. Gallium-68 is particularly useful in tracers like Ga-68 DOTATATE and Ga-68 PSMA, which are effective in diagnosing neuroendocrine tumors and prostate cancer, respectively. Copper-64, with its 12.7-hour half-life, is suitable for both imaging and radiotherapy. It decays by positron emission and beta-minus decay, making it versatile for various clinical applications, such as imaging hypoxia in tumors using ⁶⁴Cu-ATSM. Rubidium-82, with a short half-life of 1.27 minutes, is primarily used in cardiac PET imaging to assess myocardial perfusion, helping diagnose coronary artery disease. Carbon-11, which has a half-life of 20.4 minutes, is versatile in labeling various organic compounds for neuroreceptor studies, despite its short half-life.

SPECT imaging relies on gamma-emitting radionuclides to create high-resolution images of the body's internal structures. Iodine-123 is a widely used SPECT isotope, favored for its suitable half-life of 13.2 hours and gamma photon energy of 159 keV. It is primarily used for imaging thyroid disease due to its intrinsic accumulation in the thyroid gland. Technetium-99m, another popular SPECT isotope, is known for its optimal gamma emission and availability from a generator system. With a half-life of 6 hours, ^{99m}Tc is extensively used in various diagnostic procedures, including cardiac imaging and bone scans. Iodine-131, with its longer half-life of 8 days, is used in both imaging and therapy, particularly for thyroid cancer treatment. Iodine-123 forms a theranostic pair with iodine-131, combining diagnostic and therapeutic capabilities. Astatine-211 is primarily a therapeutic nuclide that emits alpha particles, but it also produces X-rays that can be imaged with a gamma camera or SPECT scanner, allowing for clinical assessment of its distribution within the patient.

On the other hand, Targeted Radionuclide Therapy (TRT) use beta-emitters, Auger electron emitters, or alpha-emitters radionuclides, each offering unique advantages. Beta-emitters such as Yttrium-90 (⁹⁰Y), Lutetium-177 (¹⁷⁷Lu), and lodine-131 (¹³¹I) decay by emitting high-energy electrons (beta particles), which travel significant distances in tissue. This property makes them effective for treating medium-sized tumors, but less so for micrometastases, as a considerable fraction of the irradiation dose can be absorbed by surrounding healthy cells. For instance, ¹⁷⁷Lu is used in peptide receptor radionuclide therapy (PRRT) for treating neuroendocrine tumors. In PRRT, radiolabeled somatostatin analogs bind to somatostatin receptors overexpressed on neuroendocrine tumors, delivering targeted radiation to the cancer cells. Iodine-131 is used in the treatment of thyroid cancer, exploiting the thyroid gland's natural uptake of iodine to deliver therapeutic doses of radiation.

Alpha-emitters, including Actinium-225 (²²⁵Ac), Astatine-211 (²¹¹At), and Lead-212 (²¹²Pb), emit alpha particles with very short tracks in tissue, delivering their energy to only a few neighboring cells. This characteristic makes alpha-emitters particularly effective for treating micrometastases and small clusters of cancer cells with minimal damage to surrounding healthy tissue. Actinium-225 is being explored for use in targeted alpha therapy (TAT) for leukemia and prostate cancer. In prostate cancer, ²²⁵Ac labeled with prostate-specific membrane antigen (PSMA) targets PSMA-expressing cells, providing highly focused treatment. Astatine-211 is being investigated for its potential in treating brain tumors, such as gliomas, where its ability to deliver high doses of radiation precisely to cancer cells while sparing adjacent brain tissue is highly advantageous.

Auger electron emitters, such as lodine-123 (¹²³I) and lodine-125 (¹²⁵I), deliver extremely localized radiation therapy due to the very short path lengths of Auger electrons. This makes them ideal for treating microscopic disease and targeting individual cancer cells. Iodine-125 is used in brachytherapy for prostate cancer, where radioactive seeds are implanted directly into the prostate gland, allowing continuous low-dose radiation to be delivered over several months, effectively treating the cancer while minimizing exposure to surrounding tissues. Iodine-123, used in diagnostic imaging, also has therapeutic potential due to its emission of Auger electrons, which can be exploited for treating residual or microscopic disease post-surgery.

Introducing radionuclides into molecules poses practical challenges, limiting radionuclide-based diagnostics and therapy. Radiometals, such as Gallium-68, Copper-64, and Zirconium-89, are typically introduced into molecules through chelation. This process involves using chelator groups that form coordinate bonds with the metal ions, ensuring stable attachment. The chelation reaction usually involves mixing the radiometal ions with a precursor molecule containing the chelator group. This mixture is then heated to facilitate the chelation process. Common chelators include DOTA (1,4,7,10-tetraazacyclododecane-1,4,7,10-tetraacetic acid) and NOTA (1,4,7-triazacyclononane-1,4,7-triacetic acid), which form strong, stable complexes with radiometals. This method ensures that the radiometal remains securely bound to the molecule, maintaining the integrity and functionality of the radiopharmaceutical. For example, Gallium-68 is often incorporated into molecules using the DOTA chelator, resulting in compounds like Ga-68 DOTATATE, which targets somatostatin receptors in neuroendocrine tumors.

Radiohalogens, such as lodine-123, Fluorine-18, and Astatine-211, are incorporated into molecules through covalent bonding, typically via nucleophilic substitution reactions. This process involves the replacement of a leaving group in the molecule with the radiohalogen. For instance, in the case of iodine, an electrophilic substitution reaction can be employed, where radioactive iodide is oxidized to a positively charged iodine species that then replaces a leaving group, usually a stannyl group, in an aromatic substitution reaction. This reaction is conducted at room temperature and often yields high efficiency. Another method for introducing iodine into molecules is isotopic exchange, where a nonradioactive iodine atom in the molecule is replaced by a radioactive one. This process usually requires elevated temperatures and acidic conditions, often catalyzed by copper. Astatine-211, being a halogen, can also be attached to aryl rings forming astatoaryl moieties. However, due to its unique chemistry, astatine cannot be stably coupled to tyrosine residues of proteins like iodine can. Instead, it forms bonds with sulfhydryl groups of cysteine, necessitating the synthesis of dedicated precursors with suitable leaving groups like trialkylstannyl. The labeling reaction for astatine often uses oxidation agents such as chloramine-T or N-chlorosuccinimide, which can degrade biomolecules used as targeting vectors.

Overall, the reactions to incorporate chelators usually needs a purification process and heating, which can degrade temperature-sensitive vectors. In contrast, radiohalogens form covalent bonds with targeting vectors, often under harsh and lengthy conditions incompatible with peptides and other sensitive biomolecules. To overcome these challenges, synthon-based methods have been developed, allowing radiolabeling under mild conditions without exposing the vector to harsh environments. Tetrazine ligation exemplifies this approach and the dual-functional tetrazine compounds facilitate these radiolabeling processes, providing a versatile platform for creating advanced radiopharmaceuticals.

Prostate cancer, one of the most common cancers among men, demands precise diagnostic and therapeutic approaches for effective management. While PSMA (Prostate-Specific Membrane Antigen) targeting has been extensively studied, exploring additional molecular targets can further enhance the detection and treatment of prostate cancer. One such promising target is Fibroblast Activation Protein (FAP), which is overexpressed in cancer-associated fibroblasts (CAFs) within the tumor microenvironment. FAP inhibitors (FAPi) represent a significant development in cancer theranostics, including prostate cancer. Beyond cancer, FAP expression is also significant in several non-cancerous conditions such as fibrosis, arthritis, atherosclerosis, and inflammatory diseases like spondyloarthritis. This broad range of expression underscores FAP's potential as a target for diagnostic and therapeutic applications across various pathological conditions.

Click chemistry, particularly tetrazine ligation, offers significant advantages in radiolabeling for these applications. The rapid and specific reaction between tetrazines and trans-cyclooctene (TCO) allows for efficient and versatile radiolabeling of various targeting vectors, including antibodies, peptides, and small molecules. This adaptability enables the development of customized radiopharmaceuticals tailored to different biomarkers, improving both diagnostic imaging and targeted radionuclide therapy.

On the other hand, tetrazine ligation has gained prominence for its utility in pretargeting strategies due to its rapid reaction kinetics and high specificity. This method involves a two-step bioorthogonal process. Initially, a substrate modified with a trans-cyclooctene (TCO) functional group is administrated. Subsequently, a radiolabeled tetrazine (Tz) probe is injected. The tetrazine rapidly reacts with the TCO-modified substrate through an inverse electron demand Diels-Alder (IEDDA) reaction, forming a stable covalent bond. This reaction is highly selective, occurs rapidly in vivo, and is compatible with a variety of biological conditions.

Pretargeting using the TCO-Tz system offers several advantages. By separating the slow targeting phase from the imaging or therapeutic step, this strategy allows the use of radionuclides with short half-lives, enhancing imaging contrast and reducing radiation doses to healthy tissues. The tetrazine ligation method has been shown to significantly improve target-to-background ratios in radiopharmaceutical applications, making it a powerful tool for both diagnostic imaging and targeted radionuclide therapy.

For instance, in preclinical studies, radiolabeled tetrazine probes have been successfully used in combination with TCO-modified antibodies to target tumors, achieving high activity concentrations in target tissues with minimal off-target effects. This has been particularly effective in imaging and treating various cancers, including colorectal cancer and metastatic prostate cancer. Such strategies have demonstrated that tetrazine ligation not only enhances the efficiency of radiolabeling but also improves the therapeutic and diagnostic capabilities of radiopharmaceuticals by providing high specificity and reduced toxicity.

The present invention provides dual-functional tetrazine compounds, further enhancing the pretargeting approach. The tetrazines presented herein allows for easy swapping of radionuclides without altering the compound's final structure. This makes the radiolabeling process more efficient and adaptable to various clinical needs. The ability to efficiently label with both radiometals and radiohalogens vectors under mild conditions significantly broadens the application scope of tetrazine ligation in pretargeted strategies. In addition, the tetrazines provided herein may be used as precursors for providing tetrazines labeled with radiohalogens. Consequently, the tetrazines disclosed herein advance the field of nuclear medicine, offering more precise, effective, and versatile diagnostic and therapeutic options.

### Summary of the invention

The present invention relates in a first aspect to 1 ,2,4,5-tetrazine compound of formula (I): 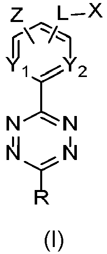 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is a moiety selected from:
   Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At or ²¹¹At;
X is a chelator selected from:
   1,4,7,10-tetraazacyclododecane-*N*,*N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N*,*N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(tert-butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N'*-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
and wherein X is optionally labeled with a metal selected from:
   ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹TI, ²⁰³TI, ²⁰⁵TI, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.

A second aspect of the invention relates to 1,2,4,5-tetrazines of formula (I): 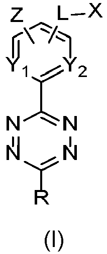 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
X is a chelator selected from:
   1,4,7,10-tetraazacyclododecane-*N*,*N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N*,*N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((12-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(tert-butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is a radionuclide halogen selected from: ¹⁸F, ⁷⁶Br, ⁷⁷Br, ^{80m}Br, ¹²³I, ¹²⁴I, ¹²⁵I, ¹²⁶I, ¹³¹I, ²¹⁰At,²¹¹At, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y ⁹⁰Zr, ⁹¹Zr, ⁹²Zr, ⁹⁴Zr, ¹⁰³Rh, ¹⁹²Pt ¹⁹⁴Pt, ¹⁹⁵Pt, ¹⁹⁶Pt ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³TI, ²⁰⁵TI, ²⁰⁶Pb, ²⁰⁷Pb, ²⁰⁸Pb, ²⁰⁹Bi;
for use in pre-targeting based therapy, diagnostics, imaging, or theranostics.

A third aspect of the present invention relates to 1,2,4,5-tetrazines of formula (I): 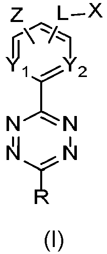 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
X is a chelator selected from:
   1,4,7,10-tetraazacyclododecane-*N*,*N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N*,*N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N'*-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is selected from: ¹⁹F, ⁷⁹Br, ⁸¹Br, ¹²⁷I, and
X is labeled with a radionuclide metal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In ¹¹³In, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹TI, ²⁰³Pb, ²¹²Pb, ²⁰⁹Bi, ²¹²Bi, ²¹³Bi, ²²³Ra, ²²⁵Ac, ²²⁷Th, ²³²Th,
for use in pre-targeting based therapy, diagnostics, imaging, or theranostics.

A fourth aspect of the present invention relates to the use of compounds of formula (I): 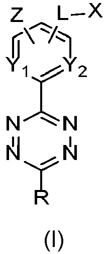 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is selected from: Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³TI, ²⁰⁵TI, ²⁰⁶Pb, ²⁰⁷Pb, ²⁰⁸Pb, ²⁰⁹Bi,
as a precursor for radiolabeling.

A fifth aspect of the present invention relates to the use of a 1,2,4,5-tetrazine compound of formula (I): 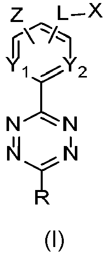 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is a moiety selected from:
   ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At or ²¹¹At;
X is a chelator selected from:
   1,4,7,10-tetraazacyclododecane-*N*,*N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N*,*N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(tert-butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);

and wherein X is optionally labeled with a metal selected from:
   ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹TI, ²⁰³TI, ²⁰⁵TI, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.
as reagent in tetrazine ligation.

### Brief description of the drawings

Figure 1A and 1B is a scheme showing the synthesis of compound 28.
Figure 2 is a scheme showing the synthesis of compound 33.
Figure 3 is a scheme showing the synthesis of compound 40.
Figure 4 is a scheme showing the synthesis of compound 45.
Figure 5A and 5B is a scheme showing the synthesis of compound 50.
Figure 6A and 6B is a scheme showing the synthesis of compound 56.
Figure 7A, 7B, and 7C is a scheme showing the synthesis of compound 60.
Figure 8A, 8B, and 8C is a scheme showing the synthesis of compound 64.
Figure 9 is a scheme showing the synthesis of compound 65.

### Detailed description of the invention

This invention provides tetrazines which can be radiolabeled with any radionuclide either for diagnosis or therapy approaches. These tetrazines are unique in that they contain a halogen or a leaving group that facilitates the labeling of radiohalogens and a chelator that allows for the optional incorporation of metals, including radiometals. This dual functionality enables the final structure to be interchangeable, allowing for the easy swapping of radionuclides without altering the final structure of the compounds. This flexibility is particularly beneficial during drug development.

The various PET, SPECT and therapeutic isotopes that are available in the art offer unique advantages for specific clinical applications, enhancing the versatility and efficacy of nuclear imaging in diagnosing and treating various medical conditions. The dual-functional tetrazine compounds described in this invention can efficiently incorporate these isotopes, providing unmatched versatility and practicality for diagnostic and therapeutic applications.

The novel tetrazine compound of this invention have been designed with the option of applying one of two key functional groups depending on the final use of the compound: Leaving group for radiohalogens: The tetrazine may include a leaving group that enables the introduction of radiohalogens. This group facilitates nucleophilic substitution reactions where radiohalogens such as iodine-123, iodine-124, and astatine-211 can be incorporated into the molecule. The leaving group on the tetrazine molecule enables the incorporation of radiohalogens under mild conditions, addressing issues related to the harsh conditions typically required for covalent bonding. This makes the tetrazine compounds compatible with a wide range of vectors. When a compound with no radioalogen is desired, a stable halogen is present instead of a radiohalogen or instead of a leaving group.

Chelator group for radiometals: Additionally, the tetrazine has a chelator group attached to the phenyl ring, allowing the formation of coordinate bonds with metals, including radiometals such as gallium-68, lutetium-177, and copper-64. The chelator ensures stable binding of the metal / radiometal, which is essential for maintaining the integrity and functionality of the radiolabeled compound. Furthermore, challenges such as degradation of temperature-sensitive vectors during the chelation process are overcome. This stability is crucial for the development of effective radiopharmaceuticals. When a compound with no radiometal is desired, the chelator may be unlabeled or the chelator may be labeled with a stable metal.

Radiopharmaceuticals are increasingly used in theranostics, particularly in oncology, for both diagnostic imaging and targeted radionuclide therapy. Positron Emission Tomography (PET) and Single Photon Emission Computed Tomography (SPECT) are the primary imaging modalities that benefit from these radiolabeled tetrazine compounds.

PET Imaging: PET imaging benefits from radionuclides like fluorine-18 and gallium-68, which are known for their high resolution and sensitivity. The use of tetrazine compounds allows for the efficient labeling with these radionuclides, providing high-quality images for diagnosis and treatment monitoring.

SPECT Imaging: SPECT imaging utilizes radionuclides such as iodine-123 and technetium-99m. The tetrazine's leaving group facilitates the incorporation of these radiohalogens, enabling their use in high-resolution imaging.

Targeted Radionuclide Therapy (TRT) can utilize beta-emitters, Auger electron emitters, or alpha-emitters, each suited for different therapeutic purposes. The tetrazine compounds can be labeled with radionuclides like lutetium-177 (beta-emitter), iodine-123 (Auger electron emitter), and astatine-211 (alpha-emitter), providing a versatile platform for various therapeutic applications.

The tetrazine compounds provided herein also enable a pretargeting strategy, which involves a two-step process using bioorthogonal chemistry. Initially, a substrate modified with a bioorthogonal functional group is introduced to the patient. A probe containing the complementary functional group is then introduced, reacting and labeling the substrate. This approach improves target-to-background ratios and reduces radiation exposure to healthy tissue, enhancing the specificity and efficiency of imaging and therapeutic processes.

The tetrazine compounds described in this invention represent a significant advancement in the field of radiopharmaceuticals. Their dual functionality, with a leaving group for radiohalogen labeling and a chelator for radiometal incorporation, provides unmatched versatility and practicality for diagnostic and therapeutic applications. This invention has the potential to greatly enhance the development and use of radiopharmaceuticals in personalized medicine.

In a first aspect, the present invention provides a 1,2,4,5-tetrazine compound of formula (I): 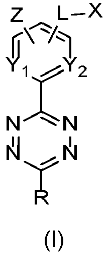 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is a moiety selected from:
   Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I, ¹²⁵I, ¹²⁶I, ¹²⁷I, ¹³¹I, ²¹⁰At or ²¹¹At;
X is a chelator selected from:
   1,4,7,10-tetraazacyclododecane-*N*,*N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N*,*N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((I2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(tert-butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N'*-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
and wherein X is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹TI, ²⁰³TI, ²⁰⁵TI, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.

Formula I thus comprises a number of compounds the use of which is dependent on the selections made at position "Z" and at position "X".

At position "Z" the selection is to be made between a halogen - either a stable halogen or a radionuclide halogen - and a leaving group. The stable halogens are ¹⁹F, ⁷⁹Br, ⁸¹Br, and ¹²⁷I. These are selected when the application of the final compound is based on the features of a radiometal. The radiometals are ¹⁸F, ⁷⁶Br, ⁷⁷Br, ^{80m}Br, ¹²³I, ¹²⁴I, ¹²⁵I, ¹²⁶I, ¹³¹I, ²¹⁰At, and ²¹¹At. These are selected when the application of the final compound is based on the features of the radiohalogen. Alternatively, Z is a leaving group. The leaving groups are Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, and Sn(Bu)₃. When a leaving group is selected at position Z, the compound is applicable as a precursor for use in radiolabeling the compound with a radiohalogen.

"X" is a chelator and such structures are suitable for binding / being labeled with a metal ion - either a stable metal or a radiometal. Alternatively, the chelator is unlabled meaning that the structure does not comprise a metal ion. The stable metals are ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³TI, ²⁰⁵TI, ²⁰⁶Pb, ²⁰⁷Pb, ²⁰⁸Pb, and ²⁰⁹Bi. The radiometals are ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y ⁹⁰Y ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In ¹¹³In ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹TI, ²⁰³Pb, ²¹²Pb, ²⁰⁹Bi, ²¹²Bi, ²¹³Bi, ²²³Ra, ²²⁵Ac, ²²⁷Th, ²³²Th.

When the application of the 1,2,4,5-tetrazine of formula (I) is based on the features of a radiohalogen, the chelator is preferably unlabeled or labeled with a stable metal.

Accordingly, in one embodiment of the first aspect, the compound according to formula (I) is a compound wherein Z is a radionuclide halogen selected from: ¹⁸F, ⁷⁶Br, ⁷⁷Br, ^{80m}Br, ¹²³I, ¹²⁴I, ¹²⁵I, ¹²⁶I, ¹³¹I, ²¹⁰At ²¹¹At, and wherein X is unlabeled or labeled with a metal selected from:⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³TI, ²⁰⁵TI, ²⁰⁶Pb, ²⁰⁷Pb, ²⁰⁸Pb, ²⁰⁹Bi.

In a preferred embodiment, the compound according to formula (I) is a compound wherein Z is a radionuclide halogen selected from: ¹²³I, ¹³¹I, ²¹⁰At, ²¹¹At, and wherein X is unlabeled or X is labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re,²⁰³TI,²⁰⁵TI, ²⁰⁶Pb, ²⁰⁷Pb, ²⁰⁸Pb,²⁰⁹Bi.

In the preferred embodiment the compound according to formula (I) includes a radiohalogen selected from ¹²³I, ¹³¹I, ²¹⁰At, or ²¹¹At. These radionuclide halogens are advantageous due to their well-defined nuclear properties, which are particularly beneficial for both diagnostic imaging and therapeutic applications. For diagnostic imaging, ¹²³I is commonly used in single-photon emission computed tomography (SPECT) due to its favorable half-life of approximately 13 hours and gamma emission energy. This allows for high-quality imaging while minimizing radiation exposure to the patient. On the other hand, ¹³¹I, with a longer half-life of about 8 days and dual properties of emitting both beta and gamma radiation, is suitable for both diagnostic and therapeutic purposes. Its gamma emissions facilitate imaging, while the beta emissions provide therapeutic effects, particularly in treating thyroid-related diseases. For therapeutic applications, ²¹⁰At and ²¹¹At, as alpha-emitting radionuclides, are particularly effective in targeted radionuclide therapy due to their high linear energy transfer (LET). This results in significant cytotoxicity localized to targeted cells, thereby reducing damage to surrounding healthy tissues. Specifically, ²¹¹At, with a half-life of approximately 7.2 hours, provides a balance between delivery time and therapeutic efficacy, making it an optimal choice for certain cancers.

Furthermore, the tetrazine compounds designed with these radionuclide halogens demonstrate excellent stability under physiological conditions, which is crucial for maintaining the integrity of the radiopharmaceuticals during transport and administration. The inclusion of these specific halogens facilitates efficient labeling and stable incorporation, enhancing the overall performance and reliability of the radiopharmaceuticals. Additionally, the selected radionuclide halogens are accessible and well-characterized, allowing for streamlined synthesis and scalability in a clinical setting. The use of these isotopes aligns with existing production and regulatory frameworks, thereby facilitating rapid deployment in clinical practice.

In another preferred embodiment, the 1,2,4,5-tetrazine compound of formula (I) comprising a radiohalogen is selected from the following structures:

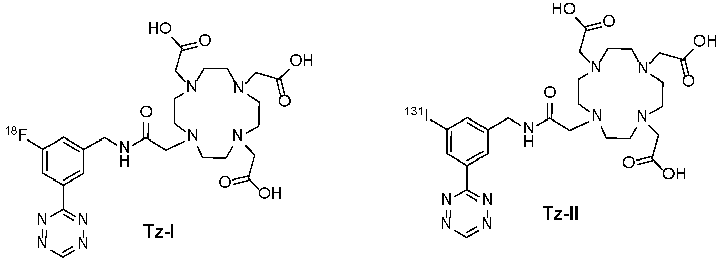

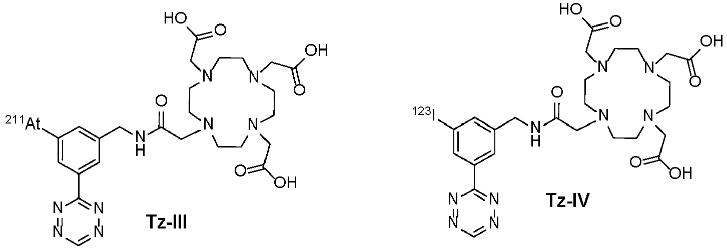

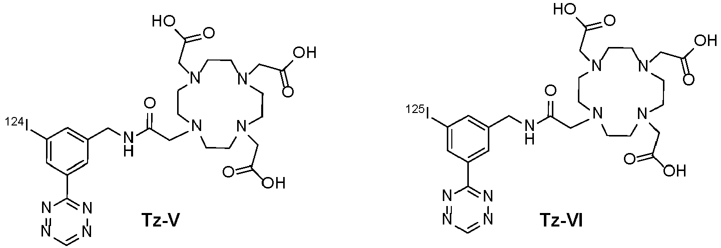

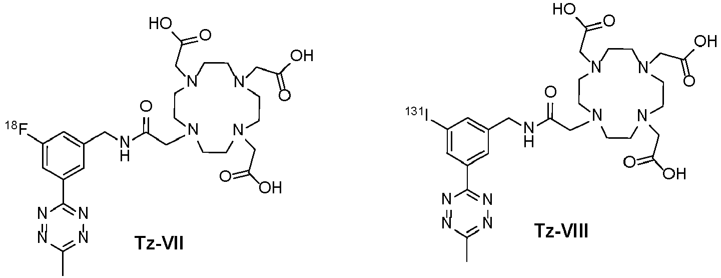

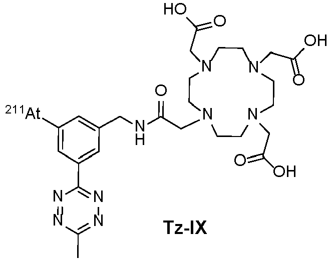

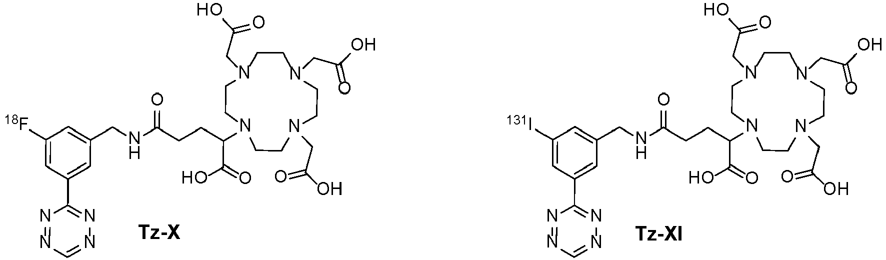

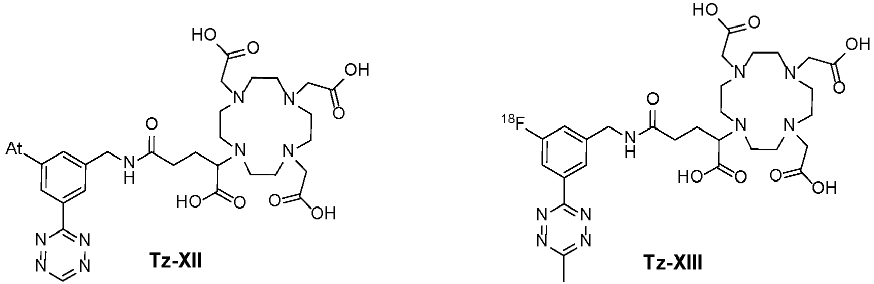

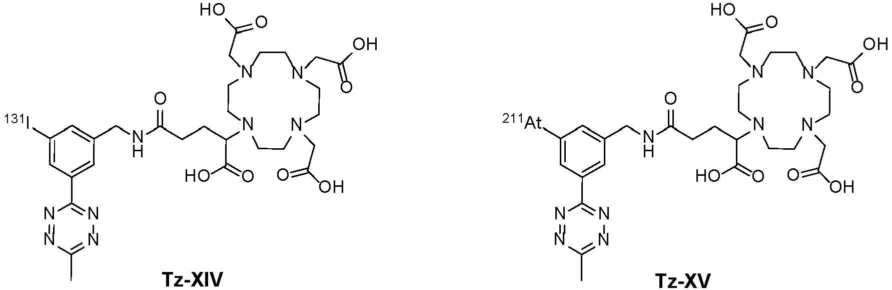

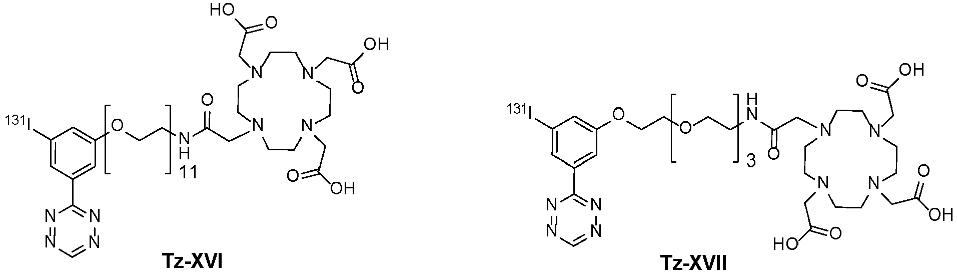

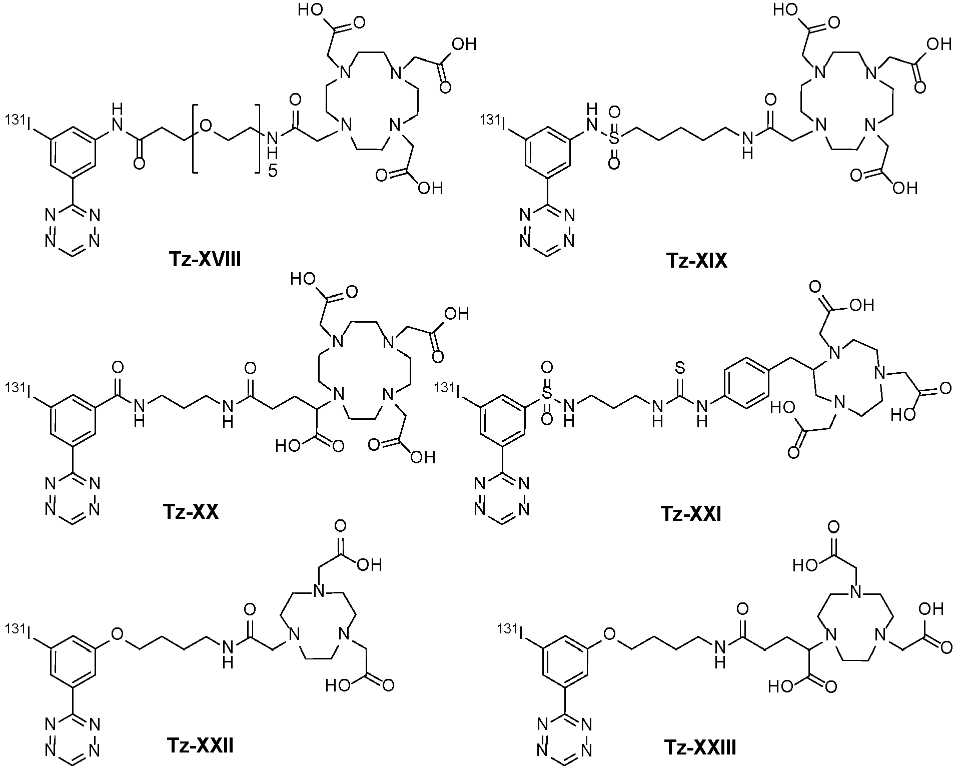

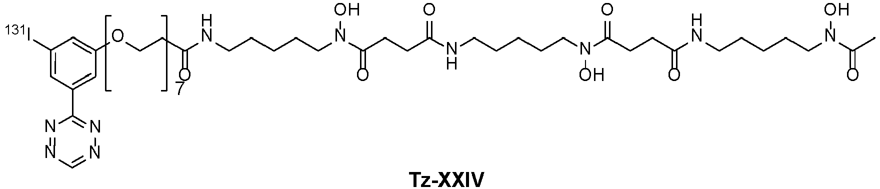

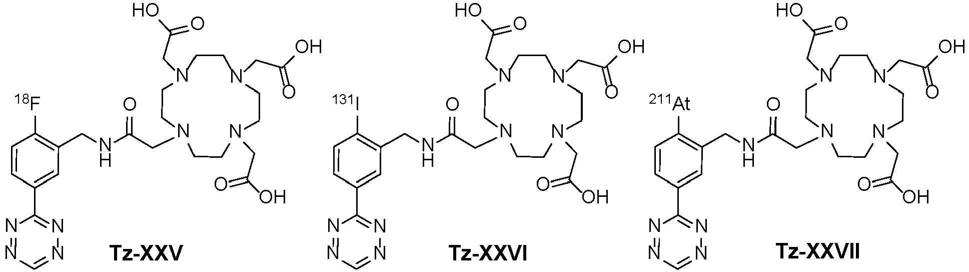

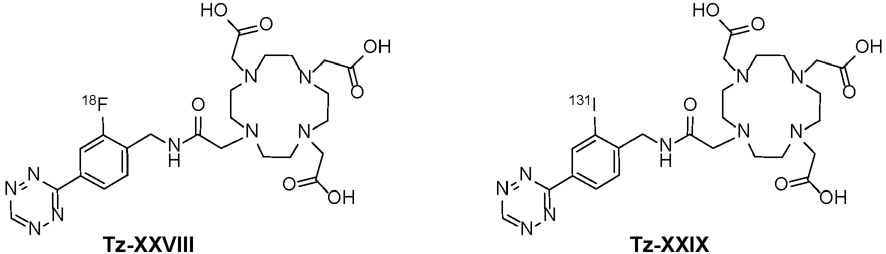

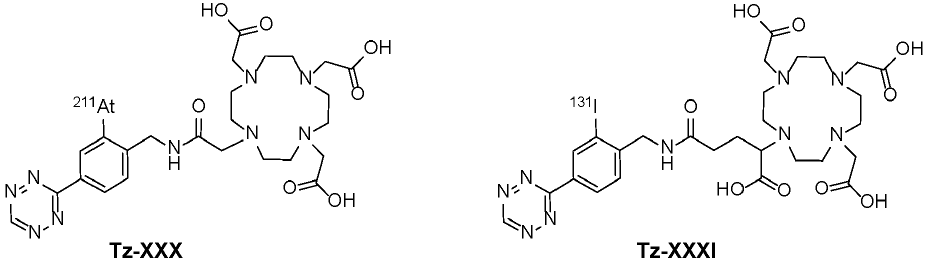

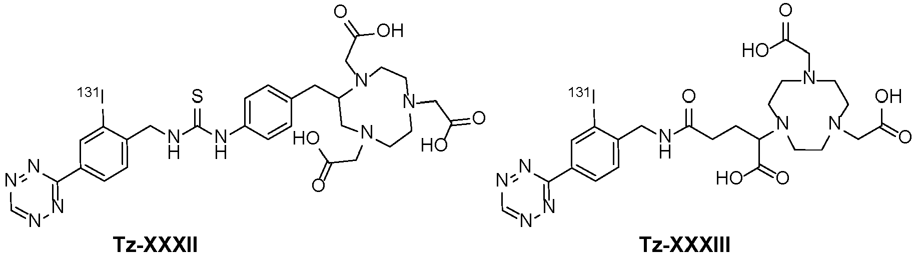

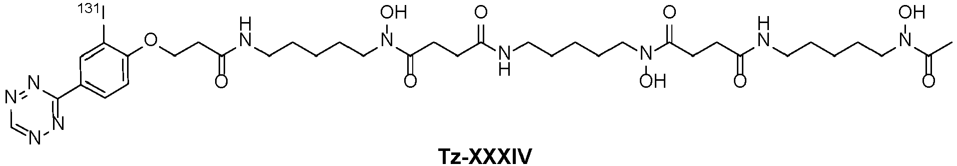

When the application of the 1,2,4,5-tetrazine of formula (I) is based on the features of a radiometal, a stable halogen is selected at position "Z".

Accordingly, in another embodiment of the first aspect, the compound according to formula (I) is a compound wherein Z is selected from: ¹⁹F, ⁷⁹Br, ⁸¹Br, ¹²⁷I, and wherein X is labeled with a radionuclide metal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In ¹¹³In ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th.

In a preferred embodiment, the 1,2,4,5-tetrazine compound of formula (I) labeled with a radiometal is a compound wherein X is labeled with a radionuclide metal selected from: ⁶⁴Cu, ⁶⁷Cu, ⁶⁸Ga, ⁹⁰Y, ⁸⁹Zr, ^{99m}Tc, ¹¹¹In, ¹⁷⁷Lu, ²⁰³Pb, ²¹²Pb, ²²⁵Ac.

In this preferred embodiment, the 1,2,4,5-tetrazine compound of formula (I) is labeled with a radiometal, where the radionuclide metal, designated as X, is selected from a group including ⁶⁴Cu, ⁶⁷Cu, ⁶⁸Ga, ⁹⁰Y, ⁸⁹Zr, ^{99m}Tc, ¹¹¹In, ¹⁷⁷Lu, ²⁰³Pb, ²¹²Pb and ²²⁵Ac. The selection of these radiometals is supported by their unique nuclear properties and the broad spectrum of applications they offer, making them suitable for both diagnostic imaging and targeted radiotherapy in nuclear medicine. One of the key advantages of these radiometals lies in their suitability for various imaging modalities, enabling precise diagnostic capabilities. For example, ⁶⁴Cu and ⁶⁸Ga are positron-emitting isotopes ideal for positron emission tomography (PET) imaging. ⁶⁴Cu, with its half-life of approximately 12.7 hours, provides sufficient time for imaging and prolonged monitoring of radiopharmaceutical distribution. Meanwhile, ⁶⁸Ga, with its shorter half-life of about 68 minutes, is excellent for rapid imaging scenarios, facilitating the timely assessment of pharmacokinetics and dynamic biological processes. For therapeutic purposes, radionuclides such as ⁶⁷Cu, ⁹⁰Y, ¹⁷⁷Lu, ²⁰³Pb, ²¹²Pb, and ²²⁵Ac are chosen due to their emission of beta or alpha particles, which are highly effective in destroying malignant cells. ⁹⁰Y and ¹⁷⁷Lu, as beta emitters, are particularly valuable in treating various cancers through targeted radionuclide therapy. They deliver high radiation doses to tumors with precision, thereby maximizing therapeutic efficacy while minimizing damage to surrounding healthy tissues. ²²⁵Ac stands out for its alpha emission, offering extremely high linear energy transfer (LET), which induces double-strand breaks in DNA, resulting in potent cytotoxic effects with minimal collateral damage. This is especially beneficial in treating micro-metastatic disease where precision is crucial.

Furthermore, ⁸⁹Zr and ^{99m}Tc play important roles in imaging. ⁸⁹Zr, with its relatively long half-life of 78.4 hours, is ideally suited for immuno-PET imaging, enabling prolonged tracking of monoclonal antibodies and providing detailed insights into the biodistribution and tumor targeting of antibody-based therapeutics. ^{99m}Tc, the most widely used isotope in clinical SPECT imaging, offers optimal gamma emission and a convenient half-life of approximately 6 hours. Its versatility and availability make it an invaluable tool for a wide range of diagnostic procedures, from cardiac imaging to bone scans.

The compatibility of these radionuclide metals with existing chelator technologies and radiolabeling protocols further enhances their practical application, enabling seamless integration into established clinical workflows. The chelators, including DOTA, NOTA, and DFO, are specifically chosen to match the coordination chemistry of these metals, ensuring the formation of highly stable complexes. This stability is essential for maintaining the integrity of the radiopharmaceuticals in vivo, preventing decomplexation, and minimizing off-target effects. The tailored chelator design also allows for the adjustment of pharmacokinetic properties, enhancing the targeting efficiency and overall efficacy of the radiopharmaceuticals. This chelator chemistry not only ensures robust radiolabeling but also enhances the versatility and safety of the compounds, making them suitable for a wide range of diagnostic and therapeutic applications in nuclear medicine. This not only facilitates regulatory approval processes but also ensures the scalability of production, making these radiopharmaceuticals readily accessible for widespread clinical use. In another preferred embodiment wherein the 1,2,4,5-tetrazine of formula (I) is labeled with a radiometal, the compound is selected from the following structures:

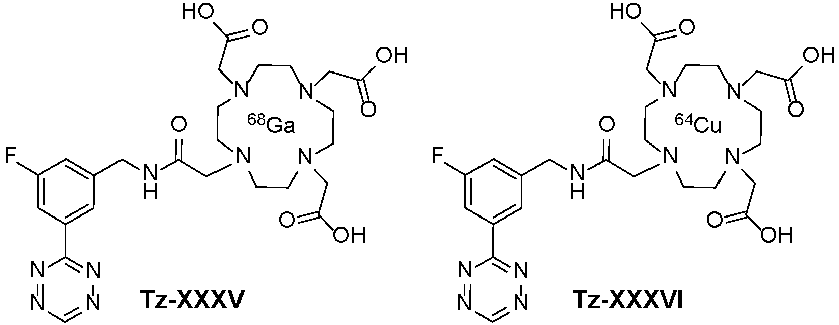

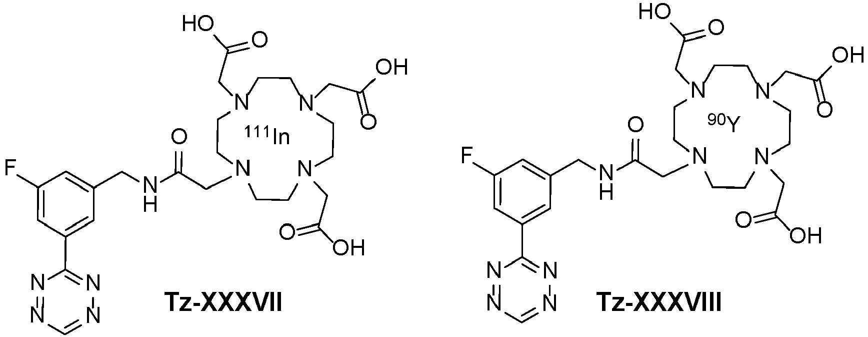

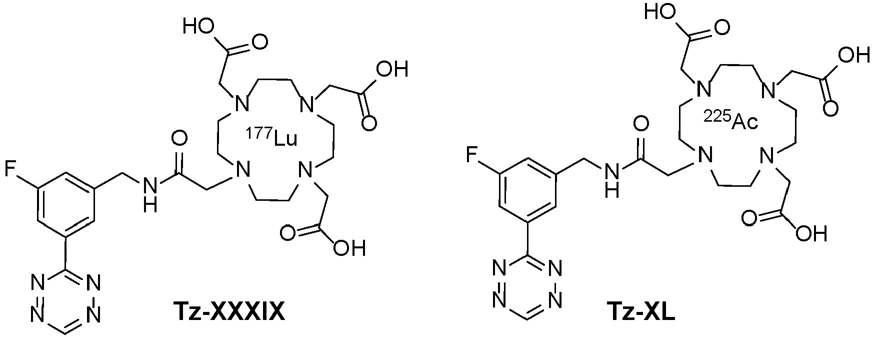

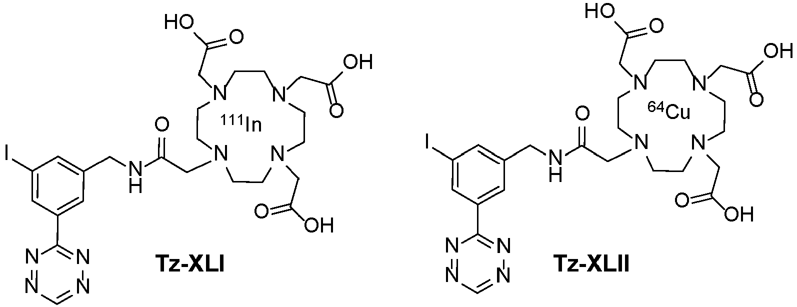

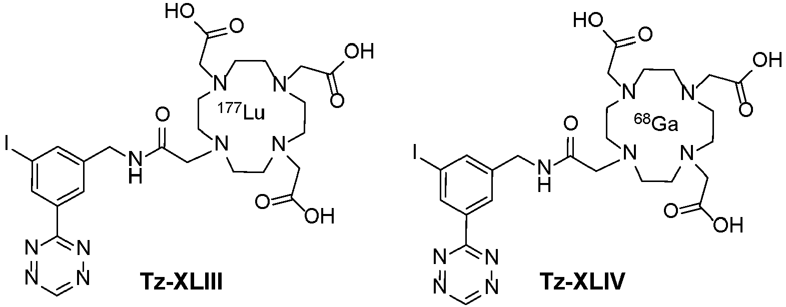

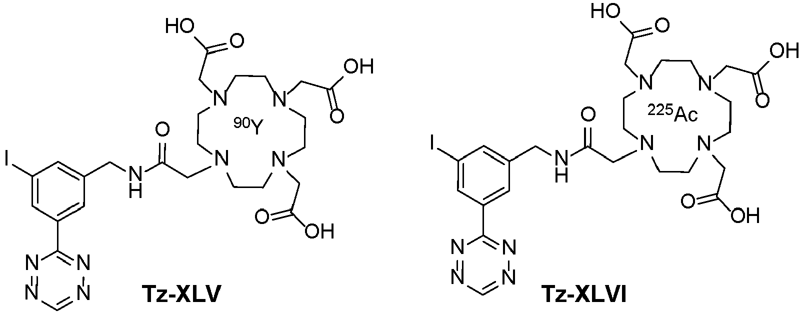

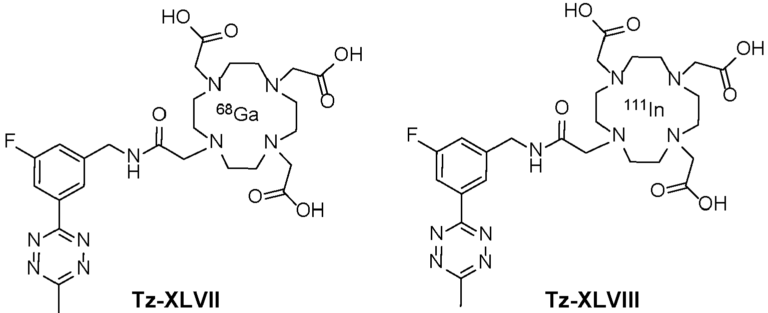

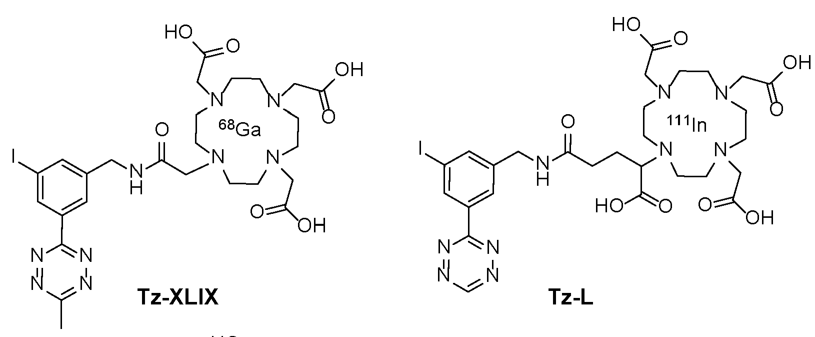

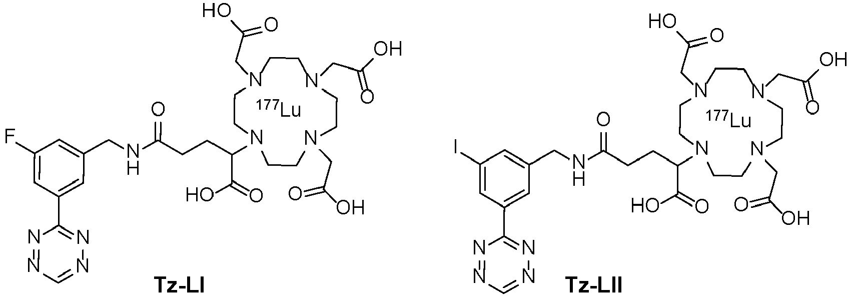

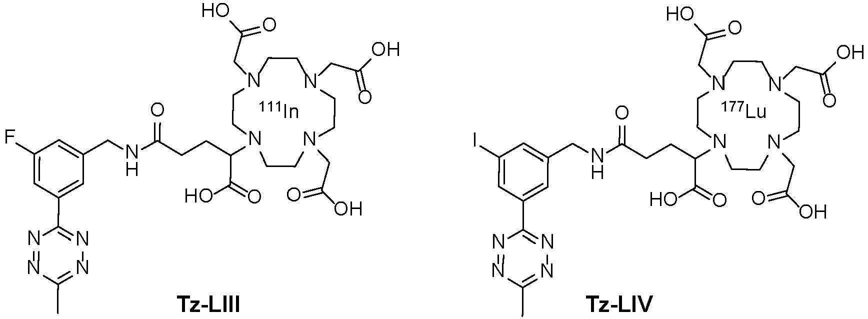

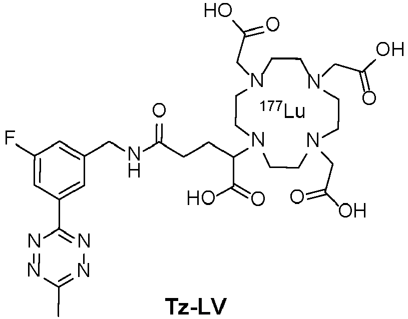

In another embodiment of the first aspect, the compound of formula (I) is to be applied as a precursor and thus, a leaving group is selected at position "Z". Such a precursor compound is suitable for labeling with a radiohalogen whereby the leaving group at position "Z" is replaced by a radiohalogen. These precursor compounds are thus applicable for providing compounds of formula (I) comprising a radiohalogen. Since the final application of precursor after labeling with a radiohalogen is based on the features of the radiohalogen, the precursor compounds of formula (I) need not comprise a metal or a radiometal. Therefore, the chelator "X" is unlabeled or comprise a stable metal.

Accordingly, in a preferred embodiment the 1,2,4,5-tetrazine compound of formula (I) is a compound wherein Z is selected from:
Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, and wherein X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi.

In another preferred embodiment, the compounds of formula (I) wherein "Z" is a leaving group, is a compound selected from:

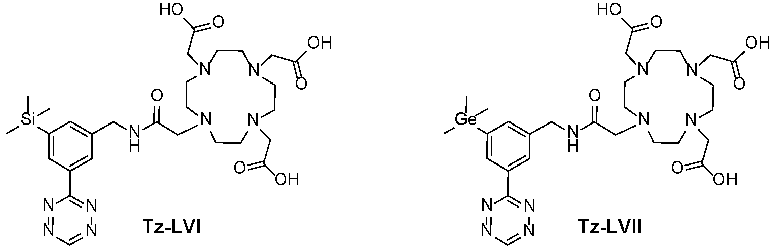

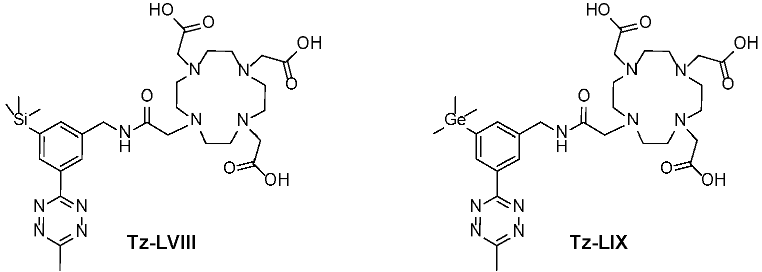

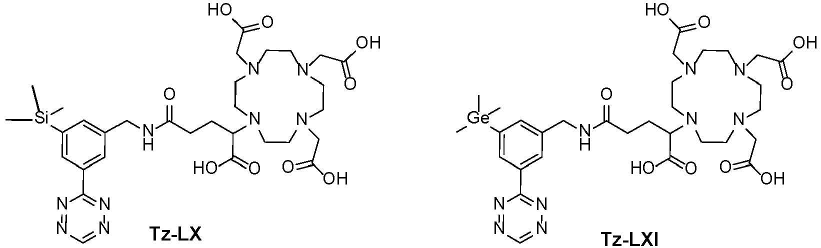

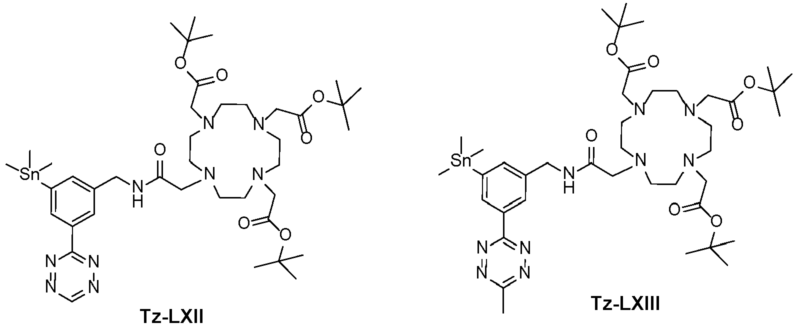

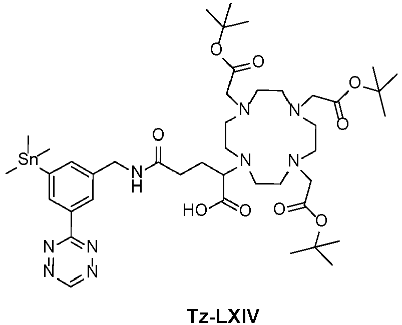

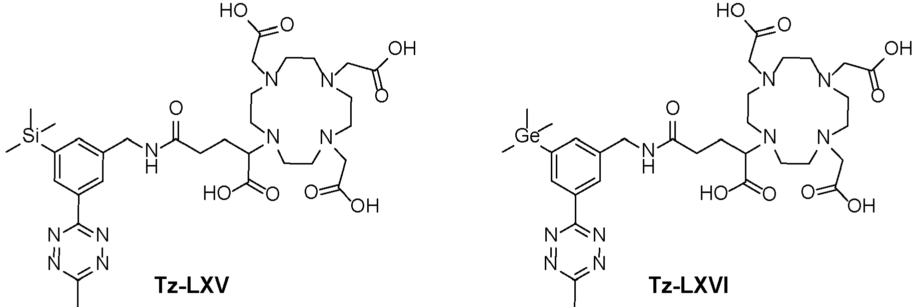

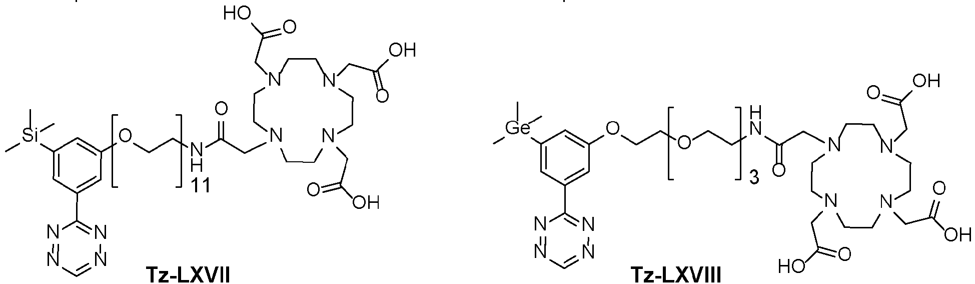

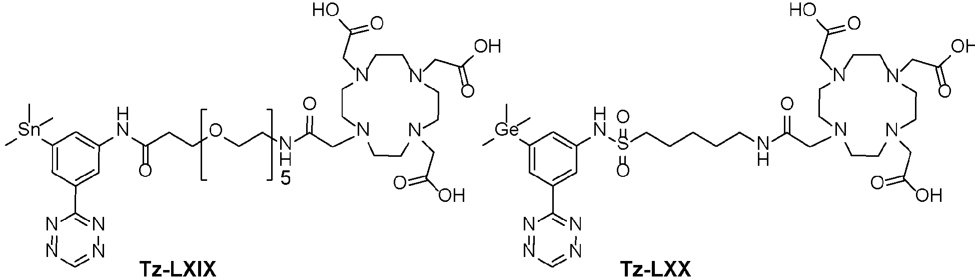

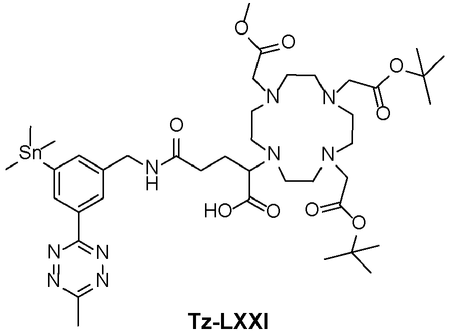

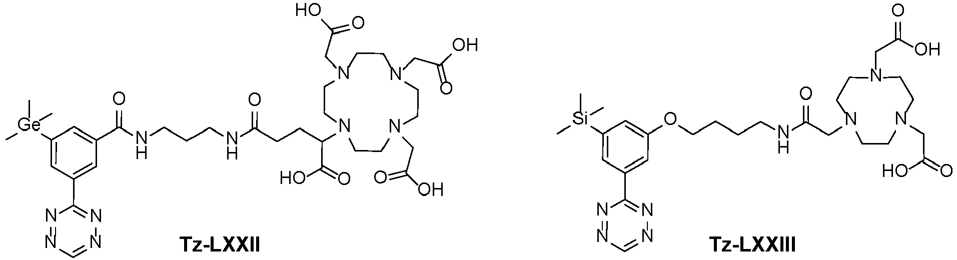

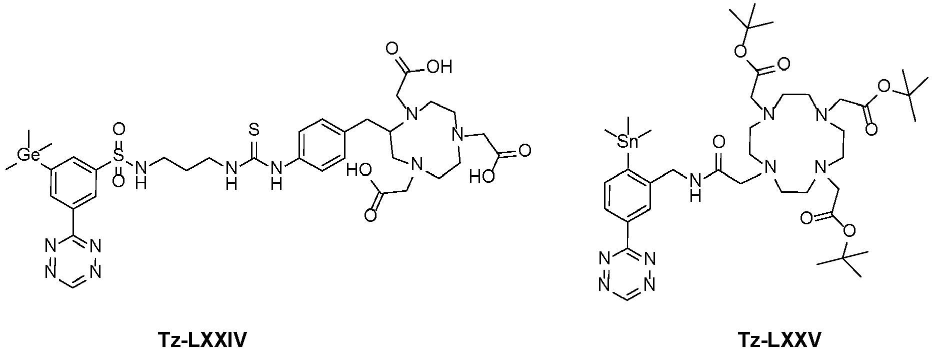

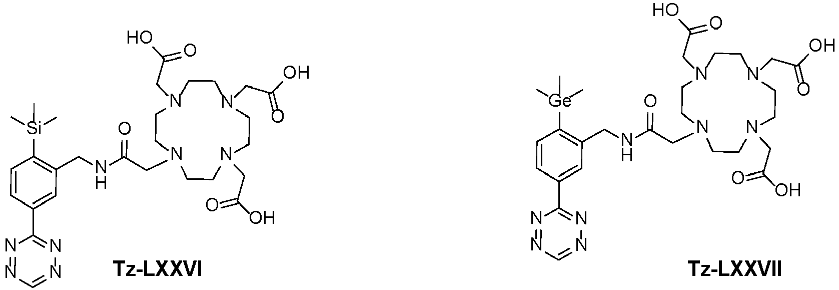

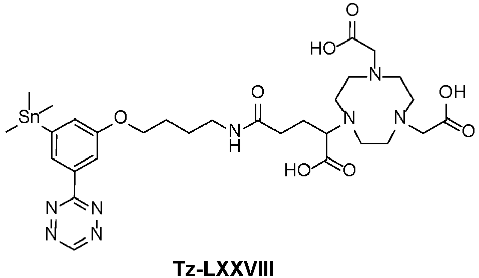

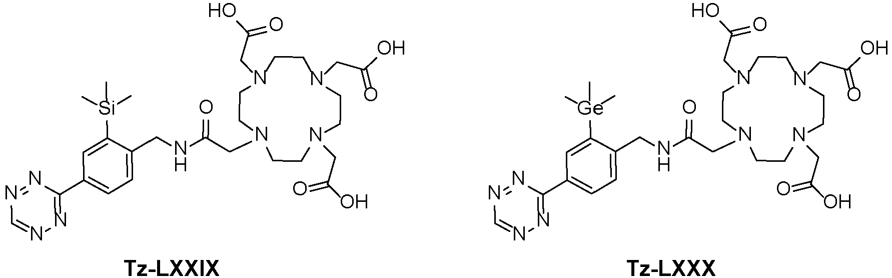

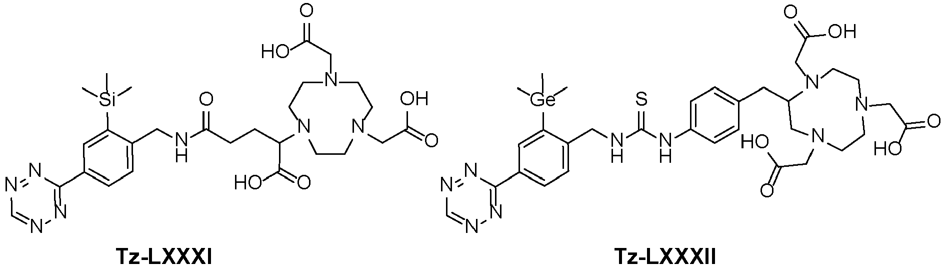

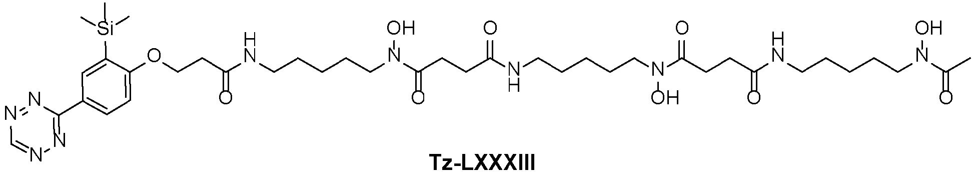

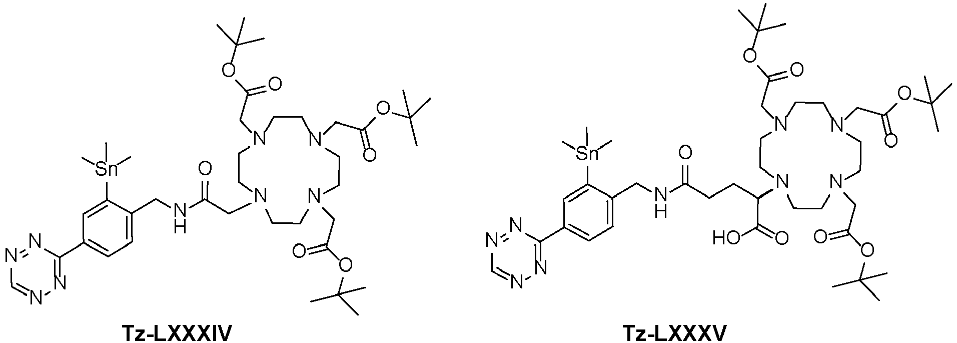

When the 1,2,4,5-tetrazine compounds of formula (I) is labeled with either a radiohalogen or a radiometal, the compounds are applicable for therapy, diagnostics, imaging, or theranostics depending on the features of the radionuclide. Which radionuclide to select for which purpose is known in the art because the applicability of the various radionuclides depends on their emission and half-life.

Thus, a second aspect of the present invention regards the tetrazines of formula (I) for use in therapy, diagnostics, imaging, and theranostics.

In one embodiment, the 1,2,4,5-tetrazine compounds of formula (I) for use in therapy, diagnostics, imaging, and theranostics is labeled with a radiohalogen.

Accordingly, this embodiment relates to 1,2,4,5-tetrazine compounds of formula (I): 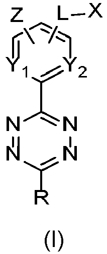 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
X is a chelator selected from:
   1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((l2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is a radionuclide halogen selected from: ¹⁸F, ⁷⁶Br, ⁷⁷Br, ^{80m}Br, ¹²³I, ¹²⁴I,¹²⁵I, ¹²⁶I, ¹³¹I, ²¹⁰At,²¹¹At, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi;
for use in pre-targeting based therapy, diagnostics, imaging, or theranostics.

A third aspect of the invention relates to the 1,2,4,5-tetrazine compounds of formula (I) being labeled with a radiometal for their use in therapy, diagnostics, imaging, and theranostics.

Accordingly, this embodiment relates to 1,2,4,5-tetrazine of formula (I): 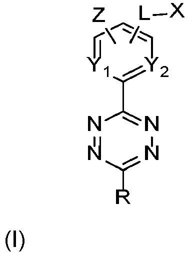 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
X is a chelator selected from:
   1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((l2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N-*hydroxysuccinamide (DFO), diethylenetriaminepentaacetic acid (DTPA), *trans-*cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is selected from: ¹⁹F, ⁷⁹Br, ⁸¹Br, ¹²⁷I, and
X is labeled with a radionuclide metal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In, ¹¹³In ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th,
for use in pre-targeting based therapy, diagnostics, imaging, or theranostics.

A fourth aspect of the invention relates to the use of the 1,2,4,5-tetrazines of formula (I) comprising a leaving group at position "Z" as precursors. Such precursors are very useful for labeling with radiohalogens to provide compounds of formula (I) labeled with one of the radiohalogens ¹⁸F, ⁷⁶Br, ⁷⁷Br, ^{80m}Br, ¹²³I, ¹²⁴I,¹²⁵I, ¹²⁶I, ¹³¹I, ²¹⁰At, and ²¹¹At.

Accordingly, this aspect relates to the use of compound of formula (I): 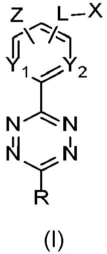 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is selected from: Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi,
as a precursor for radiolabeling.

A fifth aspect of the invention relates to the use of the 1,2,4,5-tetrazines of formula (I) comprising a halogen group at position "Z" for tetrazine labeling. Such compounds are very useful for labeling synthons applied in tetrazine ligations.

Accordingly, in a fifth aspect, the present invention relates to the use of a 1,2,4,5-tetrazine compound of formula (I): 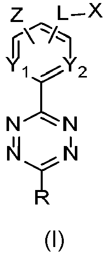 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)ₙNH-, - CONH(CH₂)ₙNH-, -NHCO(CH₂)ₙNH-, -(OCH₂CH₂)ₙ(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is a moiety selected from:
   ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At or ²¹¹At;
X is a chelator selected from:
   1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((l2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (*t*Bu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);

and wherein X is optionally labeled with a metal selected from:
   ⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr,⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.
as reagent in tetrazine ligation.

### Examples:

### Example 1: Synthesis of compound Tz-LXII, Scheme 1.

Scheme 1 shows the synthesis of compound **Tz-LXII.** The following reagents and conditions were used in the steps i - viii: i) NBS, AIBN, MeCN, reflux, 12 h; ii) NH4OH, THF, 50 °C, 5 h; iii) Boc2O, Et3N, DCM, rt, 12 h; iv) CH2Cl2, S8, NH2NH2 H2O, EtOH, 50 °C, 24 h; v) HCl, dioxane, rt, 2 h; vi) DOTA-NHS, DIPEA, DMF, rt, 12 h; vii) tBuDOTA-NHS, DIPEA, DMF, rt, 12 h; viii) (Me3Sn)2, Pd(PPh3)4, THF, 65 °C, MW, 2 h.

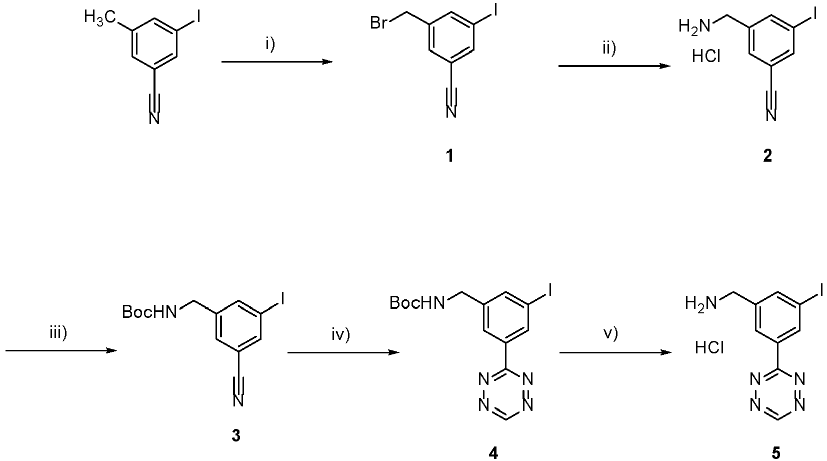

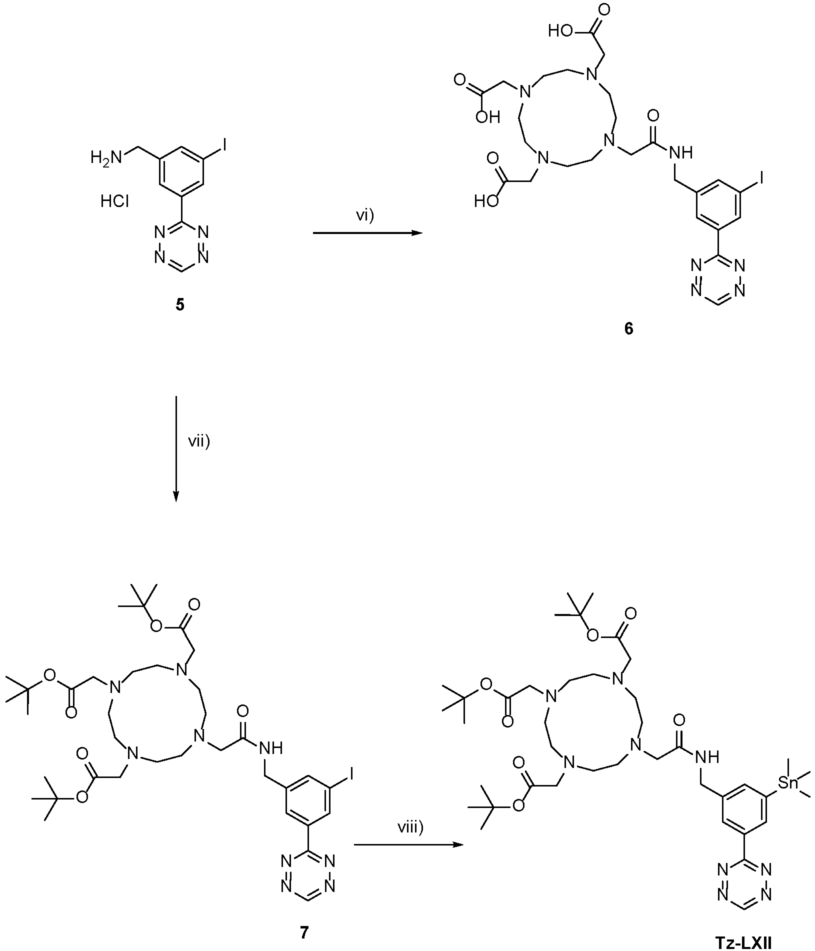

### 3-(Bromomethyl)-5-iodobenzonitrile (1)

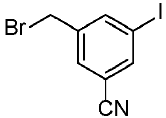

To a solution of 3-iodo-5-methylbenzonitrile (2.50 g, 10.28 mmol) and N-bromosuccinimide (2.28 g, 12.86 mmol) in CHCl₃ (40 mL) was added AIBN (0.67 g, 4.11 mmol). The reaction was refluxed for 24 h. The solvent was removed under vacuum and the crude purified by flash chromatography (heptane/EtOAc 95/5) to give 1.61 g (49%) of a white solid. *Rf* = 0.28 (n-Heptane:5%EtOAc); ¹H NMR (400 MHz, CDCl₃) δ 7.96 (d, *J* = 1.6 Hz, 1H), 7.89 (d, *J* = 1.6 Hz, 1H), 7.64 (t, *J* = 1.6 Hz, 1H), 4.38 (s, 2H) ¹³C NMR (101 MHz, CDCl₃) δ 142.20, 140.90, 140.12, 131.67, 116.55, 114.56, 94.05, 29.90.

### 3-(Aminomethyl)-5-iodobenzonitrile hydrochloride (2)

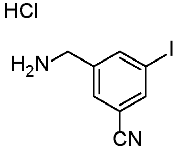

The compound was obtained from 3-(bromomethyl)-5-iodobenzonitrile (0.6 g, 1.86 mmol) and 7N solution of ammonia in MeOH (5 mL) following the general procedure E to give 0.43 g (78%) of 3-(aminomethyl)-5-iodobenzonitrile hydrochloride as a white solid. ¹H NMR (400 MHz, DMSO-d₆) δ 8.58 (br s, 3H), 8.29 - 8.26 (m, 2H), 8.03 (d, *J* = 1.6 Hz, 1H), 4.15 - 3.98 (m, 2H); ¹³C NMR (101 MHz, DMSO-d₆) δ 143.04, 140.21, 137.94, 132.70, 117.50, 113.43, 95.78, 41.15.

### Tert-butyl (3-cyano-5-iodobenzyl)carbamate (3)

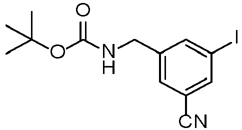

3-(Aminomethyl)-5-iodobenzonitrile hydrochloride (0.43 g, 1.46 mmol) and triethylamine (3.0 eq.) were dissolved in anhydrous CH₂Cl₂ (10 mL) at 0 °C. To this stirred solution was added di-tert-butyl dicarbonate (1.1 eq.), and the reaction allowed to warm to room temperature and stirred for 12 hours. The reaction mixture was evaporated under reduced pressure, and the residue was re-dissolved in diethyl ether (10 mL), which was washed successively with 0.5 M aq. HCl (2 x 5 mL), saturated NaHCO₃ (2 x 5 mL) and brine (5 mL). The organic layer was dried over anhydrous MgSO₄, filtered and evaporated under reduced pressure to give a white solid. The residue was purified by flash column chromatography (Heptane/EtOAc = 85/15) to afford 0.51 g (97%) of the desired compound as an orange solid (mixture of rotamers). *Rf* = 0.28 (Heptane:20%EtOAc); ¹H NMR (400 MHz, CDCl₃) δ 7.78 (br s, 2H), 7.47 (s, 1H), 5.05 (t, *J* = 6.5 Hz, 1H), 4.22 (d, *J* = 6.3 Hz, 2H), 1.39 (s, 9H); ¹³C NMR (101 MHz, CDCl₃) δ 155.79, 142.77, 140.64, 139.10, 129.88, 117.02, 114.22, 94.04, 80.29, 43.25, 27.41.

### Tert-butyl (3-iodo-5-(1,2,4,5-tetrazin-3-yl)benzyl)carbamate (4)

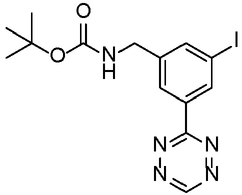

*Tert*-butyl (3-cyano-5-iodobenzyl)carbamate (0.43 g, 1.20 mmol), CH₂Cl₂ (1 equiv.), sulfur (0.25 equiv.), hydrazine monohydrate (8 equiv.) and ethanol (4.0 mL) were added to a microwave vial equipped with a stir bar. The vessel was sealed, and the reaction mixture was heated to 50 °C for 24 hours, before being allowed to cool to room temperature and unsealed. Then 3 ml of CH₂Cl₂ and NaNO₂ (10 equiv.) in water (40 ml) were added to the now yellow mixture followed by dropwise addition of acetic acid (14 mL), producing a mixture red in colour. The reaction mixture was extracted with CH₂Cl₂, washed with brine, dried with MgSO₄ and filtered before concentrating *in vacuo.* Purification by flash chromatography (95/5 Heptane/EtOAc) afforded 0.11 g (22%) of the desired compound as a red solid. *Rf* = 0.35 (Heptane:20%EtOAc); ¹H NMR (400 MHz, CDCl₃) δ 10.24 (s, 1H), 8.85 (d, *J* = 1.7 Hz, 1H), 8.49 (s, 1H), 7.90 (d, *J* = 1.8 Hz, 1H), 5.07 (s, 1H), 4.40 (d, *J* = 4.9 Hz, 2H), 1.47 (s, 9H); ¹³C NMR (101 MHz, CDCl₃) δ 165.16, 158.03, 155.83, 142.66, 140.69, 135.88, 133.54, 126.13, 95.10, 80.12, 43.76, 28.37.

### (3-lodo-5-(1,2,4,5-tetrazin-3-yl)phenyl)methanamine hydrochloride (5)

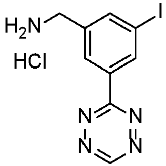

*Tert*-butyl 3-iodo-5-(1,2,4,5-tetrazin-3-yl)benzylcarbamate (0.055 g, 0.13 mmol) was solubilized in CH₂Cl₂ (1 mL); subsequently a solution of HCl in dioxane (4.0 M, 3.0 mL) was added. The mixture was stirred at room temperature for 2 h. The reaction was then concentrated under reduced pressure to give 0.045 g (97%) of a pink solid. ¹H NMR (600 MHz, DMSO) δ 10.69 (s, 1H), 8.79 (t, *J* = 1.6 Hz, 1H), 8.68 (t, *J* = 1.6 Hz, 1H), 8.41 (br s, 3H), 8.25 (t, *J* = 1.6 Hz, 1H), 4.17-4.22 (m, 2H); ¹³C NMR (151 MHz, DMSO) δ 164.61, 158.93, 142.12, 138.14, 136.30, 134.59, 128.18, 96.15.

### 2,2',2"-(10-(2-((3-lodo-5-(1,2,4,5-tetrazin-3-yl)benzyl)amino)-2-oxoethyl)-1,4,7,10-tetraazacyclo dodecane-1,4,7-triyl)triacetic acid (6)

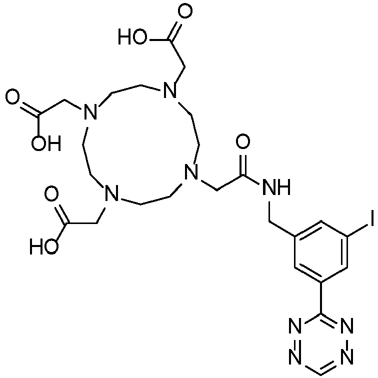

To a solution of (3-iodo-5-(1,2,4,5-tetrazin-3-yl)phenyl)methanamine hydrochloride (0.05 g, 0.14 mmol) and DIPEA (0.19 mL, 1.14 mmol) in DMF (1 mL) was added DOTA-NHS HPF₆/TFA salt (0.12 g, 0.15 mmol). The reaction was stirred at rt for 12 hours and directly purified by preparative HPLC to give 0.065 g (65%) of the desired compound as a red solid. ¹H NMR (600 MHz, D₂O) δ 10.35 (s, 1H), 8.60 (s, 1H), 8.24 (s, 1H), 7.92 (s, 1H), 4.44 (s, 2H), 3.71 (br s, 6H), 3.24 (br s, 18H).

### Tri-tert-butyl 2,2',2"-(10-(2-((3-iodo-5-(1,2,4,5-tetrazin-3-yl)benzyl)amino)-2-oxoethyl)-1,4,7, 10-tetraazacyclododecane-1,4,7-triyl)triacetate (7)

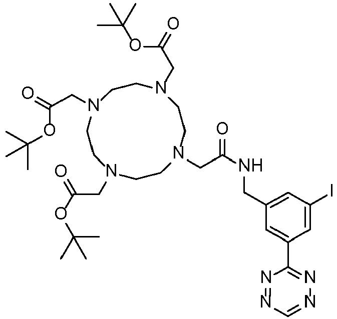

To a solution of (3-iodo-5-(1,2,4,5-tetrazin-3-yl)phenyl)methanamine hydrochloride (0.14 g, 0.42 mmol) and DIPEA (0.31 mL, 1.73 mmol) in DMF (1 mL) was added tBu-DOTA-NHS (0.37 g, 0.55 mmol). The reaction was stirred at rt for 12 hours and directly purified by flash chromatography to give 0.34 g (95%) of the desired compound as a red solid. ¹H NMR (600 MHz, CDCl₃) δ 10.25 (s, 1H), 8.83 (s, 1H), 8.47 (s, 1H), 7.92 (s, 1H), 7.57 (s, 1H), 4.47 (s, 2H), 4.18 (d, *J* = 13.9 Hz, 1H), 3.77 - 3.17 (m, 18H), 2.98 (s, 4H), 1.49 (s, 9H), 1.39 (s, 18H).

### Tri-tert-butyl 2,2',2"-(10-(2-((3-(1,2,4,5-tetrazin-3-yl)-5-(trimethylstannyl)benzyl)amino)-2-oxoethyl)-1,4,7, 10-tetraazacyclododecane-1,4,7-triyl)triacetate (Tz-LXII)

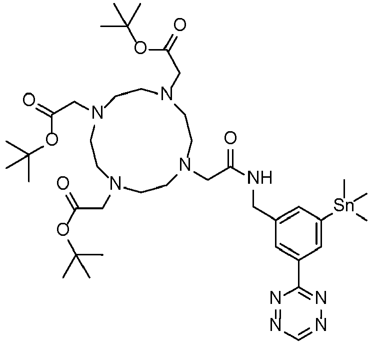

Pd(PPh₃)₄ (13.3 mg, 0.011 mmol) and hexamethylditin (50 µL, 0.57 mmol) were successively added to a microwave vial equipped with a stir bar which was then sealed and purged with N₂. A solution of 7 (0.05 g, 0.057 mmol) in dry THF (2.5 mL) was added via a syringe and the reaction allowed to stir at 65 °C in a microwave for 1 hours. The reaction was allowed to cool to room temperature and unsealed before being quenched with saturated aqueous KF (1 mL). The solution was extracted with CH₂Cl₂ (3 x 10 mL) washed with brine (10 mL), dried over MgSO₄, filtered, and concentrated under reduced pressure. The crude was purified using flash chromatography (98/2 CH₂Cl₂/MeOH) to yield 0.03 g (57%) of 8 as a purple solid. *Rf* = 0.31 (98/22 CH₂Cl₂/MeOH); ¹H NMR (400 MHz, MeOD) δ 10.35 (s, 1H), 8.60 (s, 1H), 8.43 (s, 1H), 7.71 (s, 1H), 4.76 - 4.24 (m, 2H), 3.80 - 1.91 (m, 22H), 1.4
7 (s, 9H), 1.34 (s, 18H), 0.37 (s, 9H).

### Example 2: Synthesis of compound Tz-LVI, Scheme 2.

Scheme 2 shows the synthesis of compound Tz-LVI. The following reagents and conditions were used in the steps i - vii: i) (CH3)3SiCl, nBuLi, THF, -78 °C to rt, 12 h; ii) NBS, AIBN, chlorobenzene, reflux 12 h; iii) NH4OH, THF, 50 °C, 5 h; iv) Boc2O, Et3N, DCM, rt, 12 h; v) CH2Cl2, S8, NH2NH2 . H2O, EtOH, 50 °C, 24 h; vi) HCl, dioxane, rt, 2 h; vii) DOTA-NHS, DIPEA, DMF, rt, 12 h.

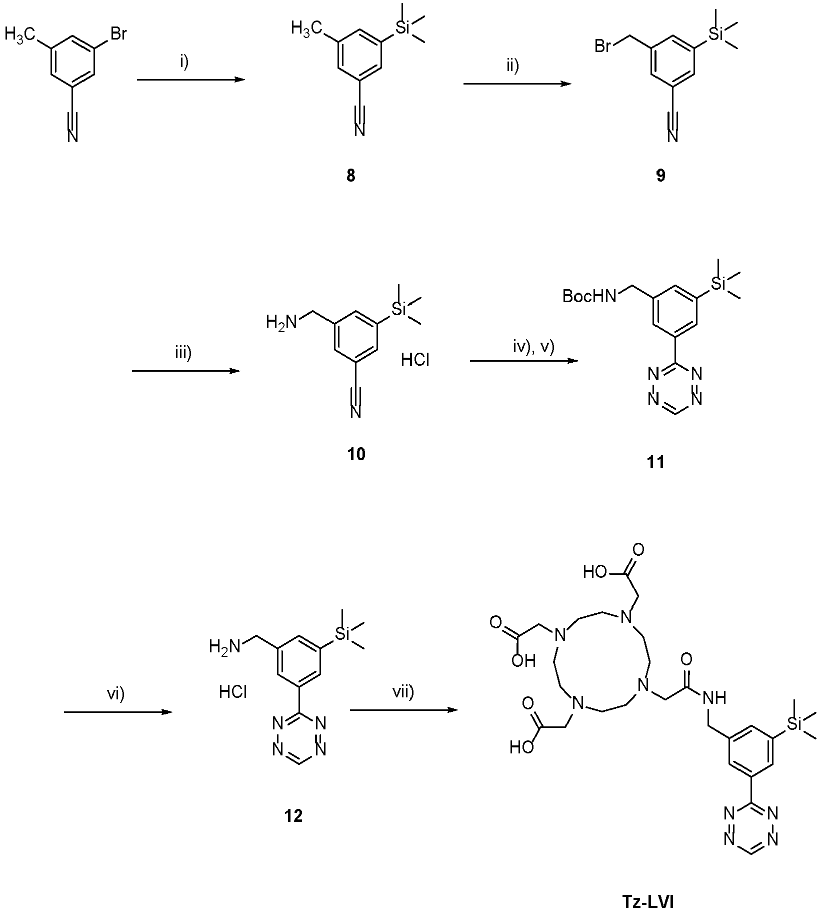

### 3-Methyl-5-(trimethylsilyl)benzonitrile (8)

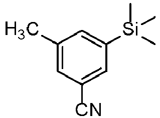

To a solution of bromobenzonitrile (3.0 g, 15.30 mmol) and trimethylsilyl chloride (2.91 ml, 22.95 mmol) in anhydrous THF (50 mL) at -78 °C was added 2M n-butyllithium (8.41 mL, 16.83 mmol) dropwise under an N₂ atmosphere. The solution was stirred at -78 °C for 15 min and then at room temperature for 1 h. H₂O (50 mL) was added, then the layers were separated, and the aqueous layer was extracted with Et₂O (3 x 50 ml). The combined organic portions were washed with brine, dried over MgSO₄, filtered and concentrated in vacuo to give 2.89 g (99%). Rf = 0.51 (Heptane/EtAOC 97/3). ¹H NMR (400 MHz, CDCl₃) δ 7.58 (s, 1H), 7.51 (s, 1H), 7.43 (s, 1H), 2.39 (s, 3H), 0.28 (s, 9H); ¹³C NMR (101 MHz, CDCl₃) δ 143.71, 139.67, 139.48, 135.46, 133.95, 120.80, 113.29, 22.56.

### 3-(Bromomethyl)-5-(trimethylsilyl)benzonitrile (9)

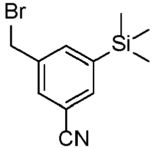

To a solution of 3-methyl-5-(trimethylsilyl)benzonitrile (0.008 g, 5.28 mmol) and N-bromosuccinimide (0.94 g, 5.28 mmol) in chlorobenzene (40 mL) was added AIBN (0.67 g, 0.005 mmol). The reaction was refluxed for 12 h. The solvent was removed under vacuum and the crude purified by flash chromatography (heptane/EtOAc 99/1) to give 1.32 g (93%) of a white solid. *Rf* = 0.28 (99/1 n-heptane/EtOAc). ¹H NMR (600 MHz, CDCl₃) δ 7.69 (s, 2H), 7.65 (s, 1H), 4.47 (s, 2H), 0.30 (s, 9H), ¹³C NMR (151 MHz, CDCl₃) δ 143.83, 138.37, 137.91, 136.84, 132.72, 118.77, 112.73, 31.68, -1.29.

### 3-(Aminomethyl)-5-(trimethylsilyl)benzonitrile hydrochloride (10)

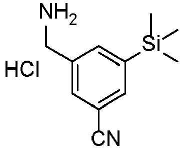

To a solution of the 3-(bromomethyl)-5-(trimethylsilyl)benzonitrile (0.6 g, 1.86 mmol) in THF (20 mL) was added a 7N solution of ammonia in MeOH (12 mL). The reaction was stirred at room temepearture for 5 hours. The solution was concentrated and taken up with water (20 mL) and the resulting water phase was extracted with EtOAc (3 x 20 mL). The combined organic portions were washed with brine, dried over MgSO₄, filtered and concentrated in vacuo to give the compound as a crude. The compound was resolubilized in 4 M HCl in Dioxane. The solvent was evaporated, and the triturated in diethyl ether to give 150 mg (28 %) of a white solid. ¹H NMR (600 MHz, DMSO) δ 8.25 (s, 2H), 7.99 - 7.94 (m, 2H), 7.92 (s, 1H), 4.11 (s, 2H), 0.30 (s, 9H); ¹³C NMR (151 MHz, DMSO) δ 142.78, 138.43, 136.65, 134.66, 132.91, 118.67, 111.15, 41.57, -1.48.

### Tert-butyl (3-(1,2,4,5-tetrazin-3-yl)-5-(trimethylsilyl)benzyl)carbamate (11)

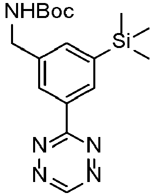

3-(Aminomethyl)-5-(trimethylsilyl)benzonitrile hydrochloride (0.15 g, 0.62 mmol) and triethylamine (3.0 eq.) were dissolved in anhydrous CH₂Cl₂ (10 mL) at 0 °C. To this stirred solution was added di-tert-butyl dicarbonate (1.1 eq.), and the reaction allowed to warm to room temperature and stirred for 12 hours. The reaction mixture was evaporated under reduced pressure, and the residue was re-dissolved in diethyl ether (10 mL), which was washed successively with 0.5 M aq. HCl (2 x 5 mL), saturated NaHCO₃ (2 x 5 mL) and brine (5 mL). The organic layer was dried over anhydrous MgSO₄, filtered and evaporated under reduced pressure to give a white solid. The crude (0.19 gr, 0.59 mmol), CH₂Cl₂ (1 equiv.), sulfur (0.25 equiv.), hydrazine monohydrate (8 equiv.) and ethanol (4.0 mL) were added to a microwave vial equipped with a stir bar. The vessel was sealed, and the reaction mixture was heated to 50 °C for 24 hours, before being allowed to cool to room temperature and unsealed. Then 3 ml of CH₂Cl₂ and NaNO₂ (10 equiv.) in water (20 ml) were added to the now yellow mixture followed by dropwise addition of acetic acid (7 mL), producing a mixture red in colour. The reaction mixture was extracted with CH₂Cl₂, washed with brine, dried with MgSO₄ and filtered before concentrating *in vacuo.* Purification by flash chromatography (95/5 n-Heptane/EtOAc) afforded 0.035 g (16% over 2 steps) of the desired compound as a red solid. *Rf* = 0.35 (n-Heptane/EtOAc 80/20); ¹H NMR (400 MHz, CDCl₃) δ 10.04 (s, 1H), 8.48 (s, 1H), 8.24 (s, 1H), 7.40 (s, 1H), 4.84 (s, 2H), 1.49 (s, 9H), 0.15 (s, *J* = 1.2 Hz, 9H); ¹³C NMR (151 MHz, CDCl₃) δ 166.57, 158.00, 152.23, 143.19, 137.62, 135.62, 132.66, 131.41, 128.38, 86.06, 44.44, 28.21, -1.09.

### (3-(1,2,4,5-Tetrazin-3-yl)-5-(trimethylsilyl)phenyl)methanamine hydrochloride (12)

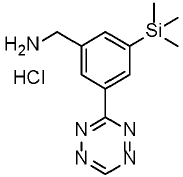

Tert-butyl (3-(1,2,4,5-tetrazin-3-yl)-5-(trimethylsilyl)benzyl)carbamate (0.020 g, 0.055 mmol) was solubilized in a solution of HCl in dioxane (4.0 M, 3.0 mL) was added. The mixture was stirred at room temperature for 2 h. The reaction was then concentrated under reduced pressure to give 0.02 g (90%) of a pink solid. ¹H NMR (600 MHz, MeOD) δ 10.42 (s, 1H), 8.84 (t, *J* = 1.4 Hz, 1H), 8.72 (t, *J* = 1.8 Hz, 1H), 7.98 (dd, *J* = 1.9, 1.0 Hz, 1H), 4.33 (s, 2H), 0.42 (s, 9H); ¹³C NMR (151 MHz, MeOD) δ 167.53, 159.53, 144.86, 139.24, 135.18, 134.46, 133.72, 129.99, 44.19, -1.28.

### 2,2',2"-(10-(2-((3-(1,2,4,5-Tetrazin-3-yl)-5-(trimethylsilyl)benzyl)amino)-2-oxoethyl)-1,4,7, 10-tetraazacyclododecane-1,4,7-triyl)triacetic acid (Tz-LVI)

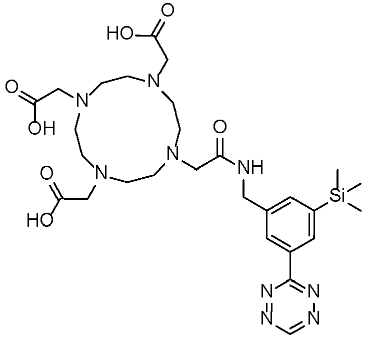

To a solution of (3-(1,2,4,5-tetrazin-3-yl)-5-(trimethylsilyl)phenyl)methanamine hydrochloride (0.01 g, 0.023 mmol) and DIPEA (0.022 mL, 0.013 mmol) in DMF (1 mL) was added DOTA-NHS HPF₆/TFA salt (0.02 g, 0.026 mmol). The reaction was stirred at rt for 12 hours and directly purified by preparative HPLC to give 0.02 g (98%) of the desired compound as a red solid. ¹H NMR (400 MHz, D₂O) δ 10.41 (s, 1H), 8.52 (s, 1H), 8.32 (s, 1H), 7.85 (s, 1H), 4.59 (s, 2H), 4.11 - 3.16 (m, 24H), 0.37 (s, 9H).

### Example 3: Synthesis of compound Tz-LVII, Scheme 3.

Scheme 3 shows the synthesis of compound **Tz-LVII.** The following reagents and conditions were used in the steps i - vii: *i*) (CH₃)₃GeCl, nBuLi, THF, -78 °C to rt, 12 h; *ii*) NBS, AIBN, chlorobenzene, reflux 12 h; *iii*) NH₄OH, THF, 50 °C, 5 h; iv) Boc₂O, Et₃N, DCM, rt, 12 h; v) CH₂Cl₂, S₈, NH₂NH_{2 .} H₂O, EtOH, 50 °C, 24 h; vi) HCl, dioxane, rt, 2 h; vii) DOTA-NHS, DIPEA, DMF, rt, 12 h.

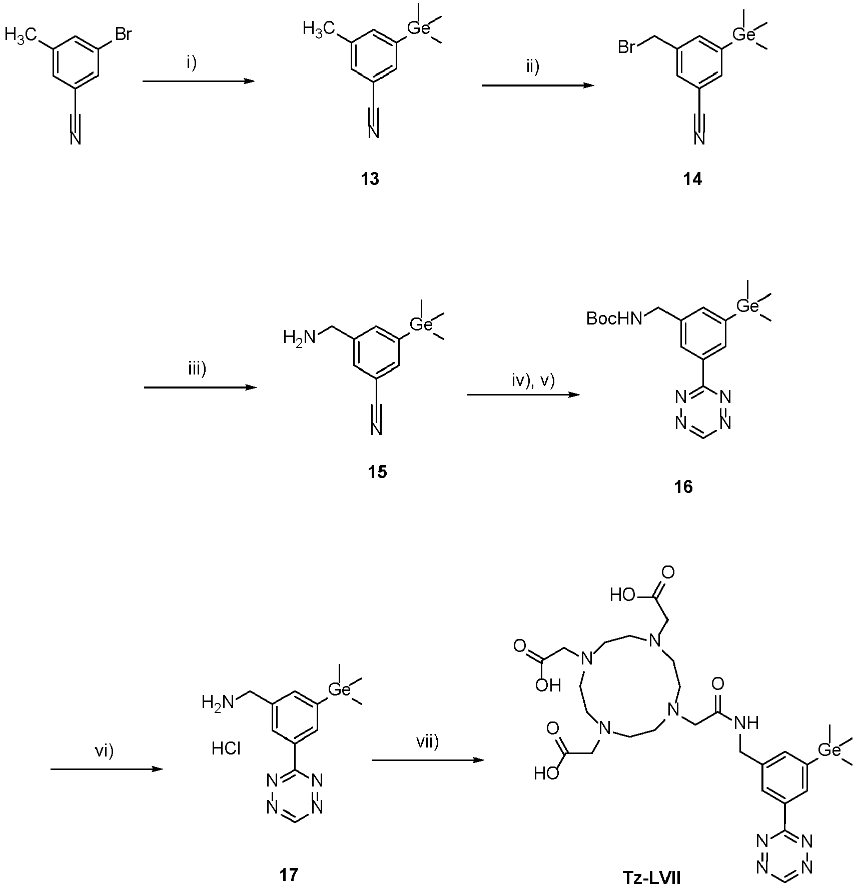

### 3-Methyl-5-(trimethylgermyl)benzonitrile (13)

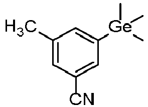

To a solution of 3-bromo-5-methylbenzonitrile (2.00 g, 10.20 mmol) and chlorotrimethylgermane (1.89 mL, 15.30 mmol) in anhydrous THF (40 mL) at -78 °C was added n-butyllithium (2M in pentane, 7.65 mL, 15.30 mmol) dropwise under an argon atmosphere. The solution was stirred at -78 °C for 15 minutes and then at room temperature for 1 h. H₂O (50 mL) was added, then the layers were separated, and the aqueous layer was extracted with EtOAc (3 x 30 mL). The combined organic portions were washed with brine, dried over MgSO4, filtered and concentrated under reduced pressure. Purification by flash chromatography (98/2 n-heptane/EtOAc) afforded 1.1 g (46%) of the desired compound as a yellow oil. *Rf* = 0.41 (95/5 n-heptane/EtOAc); ¹H NMR (600 MHz, CDCl₃) δ 7.53 (s, 1H), 7.47 (s, 1H), 7.40 (s, 1H), 2.38 (s, 3H), 0.40 (s, 9H); ¹³C NMR (151 MHz, CDCl₃) δ 146.31, 140.15, 139.97, 135.59, 134.05, 121.34, 113.87, 23.08, 0.00.

### 3-(Bromomethyl)-5-(trimethylgermyl)benzonitrile (14)

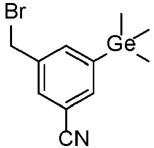

To a solution of 15 (1.55 g, 6.63 mmol) and N-bromosuccinimide (1.29 g, 7.29 mmol) in chlorobenzene (40 mL) was added AIBN (0.011 g, 0.066 mmol). The reaction was heated at 110 °C for 12 h. The solvent was removed under reduced pressure. The residue was solubilized in CH₂Cl₂ (30 mL), washed with water (2 x 30 mL), dried over MgSO₄, filtered, and concentrated under reduced pressure. The crude was purified by flash chromatography (n-Heptane) to give 1.3 g (63%) of a colorless oil. *Rf* = 0.30 (99/1 n-heptane/EtOAc); ¹H NMR (600 MHz, CDCl₃) δ 7.58 (s, 1H), 7.56 (s, 1H), 7.33 (s, 1H), 4.40 (s, 2H), 0.36 (s, 9H); ¹³C NMR (151 MHz, CDCl₃) δ 146.81, 139.51, 138.82, 136.02, 132.72, 118.85, 113.35, 32.05, 0.00.

### 3-(Aminomethyl)-5-(trimethylgermyl)benzonitrile (15)

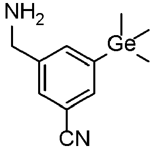

To a solution of the 3-(bromomethyl)-5-(trimethylgermyl)benzonitrile (1.0 g, 3.19 mmol) in THF (20 mL) was added a 7N solution of ammonia in MeOH (12 mL). The reaction was stirred at room temperature for 5 hours. The solution was concentrated and taken up with water (20 mL) and the resulting water phase was extracted with EtOAc (3 x 20 mL). The combined organic portions were washed with brine, dried over MgSO₄, filtered and concentrated in vacuo to give the compound as a crude. Purification by flash chromatography (98/2 CH₂Cl₂/MeOH) gave 0.69 g (87%) of the desired product as a yellow oil. *Rf* = 0.25 (99/1 CH₂Cl₂/MeOH); ¹H NMR (600 MHz, CDCl₃) δ 7.62 (s, 1H), 7.61 (s, 1H), 7.59 (s, 1H), 3.93 (s, 2H), 1.81 (br s, 2H), 0.41 (s, 9H); ¹³C NMR (151 MHz, CDCl₃) δ 146.79, 144.90, 137.98, 136.94, 132.34, 121.15, 114.10, 47.57, 0.00.

### Tert-butyl (3-(1,2,4,5-tetrazin-3-yl)-5-(trimethylgermyl)benzyl)carbamate (16)

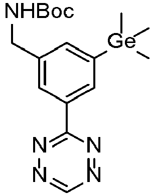

3-(Aminomethyl)-5-(trimethylgermyl)benzonitrile (0.69 g, 2.77 mmol) and triethylamine (3.0 eq.) were dissolved in anhydrous CH₂Cl₂ (10 mL) at 0 °C. To this stirred solution was added di-tert-butyl dicarbonate (1.1 eq.), and the reaction allowed to warm to room temperature and stirred for 12 hours. The reaction mixture was evaporated under reduced pressure, and the residue was re-dissolved in diethyl ether (10 mL), which was washed successively with 0.5 M aq. HCl (2 x 5 mL), saturated NaHCO₃ (2 x 5 mL) and brine (5 mL). The organic layer was dried over anhydrous MgSO₄, filtered and evaporated under reduced pressure to give a white solid. The crude (0.70 gr, 2.00 mmol), CH₂Cl₂ (1 equiv.), sulfur (0.25 equiv.), hydrazine monohydrate (8 equiv.) and ethanol (4.0 mL) were added to a microwave vial equipped with a stir bar. The vessel was sealed, and the reaction mixture was heated to 50 °C for 24 hours, before being allowed to cool to room temperature and unsealed. Then 3 ml of CH₂Cl₂ and NaNO₂ (10 equiv.) in water (40 ml) were added to the now yellow mixture followed by dropwise addition of acetic acid (14 mL), producing a mixture red in colour. The reaction mixture was extracted with CH₂Cl₂, washed with brine, dried with MgSO₄ and filtered before concentrating *in vacuo.* Purification by flash chromatography (95/5 n-Heptane/EtOAc) afforded 0.20 g (18% over 2 steps) of the desired compound as a red solid. *Rf* = 0.32 (n-Heptane/EtOAc 80/20); ¹H NMR (600 MHz, CDCl₃) δ 10.22 (s, 1H), 8.62 (s, 1H), 8.48 (s, 1H), 7.67 (s, 1H), 4.97 (s, 1H), 4.46 (d, *J* = 5.7 Hz, 2H), 1.48 (s, 9H), 0.46 (s, 9H); ¹³C NMR (151 MHz, CDCl₃) δ 168.38, 159.55, 157.69, 146.78, 141.42, 138.52, 133.53, 133.01, 128.69, 81.55, 46.26, 30.14, 0.00.

### (3-(1,2,4,5-Tetrazin-3-yl)-5-(trimethylgermyl)phenyl)methanamine hydrochloride (17)

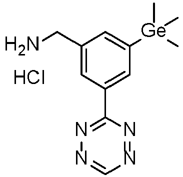

Tert-butyl (3-(1,2,4,5-tetrazin-3-yl)-5-(trimethylgermyl)benzyl)carbamate (0.20 g, 0.49 mmol) was solubilized in a solution of HCl in dioxane (4.0 M, 5.0 mL) was added. The mixture was stirred at room temperature for 2 h. The reaction was then concentrated under reduced pressure to give 0.16 g (95%) of a pink solid. ¹H NMR (400 MHz, MeOD) δ 10.41 (s, 1H), 8.77 (s, 1H), 8.68 (s, 1H), 7.94 (s, 1H), 4.32 (s, 2H), 0.53 (s, 9H); ¹³C NMR (101 MHz, MeOD) δ 166.14, 158.11, 145.59, 137.53, 133.84, 132.67, 132.39, 128.09, 65.49, - 3.33.

### 2,2',2"-(10-(2-((3-(1,2,4,5-Tetrazin-3-yl)-5-(trimethylgermyl)benzyl)amino)-2-oxoethyl)-1,4,7, 10-tetraazacyclododecane-1,4,7-triyl)triacetic acid (Tz-L VII)

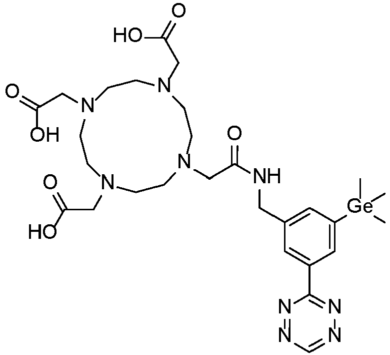

To a solution of (3-(1,2,4,5-tetrazin-3-yl)-5-(trimethylgermyl)phenyl)methanamine hydrochloride (0.04 g, 0.11 mmol) and DIPEA (0.11 mL, 0.59 mmol) in DMF (2 mL) was added DOTA-NHS HPF₆/TFA salt (0.1 g, 0.13 mmol). The reaction was stirred at rt for 12 hours and directly purified by preparative HPLC to give 0.04 g (49%) of the desired compound as a red solid. ¹H NMR (600 MHz, D₂O) δ 10.30 (s, 1H), 8.36 (s, 1H), 8.17 (s, 1H), 7.70 (s, 1H), 4.47 (s, 2H), 4.16 - 3.55 (m, 8H), 3.24 (s, 26H), 0.38 (s, 9H).

### Example 4: Radiolabeling of [⁶⁸Ga]Ga-DOTA-(3-I-H-Tz), from compound 6 (Compound Tz-XLIV, Scheme 4)

### Scheme 4 shows the synthesis of compound Tz-XLIV.

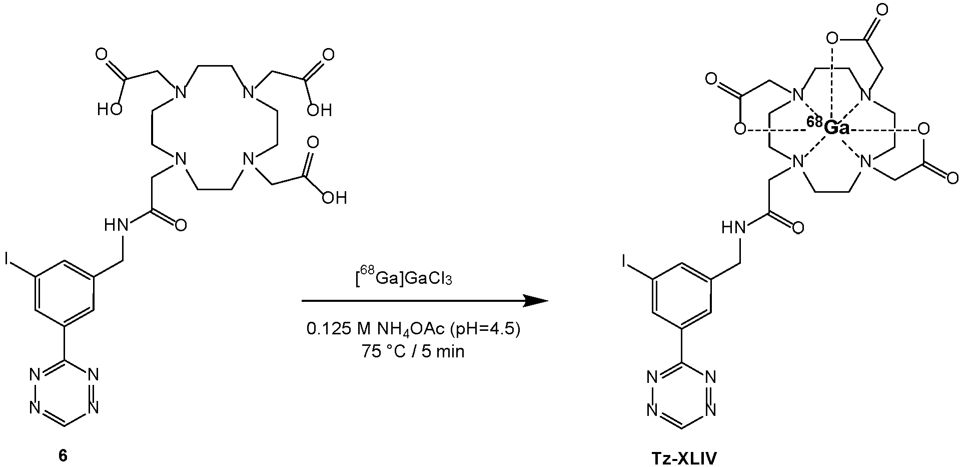

### Radiolabeling of compound Tz-XLIV

2,2',2"-(10-(2-((3-iodo-5-(1,2,4,5-tetrazin-3-yl)benzyl)amino)-2-oxoethyl)-1,4,7,10-tetraazacyclododecane-1,4,7-triyl)triacetic acid ("(3-!-Tz)-DOTA precursor", 5 nmol, 3.5 µg, 1.75 µL of 2 mg/mL stock solution in DMSO) and 125 µL 1 M NHₐOAc (pH = 5.5) was added to a solution of [⁶⁸Ga]GaCl₃ (1000 µL in 0.1 M HCl, 140 MBq) and was heated/shaken at 75 °C for 5 min (RCC > 90%). The reaction solution was rinsed through a Sep-Pak tC2 Plus Light cartridge (Waters; preconditioning: 2 mL EtOH and 5 mL H₂O), washed with 1 mL H₂O and eluted with 0.6 mL ACN + 0.1% TFA to yield [⁶⁸Ga]Ga-DOTA-(3-I-H-Tz) with d.c. RCY = 74% (83 MBq after 20 min synthesis time) and RCP > 95% (determined via radio-HPLC) and confirmed via radio-iTLCs (mobile phases: 0.2 M citrate buffer (pH=4.0) and 1 M NH₄OAc (pH=4.0)/MeOH(1:1)). Analytical radio-HPLC of [⁶⁸Ga]Ga-DOTA-(3-I-H-Tz) indicates a RCC of 92.6% (crude mixture after reaction at 75 °C for 5 min; tᵣₑₜ = 6.44 min) using Luna C18(2) 5µm 150x4.6 mm column; mobile phase H₂O/ACN + 0.1% TFA (10-50% ACN in 8 min); flow rate 1.5 mL/min.

Analytical radio-HPLC of [⁶⁸Ga]Ga-DOTA-(3-I-H-Tz) indicates a RCP of 96.0% (tᵣₑₜ = 6.47 min;) after tC2-SPE purification using Luna C18(2) 5µm 150x4.6 mm column; mobile phase H₂O/ACN + 0.1% TFA (10-50% ACN in 8 min); flow rate 1.5 mL/min.

### Example 5: Radiolabeling of [¹³¹I]I-DOTA-(3-I-H-Tz), from Tz-LVII (Compound Tz-II, scheme 5)

Scheme 5 shows the synthesis of compound **Tz-II.**

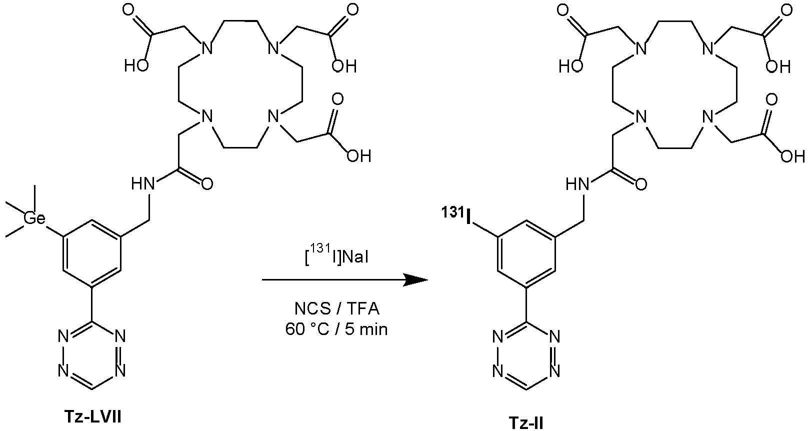

### Radiolabeling of compound Tz-II

[¹³¹I]Nal solution (806 MBq, 25 µL in 0.5 M carbonate buffer pH=10) was evaporated to dryness under nitrogen flow at 60 °C. 2,2',2"-(10-(2-((3-(1,2,4,5-tetrazin-3-yl)-5-(trimethylgermyl)benzyl)amino)-2-oxoethyl)-1,4,7,10-tetraazacyclododecane-1,4,7-triyl)triacetic acid ("DOTA-(3-Ge-H-Tz)", 217 nmol, 150 µg, 15 µL of 10 mg/mL stock solution in MeOH) and NCS (150 nmol, 20 µg, 2 µL of 10 mg/mL solution in MeOH) solutions were mixed shortly before synthesis, evaporated to dryness under nitrogen flow at room temperature, redissolved in TFA (75 µL) and added to the dried [¹³¹I]Nal solution. The reaction solution was heated at 60 °C for 5 min. After cooling down for ca. 1 min, 10 mM Na₂S₂O₃/ACN (1:1, 200 µL) was added. The crude mixture was diluted with ca. 450 µL H₂O and purified via semipreparative HPLC. The product peak was collected (tᵣₑₜ = 11.0-12.0 min), diluted with water (20 mL) and passed through Sep-Pak C18 Plus Light cartridge (Waters; preconditioning: 2 mL EtOH and 5 mL H₂O), washed with 1 mL H₂O and eluted with 0.6 mL ACN + 0.1% TFA to yield [¹³¹I]I-DOTA-(3-I-H-Tz) with RCY = 50.3% (409 MBq). RCP was analyzed via analytical HPLC (RCP = 91.6%) and radio-TLCs using RP-TLC (TLC Silica gel 60 RP-18 F₂₅₄s) with ACN/H2O (1:1) + 0.1% TFA (RCP > 97%, Rf = 0.5-0.6) and NP-TLC (TLC Silica gel 60 F₂₅₄s) using 5% NH4OAc(pH=7)/MeOH (1:3) (RCP > 98%, R_{f} = 0.4-0.5). Semipreparative radio-HPLC of [¹³¹I]I-DOTA-(3-I-H-Tz) indicates a RCC of 64.9% (tᵣₑₜ = 11.2 min;) using Luna C18(2) 5µm 250x10 mm column; mobile phase H₂O/ACN + 0.1% TFA (20-30% ACN in 11 min); flow 4 mL/min. Analytical radio-HPLC of [¹³¹I]I-DOTA-(3-I-H-Tz) indicates a RCP of 91.6% (tᵣₑₜ = 5.3 min) using Luna C18(2) 5µm 150x4.6 mm column; mobile phase H₂O/ACN + 0.1% TFA (10-50% ACN in 8 min); flow 2.0 mL/min.

### Example 6: Radiolabeling of [¹³¹I]I-DOTA-(3-I-H-Tz), from Tz-LXII (Compound Tz-II, scheme 6)

Scheme 6 shows the synthesis of compound **Tz-II.**

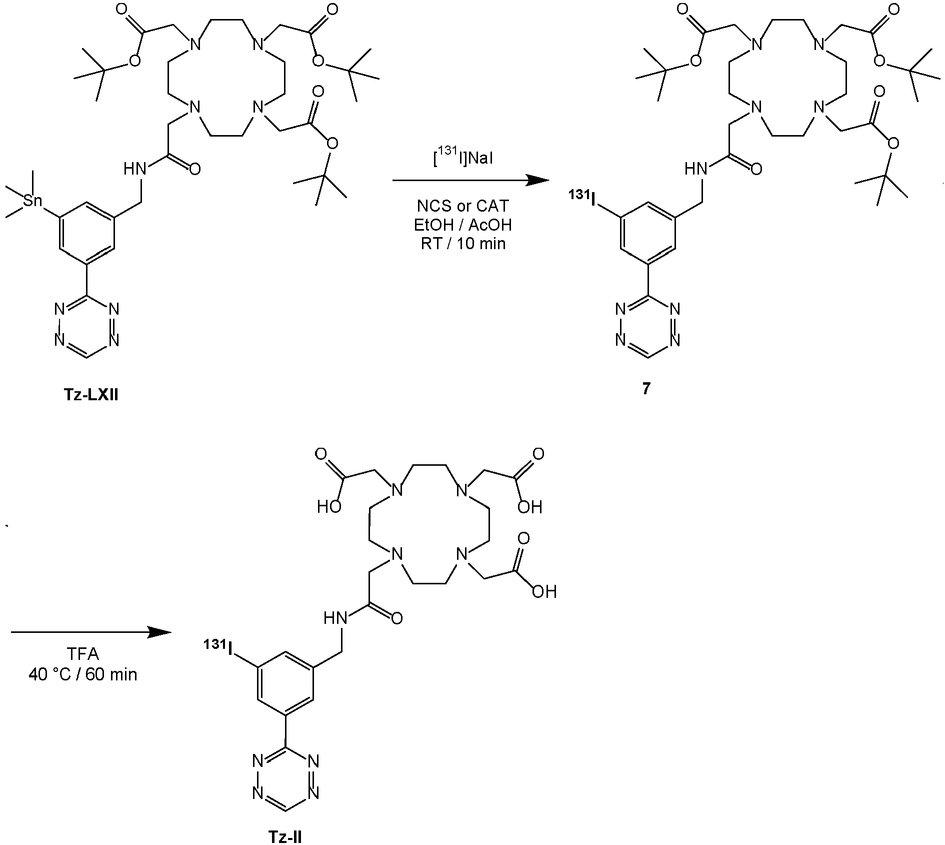

### Radiolabeling of compound Tz-II

### Procedure 1 (NCS):

[¹³¹I]Nal solution (400 MBq, 20 µL in 0.5 M carbonate buffer pH=10) was evaporated to dryness under nitrogen flow at 40 °C. Tri-tert-butyl 2,2',2"-(10-(2-((3-(1,2,4,5-tetrazin-3-yl)-5-(trimethylstannyl)benzyl)amino)-2-oxoethyl)-1,4,7,10-tetraazacyclododecane-1,4,7-triyl)triacetate ("DOTA(3tBu)-(3-Sn-H-Tz)", 110 nmol, 100 µg) was dissolved in EtOH (40 µL). Acetic acid (1 µL) and N-chlorosuccinimide (NCS, 150 nmol, 20 µg, 2 µL of 10 mg/mL solution in MeOH) were added to the DOTA(3tBu)-(3-Sn-H-Tz) solution and the mixture was finally added to the dried [¹³¹I]Nal solution. After reaction for 10 min at room temperature, the crude reaction mixture was evaporated to dryness at 40 °C and redissolved in TFA (50 µL). The deprotection was carried out at 40 °C for 60 min. The crude mixture was diluted with 100 µL ACN and 500 µL H₂O and purified via semipreparative HPLC. The product peak was collected (tᵣₑₜ = 10.5-11.3 min, A= 106 MBq, RCY = 26.5%), diluted with water (10 mL) and passed through Sep-Pak C18 Plus Light cartridge (Waters; preconditioning: 2 mL EtOH and 5 mL H₂O), washed with 1 mL H₂O and eluted with 0.6 mL ACN + 0.1% TFA to yield [¹³¹I]I-DOTA-(3-I-H-Tz) with RCY = 22.7% (90.6 MBq).

RCP was analyzed via analytical HPLC (RCP = 96.1%) and confirmed via radio-TLCs (RP- and NP-TLC), using the same reported conditions as in Example 5.

Semipreparative radio-HPLC of [¹³¹I]I-DOTA-(3-I-H-Tz) indicates a RCC of 39.2% (tᵣₑₜ = 10.75 min;) using Luna C18(2) 5µm 250x10 mm column; mobile phase H₂O/ACN + 0.1% TFA (20-30% ACN in 11 min); flow 4 mL/min. Analytical radio-HPLC of [¹³¹I]I-DOTA-(3-I-H-Tz) indicates a RCP of 96.1% (tᵣₑₜ = 9.2 min;) using Luna C18(2) 5µm 100x4.6 mm column; mobile phase H₂O/ACN + 0.1% TFA (0-60% ACN in 11 min); flow 0.5 mL/min.

### Procedure 2 (CAT):

[¹³¹I]Nal solution (390 MBq, 20 µL in 0.5 M carbonate buffer pH=10) was evaporated to dryness under nitrogen flow at 40 °C. Tri-tert-butyl 2,2',2"-(10-(2-((3-(1,2,4,5-tetrazin-3-yl)-5-(trimethylstannyl)benzyl)amino)-2-oxoethyl)-1,4,7,10-tetraazacyclododecane-1,4,7-triyl)triacetate ("DOTA(3tBu)-(3-Sn-H-Tz)", 110 nmol, 100 µg) was dissolved in EtOH (40 µL). Acetic acid (1 µL) and chloramine-T (CAT, 88 nmol, 20 µg, 1 µL of 20 mg/mL solution in MeOH) were added to the DOTA(3*t*Bu)-(3-Sn-H-Tz) solution and the mixture was finally added to the dried [¹³¹I]Nal solution. After reaction for 10 min at room temperature, the crude reaction mixture was evaporated to dryness at 40 °C and redissolved in TFA (50 µL). The deprotection was carried out at 40 °C for 60 min. The crude mixture was diluted with 100 µL ACN and 500 µL H₂O and purified via semipreparative HPLC. The product peak was collected (tᵣₑₜ = 10.8-11.5 min, A = 110 MBq, RCY = 28.2%), diluted with water (10 mL) and passed through Sep-Pak C18 Plus Light cartridge (Waters; preconditioning: 2 mL EtOH and 5 mL H₂O), washed with 1 mL H₂O and eluted with 0.6 mL ACN + 0.1% TFA to yield [¹³¹I]I-DOTA-(3-I-H-Tz) with RCY = 25.4% (99.0 MBq). Semipreparative radio-HPLC of [¹³¹I]I-DOTA-(3-I-H-Tz) indicates a RCC of 53.8% (tᵣₑₜ = 11.0 min) using Luna C18(2) 5µm 250x10 mm column; mobile phase H₂O/ACN + 0.1% TFA (20-30% ACN in 11 min); flow 4 mL/min.

### Example 7: Radiolabeling of [¹³¹I]I-DOTA-(3-I-H-Tz), from Tz-LVI (Compound Tz-II, scheme 7)

Scheme 7 shows the synthesis of compound **Tz-II.**

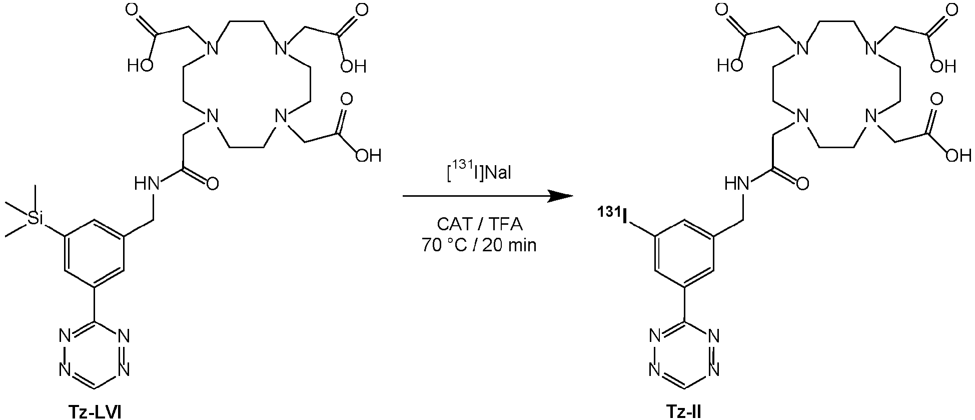

### Radiolabeling of compound Tz-II

2,2',2"-(10-(2-((3-(1,2,4,5-tetrazin-3-yl)-5-(trimethylsilyl)benzyl)amino)-2-oxoethyl)-1,4,7,10-tetraazacyclododecane-1,4,7-triyl)triacetic acid ("DOTA-(3-Si-H-Tz)", 155 nmol, 100 µg, 20 µL of 5 mg/mL stock solution in MeOH) and chloramine-T (CAT, 110 nmol, 25 µg, 1.25 µL of 20 mg/mL solution in MeOH) solutions were mixed shortly before synthesis, evaporated to dryness under nitrogen flow at room temperature, redissolved in TFA (100 µL) and [¹³¹I]Nal solution (43.9 MBq, 10 µL in 0.5 M carbonate buffer pH=10) was added. The reaction solution was heated at 70 °C for 20 min (RCC(HPLC) = 41.9%, RCC(NP-TLC = 45%). After cooling down for ca. 1 min, the crude mixture was diluted with 1000 µL H₂O. 500 µL were taken and purified via semipreparative HPLC (tᵣₑₜ = 7.75-8.25 min) to yield [¹³¹I]I-DOTA-(3-I-H-Tz) (3.19 MBq, RCY = 14.5%, RCP = 96.1%). Semipreparative radio-HPLC of [¹³¹I]I-DOTA-(3-I-H-Tz) indicates a RCC of 41.9% (tᵣₑₜ = 7.8 min) using Luna C18(2) 5µm 250x10 mm column; mobile phase H₂O/ACN + 0.1% TFA (25-30% ACN in 11 min); flow 4 mL/min. Analytical radio-HPLC of [¹³¹I]I-DOTA-(3-I-H-Tz) indicates a RCP of 96.5% (tᵣₑₜ = 8.4 min) using Luna C18(2) 5µm 100x4.6 mm column; mobile phase H₂O/ACN + 0.1% TFA (10-40% ACN in 11 min); flow 0.5 mL/min.

### Example 8: Radiolabeling of [²¹¹At]At-DOTA-(3-At-H-Tz), from Tz-LVII (Compound Tz-III, scheme 8)

Scheme 8 shows the synthesis of compound [²¹¹At]**Tz-III.**

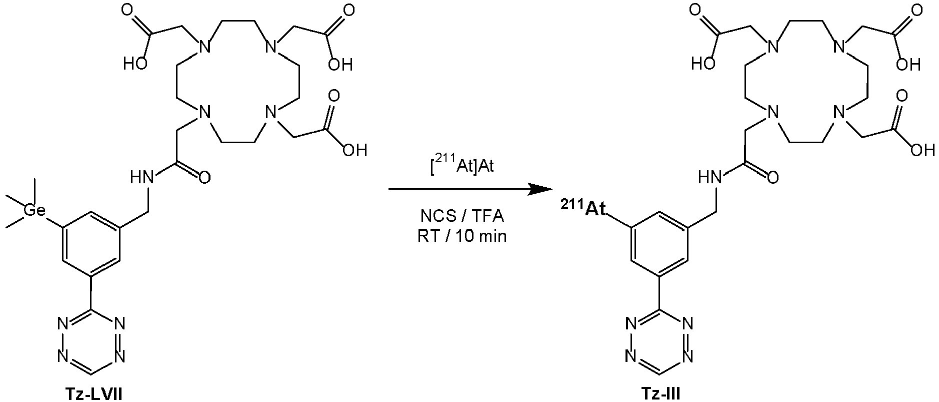

### Radiolabeling of compound Tz-III

[²¹¹At]At (ca. 10 MBq, 10 µL in chloroform) was evaporated to dryness under nitrogen flow. 2,2',2"-(10-(2-((3-(1,2,4,5-tetrazin-3-yl)-5-(trimethylgermyl)benzyl)amino)-2-oxoethyl)-1,4,7,10-tetraazacyclododecane-1,4,7-triyl)triacetic acid ("DOTA-(3-Ge-H-Tz)", 72.3 nmol, 50 µg, 10 µL of 5 mg/mL stock solution in MeOH), acetic acid (1 µL) and *N-*chlorosuccinimide (NCS, 150 nmol, 20 µg, 1 µL of 20 mg/mL solution in MeOH) was added. The mixture was evaporated to dryness under nitrogen flow again and the residue was redissolved in TFA (25 µL) and left at room temperature for 10 min to yield [²¹¹At]At-DOTA-(3-At-H-Tz) with a RCC(RP-TLC) = 61.2 ± 19.9% (n=3) and RCC(HPLC) = 71.5 ± 13.6% (n=3), respectively. Analytical radio-HPLC of [²¹¹At]At-DOTA-(3-At-H-Tz) indicates a RCC of 82.7% (tᵣₑₜ = 3.7 min) using Luna C18(2) 5µm 100x4.6 mm column; mobile phase H₂O/ACN + 0.1% TFA (0-100% ACN in 8 min); flow 1.5 mL/min.

### Example 9: Radiolabeling of [²¹¹At]At-DOTA-(3-At-H-Tz), from Tz-LXII (Compound Tz-III , scheme 9)

Scheme 9 shows the synthesis of compound **Tz-III.**

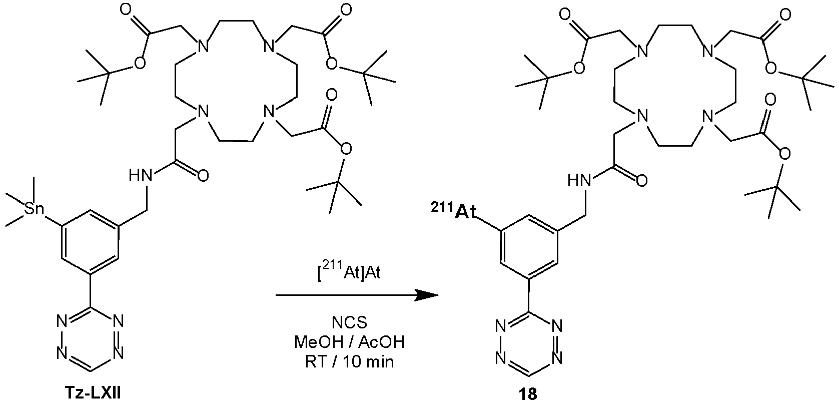

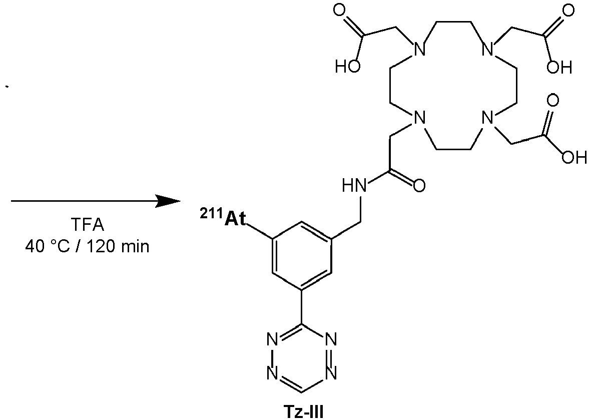

### Radiolabeling of compound [²¹¹At]Tz-III

[²¹¹At]At (ca. 10 MBq, 10 µL in chloroform) was evaporated to dryness under nitrogen flow. Tri-tert-butyl 2,2',2"-(10-(2-((3-(1,2,4,5-tetrazin-3-yl)-5-(trimethylstannyl)benzyl)amino)-2-oxoethyl)-1,4,7,10-tetraazacyclododecane-1,4,7-triyl)triacetate ("DOTA(3tBu)-(3-Sn-H-Tz)", 55 nmol, 50 µg, 10 µL 5 mg/mL in MeOH) was added, followed by MeOH (15 µL), acetic acid (1 µL) and *N*-chlorosuccinimide (NCS, 150 nmol, 20 µg, 1 µL of 20 mg/mL solution in MeOH). The mixture was left at room temperature for 10 min (RCC(RP-TLC) = 77.1%, RCC(HPLC) = 89.8%, evaporated to dryness afterwards and then redissolved in TFA (100 µL). The deprotection was carried out at 40 °C for 120 min and yielded [²¹¹At]At-DOTA-(3-At-H-Tz) with a RCC(RP-TLC) = 85.7%) and RCC(HPLC) = 72.0%, respectively. Analytical radio-HPLC of [²¹¹At]At-DOTA(3tBu)-(3-At-H-Tz) indicates a RCC of 89.8% (tᵣₑₜ = 5.8 min) using Luna C18(2) 5µm 100x4.6 mm column; mobile phase H₂O/ACN + 0.1% TFA (0-100% ACN in 8 min); flow 1.5 mL/min. Analytical radio-HPLC of [²¹¹At]At-DOTA-(3-At-H-Tz) indicates a RCC of 72.0% (tᵣₑₜ = 3.7 min) using Luna C18(2) 5µm 100x4.6 mm column; mobile phase H₂O/ACN + 0.1% TFA (0-100% ACN in 8 min); flow 1.5 mL/min.

### Example 10: Radiolabeling of [²¹¹At]At-DOTA-(3-At-H-Tz), from Tz-LVI (Compound Tz-III , scheme 10)

Scheme 10 shows the synthesis of compound **Tz-III.**

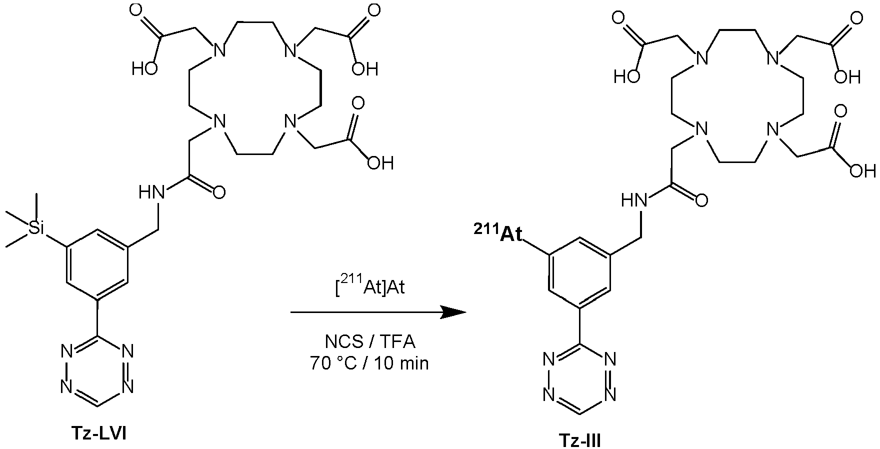

### Radiolabeling of compound Tz-III

[²¹¹At]At (ca. 10 MBq, 10 µL in chloroform) was evaporated to dryness under nitrogen flow. 2,2',2"-(10-(2-((3-(1,2,4,5-tetrazin-3-yl)-5-(trimethylsilyl)benzyl)amino)-2-oxoethyl)-1,4,7,10-tetraazacyclododecane-1,4,7-triyl)triacetic acid ("DOTA-(3-Si-H-Tz)", 77.5 nmol, 50 µg, 10 µL of 5 mg/mL stock solution in MeOH), acetic acid (1 µL) and *N-*chlorosuccinimide (NCS, 150 nmol, 20 µg, 1 µL of 20 mg/mL solution in MeOH) was added. The mixture was evaporated to dryness under nitrogen flow again and the residue was redissolved in TFA (25 µL) and heated at 70 °C for 10 min to yield [²¹¹At]At-DOTA-(3-At-H-Tz) with a RCC(RP-TLC) = 48.2 ± 15.3% (n=2) and RCC(HPLC) = 39.3 ± 7.9% (n=2), respectively. Analytical radio-HPLC of [²¹¹At]At-DOTA-(3-At-H-Tz) indicates a RCC of 44.9% (tᵣₑₜ = 3.7 min;) using Luna C18(2) 5µm 100x4.6 mm column; mobile phase H₂O/ACN + 0.1% TFA (0-100% ACN in 8 min); flow 1.5 mL/min.

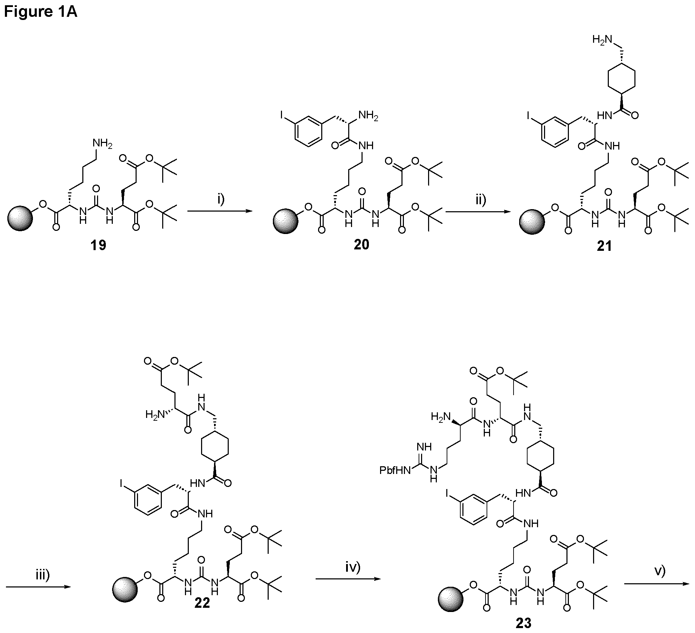

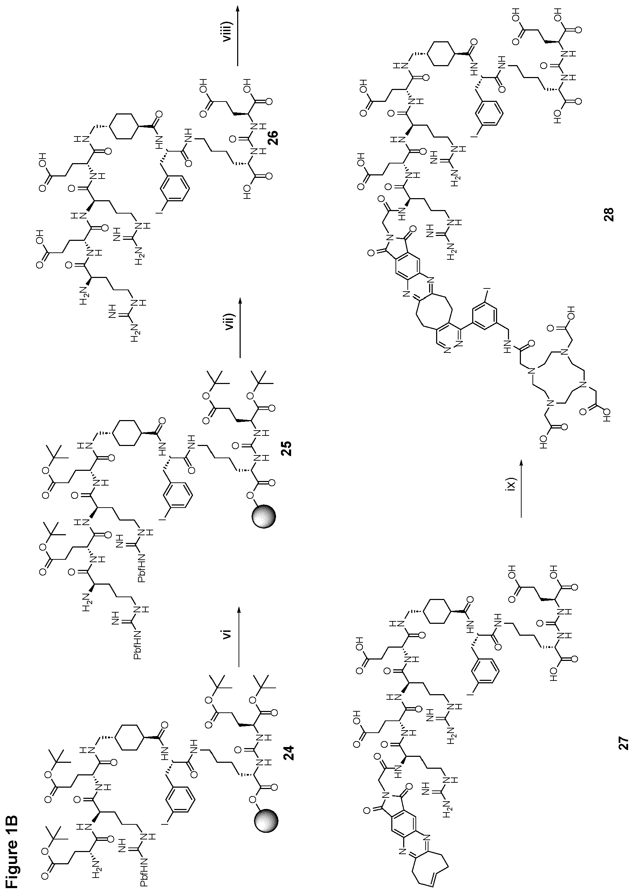

### Example 11: Synthesis of compound 28 (Figure 1)

Figure 1A and 1B shows the synthesis of compounds **28.** The grey dot in the intermediate structures symbolizes a resin. The following reagents and conditions were applied in steps i) - x): i) a) Fmoc-Phe(3-I)-OH, HATU, DIPEA, DMF, rt, 2.5h, b) 50% piperidine in DMF, rt, 20 min.; ii) Fmoc-(4-Amb)-OH, HATU, DIPEA, DMF, rt, 2.5h, b) 50% piperidine in DMF, rt, 20 min; iii) a) Fmoc-D-Glu(OtBu)-OH, HATU, DIPEA, DMF, rt, 2.5h, b) 50% piperidine in DMF, rt, 20 min.; iv) a) Fmoc-D-Arg(Pbf)-OH, HATU, DIPEA, DMF, rt, 2.5h, b) 50% piperidine in DMF, rt, 20 min; v) a) Fmoc-D-Glu(OtBu)-OH, HATU, DIPEA, DMF, rt, 2.5h, b) 50% piperidine in DMF, rt, 20 min.; vi) a) Fmoc-D-Arg(Pbf)-OH, HATU, DIPEA, DMF, rt, 2.5h, b) 50% piperidine in DMF, rt, 20 min; vii) TFA, TIPS, H₂O, rt, 2h; viii) **29,** 4-methylmorpholine, DMF, rt, 12h, ix) 6, ACN, H₂O, TFA, rt, 12h.

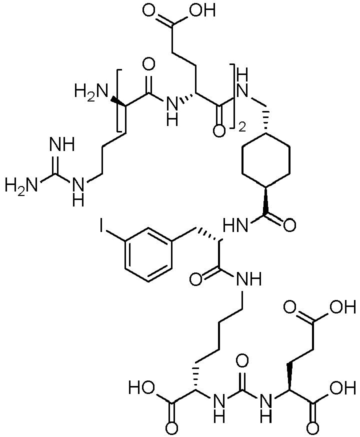

The resin-bound and tert-butyl-protected binding motif (19) was synthesized as previously described. Relative to the resin (0.22 mmol), 4 equiv. of Fmoc-L-3-I-Phe-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized Glutamate-urea-Lysine and shaken for 1 hour. The Fmoc-protecting group was cleaved with a mixture of DMF and piperidine (1:1). The product (**20**) was reacted with 4 equiv. of trans-4-(Fmoc-aminomethyl)cyclohexanecarboxylic acid (N-Fmoctranexamic acid) activated with 3.92 equiv. of HATU and 4 equiv. of DIPEA. Deprotection of the Fmoc group with a mixture of DMF and piperidine (1:1) gave **21.** Relative to this this intermediate (0.22 mmol), 4 equiv. of Fmoc-D-Glu(OtBu)-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized peptideand shaken for 1 hour. The Fmoc-protecting group was cleaved with a mixture of DMF and piperidine (1:1). The product was reacted with 4 equiv. of Fmoc-D-Arg(Pbf)-OH activated with 3.92 equiv. of HATU and 4 equiv. of DIPEA. The coupling with Fmoc-D-Glu(OtBu)-OH and Fmoc-D-Arg(Pbf)-OH was repeated and final deprotection of the Fmoc group with a mixture of DMF and piperidine (1:1) gave **25.** The product was cleaved from the resin and deprotected with trifluoroacetic acid (TFA), triisopropylsilane (TIPS) and water (95:2.5:2.5). Preparative HPLC afforded 0.09 g of the desired compound **26.** ESI-MS [M+H]⁺ = 1302.5.

### Synthesis of compound 27 (Scheme 1B)

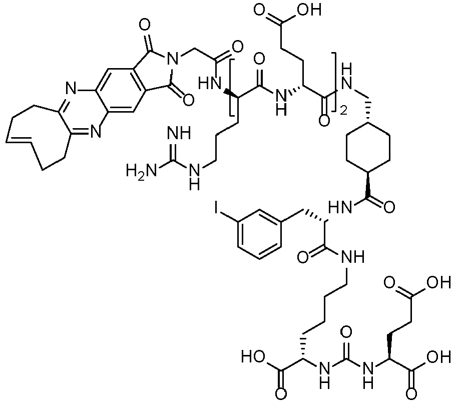

2,5-dioxopyrrolidin-1-yl (E)-2-(1,3-dioxo-1,3,6,7,10,11-hexahydro-2H-cycloocta[b]pyrrolo[3,4-g]quinoxalin-2-yl)acetate (Compound **29**) was synthesized as previously reported to give 0.056 g (72%) of the desired compound as a yellow solid (PCT/EP2023/055930). To a solution of **26** (0.05 g, 0.031 mmol) and **29** (0.015 g, 0.036 mmol) in dry DMF (3 mL) under argon, was added 4-methylmorpholine (0.036 mL, 0.33 mmol). The reaction was stirred at room temperature for 6 h. The mixture was then diluted with 10 mL of mobile phase A (1%TFA in H₂O) and purified by preparative HPLC. The collected fractions were freeze-dried to give 0.021 g (37%) of the desired compound as a white solid. ESI-MS [M+2H]²⁺ = 811.6.

### Synthesis of compound 28 (Figure 1B)

To a solution of **27** (0.005 g, 0.0027 mmol) in ACN (2 mL) and 1% TFA in H₂O (2 mL) was added **6** (0.001 g, 0.0027 mmol). The reaction was stirred at room temperature for 24 h. The mixture was directly purified by preparative HPLC. The collected fractions were freeze-dried to give 0.002 g (32%) of the desired compound as a white solid. ESI-MS [M+2H]²⁺ = 1146.4.

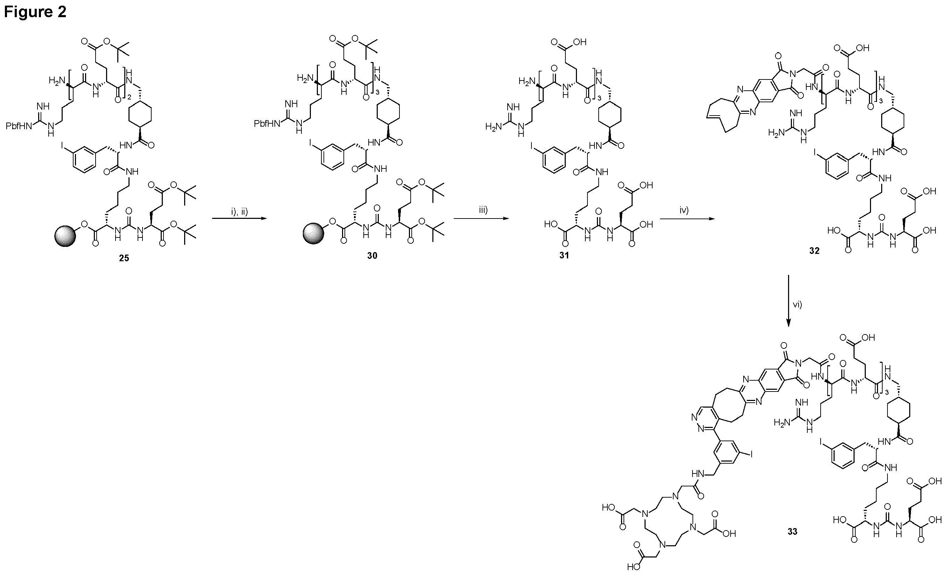

### Example 12: Synthesis of compound 33 (Figure 2)

Figure 2 shows the synthesis of compounds **33.** The grey dot in the intermediate structures symbolizes a resin. The following reagents and conditions were applied in steps i) - vi): i)a) Fmoc-D-Glu(OtBu)-OH, HATU, DIPEA, DMF, rt, 2 h, b) 50% piperidine in DMF, rt, 20 min.; ii)a) Fmoc-D-Arg(Pbf)-OH, HATU, DIPEA, DMF, rt, 2 h, b) 50% piperidine in DMF, rt, 20 min.; iii) TFA, TIPS, H₂O, rt, 2 h; iv) **5,** 4-methylmorpholine, DMF, rt, 6 h; v) **1,** H₂O, TFA, rt, 5 h; vi) **6,** H₂O, TFA, rt, 5 h.

### Synthesis of compound 31 (Figure 2)

Relative to the resin and **25** (0.22 mmol), 4 equiv. of Fmoc-D-Glu(OtBu)-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized Glutamate-urea-Lysine and shaken for 1 hour. The Fmoc-protecting group was cleaved with a mixture of DMF and piperidine (1:1). The product was reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave **30.** The product was cleaved from the resin and deprotected with trifluoroacetic acid (TFA), triisopropylsilane (TIPS) and water (95:2.5:2.5). Preparative HPLC afforded 0.12 g of the desired compound **31.** ESI-MS [M+2H]²⁺ = 794.5.

### Synthesis of compound 32 (Figure 2)

To a solution of **31** (0.11 g, 0.053 mmol) and **29** (0.023 g, 0.053 mmol) in dry DMF (6 mL) under argon, was added 4-methylmorpholine (0.064 mL, 0.58 mmol). The reaction was stirred at room temperature for 6 h. The mixture was then diluted with 10 mL of mobile phase A (1%TFA in H₂O) and purified by preparative HPLC. The collected fractions were freeze-dried to give 0.061 g (51%) of the desired compound as a white solid. ESI-MS [M+3H]³⁺ = 954.0.

### Synthesis of compound 33 (Figure 2)

To a solution of **32** (0.012 g, 0.0053 mmol) in ACN (2 mL) and 1% TFA in H₂O (2 mL) was added **6** (0.005 g, 0.0053 mmol). The reaction was stirred at room temperature for 24 h. The mixture was directly purified by preparative HPLC. The collected fractions were freeze-dried to give 0.007 g (49%) of the desired compound as a white solid. ESI-MS [M+3H]³⁺ = 859.6.

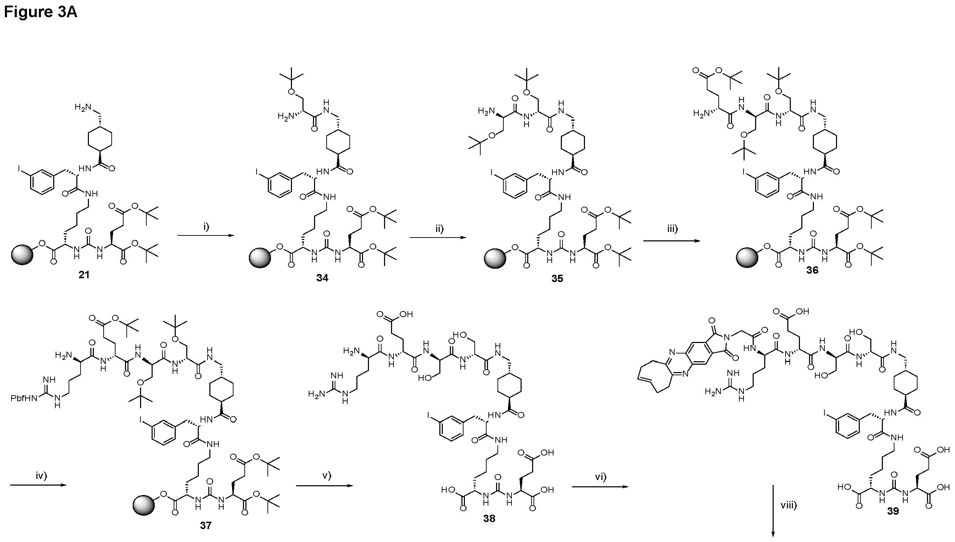

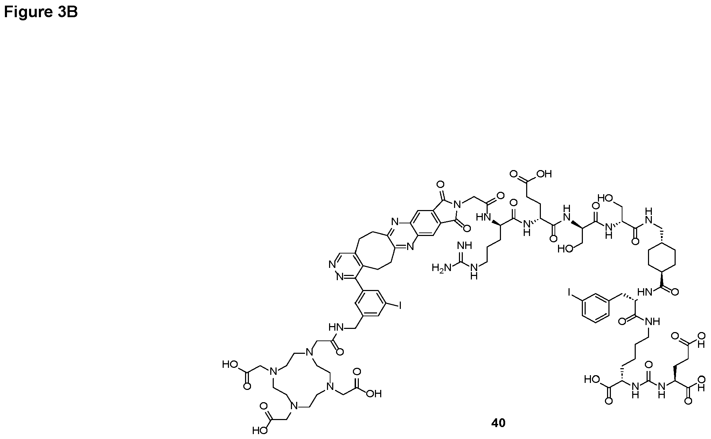

### Example 13: Synthesis of compound 40 (Figure 3)

Figure 3 shows the synthesis of compound **40.** The grey dot in the intermediate structures symbolizes a resin. The following reagents and conditions were applied in steps i) - viii): i) a) Fmoc-D-Ser(tBu)-OH, HATU, DIPEA, DMF, rt, 2.5h, b) 50% piperidine in DMF, rt, 20 min.; ii) a) Fmoc-D-Ser(tBu)-OH, HATU, DIPEA, DMF, rt, 2.5h, b) 50% piperidine in DMF, rt, 20 min; iii) a) Fmoc-D-Glu(OtBu)-OH, HATU, DIPEA, DMF, rt, 2.5h, b) 50% piperidine in DMF, rt, 20 min.; iv) a) Fmoc-D-Arg(Pbf)-OH, HATU, DIPEA, DMF, rt, 2.5h, b) 50% piperidine in DMF, rt, 20 min; v) TFA, TIPS, H₂O, rt, 2h; vi) 5, 4-methylmorpholine, DMF, rt, 6 h; vii) 6, H₂O, TFA, rt, 5 h.

### Synthesis of compound 38 (Figure 3)

Relative to **21** (0.22 mmol), 4 equiv. of Fmoc-D-Ser(tBu)-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized peptide and shaken for 1 hour. The Fmoc-protecting group was cleaved with a mixture of DMF and piperidine (1:1). The coupling with Fmoc-D-Ser(tBu)-OH and the relative deprotection were repeated an additional time. 71 was then reacted with 4 equiv. of Fmoc-D-Glu(OtBu)-OH activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized peptide and shaken for 1 hour. The Fmoc-protecting group was cleaved with a mixture of DMF and piperidine (1:1). Afterwards, the product was reacted with 4 equiv. of Fmoc-D-Arg(Pbf)-OH activated with 3.92 equiv. of HATU and 4 equiv. of DIPEA. The final deprotection of the Fmoc group with a mixture of DMF and piperidine (1:1) gave **37.** The latter was cleaved from the resin and deprotected with trifluoroacetic acid (TFA), triisopropylsilane (TIPS) and water (95:2.5:2.5). Preparative HPLC afforded 0.09 g of the desired compound **38.** ESI-MS [M+H]⁺ = 1191.4.

### Synthesis of compound 39 (Figure 3)

To a solution of **38** (0.061 g, 0.042 mmol) and **29** (0.022 g, 0.051 mmol) in dry DMF (6 mL) under argon, was added 4-methylmorpholine (0.042 mL, 0.38 mmol). The reaction was stirred at room temperature for 6 h. The mixture was then diluted with 10 mL of mobile phase A (1%TFA in H₂O) and purified by preparative HPLC. The collected fractions were freeze-dried to give 0.035 g (53%) of the desired compound as a white solid. ESI-MS [M+2H]²⁺ = 756.2

### Synthesis of compound 40 (Figure 3)

To a solution of **75** (0.005 g, 0.003 mmol) in ACN (2 mL) and 1% TFA in H₂O (2 mL) was **6** (0.002 g, 0.003 mmol). The reaction was stirred at room temperature for 24 h. The mixture was directly purified by preparative HPLC. The collected fractions were freeze-dried to give 0.005 g (83%) of the desired compound as a white solid. ESI-MS [M+2H]²⁺ = 1090.5.

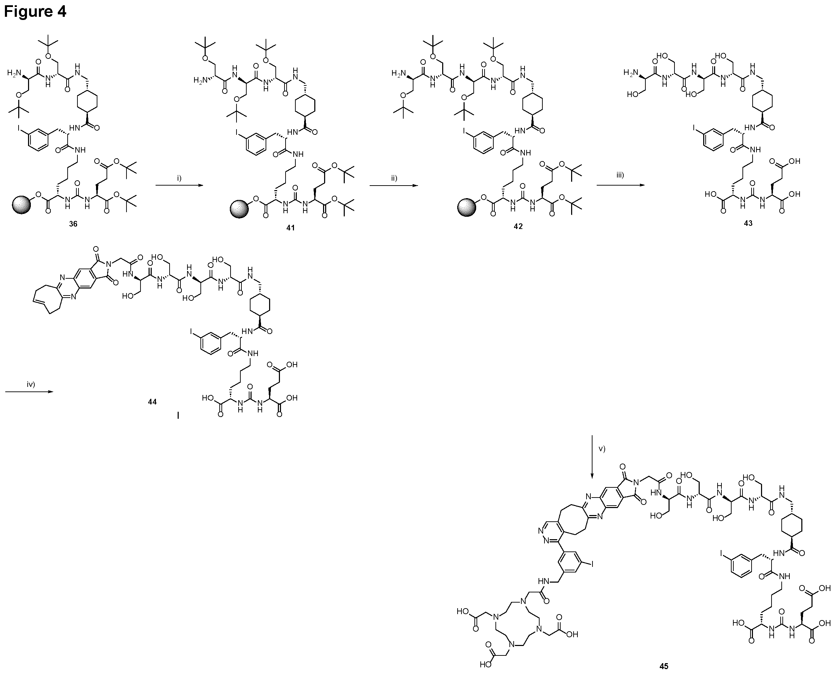

### Example 14: Synthesis of compound 45 (Figure 4)

Figure 4 shows the synthesis of compound **45.** The grey dot in the intermediate structures symbolizes a resin. The following reagents and conditions were applied in steps i) - vi): i) a) Fmoc-D-Ser(tBu)-OH, HATU, DIPEA, DMF, rt, 2.5h, b) 50% piperidine in DMF, rt, 20 min.; ii) a) Fmoc-D-Ser(tBu)-OH, HATU, DIPEA, DMF, rt, 2.5h, b) 50% piperidine in DMF, rt, 20 min; iii) TFA, TIPS, H₂O, rt, 2h; iv) **29,** 4-methylmorpholine, DMF, rt, 6 h; v) **6,** H₂O, TFA, rt, 5 h.

### Synthesis of compound 43 (Figure 4)

Relative to **36** (0.22 mmol), 4 equiv. of Fmoc-D-Ser(tBu)-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized peptide and shaken for 1 hour. The Fmoc-protecting group was cleaved with a mixture of DMF and piperidine (1:1). The coupling with Fmoc-D-Ser(tBu)-OH and the relative deprotection were repeated an additional time. The peptide was cleaved from the resin and deprotected with trifluoroacetic acid (TFA), triisopropylsilane (TIPS) and water (95:2.5:2.5). Preparative HPLC afforded 0.08 g of the desired compound **43.** ESI-MS [M+H]⁺ = 1080.3.

### Synthesis of compound 44 (Figure 4)

To a solution of **43** (0.03 g, 0.025 mmol) and **29** (0.013 g, 0.030 mmol) in dry DMF (6 mL) under argon, was added 4-methylmorpholine (0.025 mL, 0.22 mmol). The reaction was stirred at room temperature for 6 h. The mixture was then diluted with 10 mL of mobile phase A (1%TFA in H₂O) and purified by preparative HPLC. The collected fractions were freeze-dried to give 0.012 g (34%) of the desired compound as a white solid. ESI-MS [M+H]⁺ = 1399.5.

### Synthesis of compound 45 (Figure 4)

To a solution of **43** (0.0055 g, 0.0039 mmol) in ACN (2 mL) and 1% TFA in H₂O (2 mL) was added **6** (0.003 g, 0.0039 mmol). The reaction was stirred at room temperature for 24 h. The mixture was directly purified by preparative HPLC. The collected fractions were freeze-dried to give 0.003 g (37%) of the desired compound as a white solid. ESI-MS [M+2H]²⁺ = 1034.9.

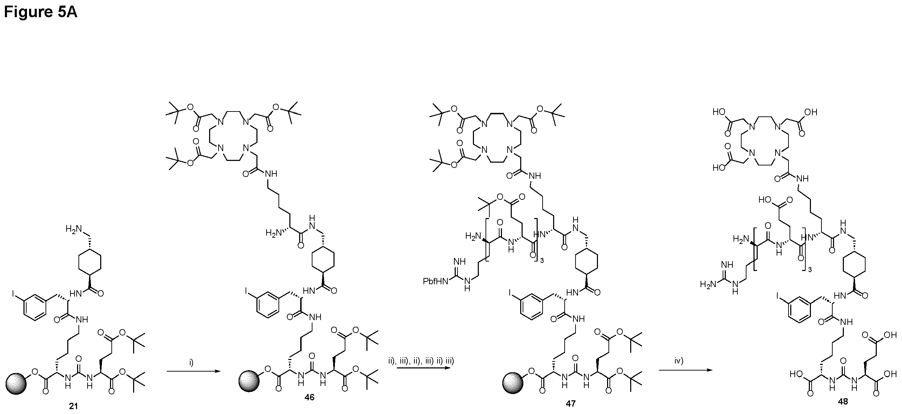

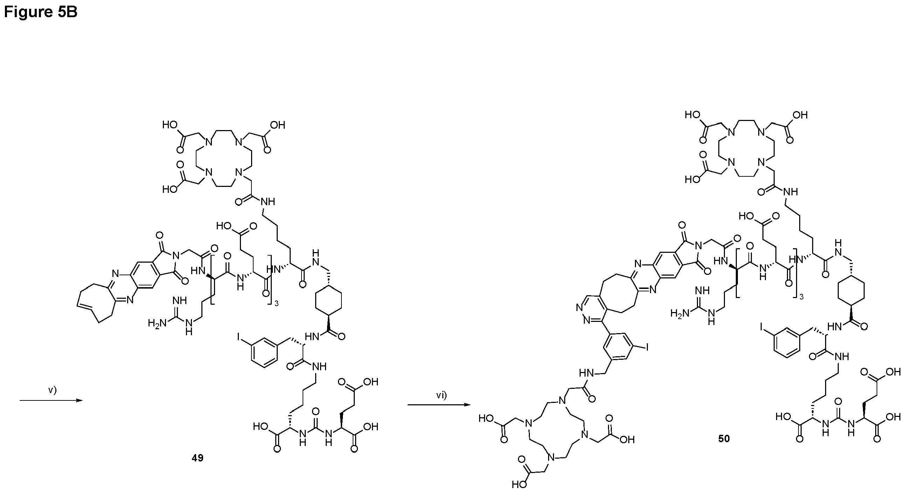

### Example 15: Synthesis of compound 50 (Figure 5A and 5B)

Figure 5A and 5B show the synthesis of compound **50.** The grey dot in the intermediate structures symbolizes a resin. The following reagents and conditions were applied in steps i) - vi): i) a) Fmoc-tBuDOTA-L-Lys-OH, HATU, DIPEA, DMF, rt, 2 h, b) 50% piperidine in DMF, rt, 20 min.; ii) a) Fmoc-D-Glu(OtBu)-OH, HATU, DIPEA, DMF, rt, 2.5h, b) 50% piperidine in DMF, rt, 20 min.; iii) a) Fmoc-D-Arg(Pbf)-OH, HATU, DIPEA, DMF, rt, 2.5h, b) 50% piperidine in DMF, rt, 20 min; iv) TFA, TIPS, H₂O, rt, 2h; v) **29,** 4-methylmorpholine, DMF, rt, 6 h; vi) **6,** H₂O, TFA, rt, 5 h.

### Synthesis of compound 48 (Figure 5A)

Relative to the resin and **21** (0.22 mmol), 1.4 equiv. of Fmoc-tBuDOTA-L-Lys-OH were activated with 1.2 equiv. of HATU and 4.2 equiv. of DIPEA. Deprotection of the Fmoc group with a mixture of DMF and piperidine (1:1) gave the deprotected intermediate **46.** Relative to this, 4 equiv. of Fmoc-D-Glu(OtBu)-OH were activated with 3.92 equiv. of HATU (1-[Bis(dimethylamino)methylene]-1H-1,2,3-triazolo[4,5-b]pyridinium 3-oxide hexafluorophosphate and 4 equiv. of DIPEA in DMF. After 2 min, the solution was added to the resin-immobilized Glutamate-urea-Lysine and shaken for 1 hour. The Fmoc-protecting group was cleaved with a mixture of DMF and piperidine (1:1). The product was reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave the deprotected compound. The coupling with Fmoc-D-Glu(OtBu)-OH and Fmoc-D-Arg(Pbf)-OH and the relative deprotection was repeated twice to give **47.** The product was cleaved from the resin and deprotected with trifluoroacetic acid (TFA), triisopropylsilane (TIPS) and water (95:2.5:2.5). Preparative HPLC afforded 0.09 g of the desired compound **48.** ESI-MS [M+2H]²⁺ = 1051.5.

### Synthesis of compound 49 (Figure 5B)

To a solution of **48** (0.082 g, 0.039 mmol) and **29** (0.017 g, 0.039 mmol) in dry DMF (6 mL) under argon, was added 4-methylmorpholine (0.064 mL, 0.58 mmol). The reaction was stirred at room temperature for 6 h. The mixture was then diluted with 10 mL of mobile phase A (1%TFA in H₂O) and purified by preparative HPLC. The collected fractions were freeze-dried to give 0.04 g (41%) of the desired compound as a white solid. ESI-MS [M+2H]²⁺ = 1211.3.

### Synthesis of compound 50 (Figure 5B)

To a solution of **49** (0.03 g, 0.012 mmol) in ACN (2 mL) and 1% TFA in H₂O (2 mL) was added 6 (0.01 g, 0.014 mmol). The reaction was stirred at room temperature for 24 h. The mixture was directly purified by preparative HPLC. The collected fractions were freeze-dried to give 0.013 g (34%) of the desired compound as a white solid. ESI-MS [M+2H]²⁺ = 1546.1.

### Example 16: General procedure for autoradiography for affinity measurement

The affinity of PSMA derivatives towards PSMA was measured in an in vitro autoradiography competition assay on rat brain slices against [¹¹¹In]In-PSMA-617 as a radioligand.

[¹¹¹In]In-PSMA-617 was prepared by mixing 2 µL of 2 mg/mL vipivotide tetraxetan (MedChemExpress, USA) solution in milliQ water with ¹¹¹InCl₃ (5-20 MBq) buffered to pH 5.5 with ammonium acetate (-0.2M final ammonium acetate concentration, 40-70 µL final volume) and heating the resulting mixture at 60°C for 5 min. This method provided [¹¹¹In]-PSMA-617 in >95% radiochemical yield, as determined by reverse-phase HPLC.

Rat brain slices were obtained from female Sprague-Dawley rats (weight~250g; Taconic Biosciences). The rats were sacrificed by decapitation, brains were extracted, frozen at - 80 °C and stored at the same temperature. To prepare slices for autoradiography, brains were cut into halves along the sagittal symmetry plane. Each half was mounted, lateral side up, on the cryostat sample holder pretreated by Tissue-Tek OCT compound and fixed by freezing in the cryostat. After fixing, brains were cut at -22 °C into sagittal slices 20 µm thick using a Leica microtome, and the slices were thaw-mounted on Superfrost (70 × 22 mm, Fischer) adhesive slides, 3-4 slices per slide. Only slices containing midbrain were used because midbrain structures express relatively high levels of PSMA. The slices were allowed to dry and were then put into storage boxes and kept at -80 °C until they were used.

Saturation assay to measure the affinity of [¹¹¹In]In-PSMA-617 towards PSMA in brain slices and competition assay to measure the affinity of PSMA derivatives towards the same target were designed according to the guidelines of Hein et al. Briefly, for saturation assay, the slides were incubated for 1.5 h with increasing concentrations (0.1-30 nM) of [¹¹¹In]In-PSMA-617 in the assay buffer (170 mM Tris-HCl pH 7.2, 5 mM MgCl₂, 1% BSA). For competition assay, the slides were incubated with 2 nM [¹¹¹In]In-PSMA-617 and increasing concentrations (0.1-30 nM) of investigated PSMA derivatives for the same amount of time in the same assay buffer. Non-specific binding was determined by adding 5 µM 2-(phosphonomethyl)pentanedioic acid (PMPA) to [¹¹¹In]In-PSMA-617 solutions.

Incubations were performed by creating a hydrophobic barrier along the edges of the slides with a PAP pen, adding 1 mL of the corresponding incubation solution per slide and then gently shaking the slides at room temperature in a closed plastic box. However, for low (<0.3 nM) concentrations of [¹¹¹In]In-PSMA-617 in the saturation assay, the slides were incubated in a bath filled with [¹¹¹In]In-PSMA-617 solution in the assay buffer instead (>20 mL / slide). Immediately before the incubation, 1 mL assay buffer was applied to each slide, the slides were shaken at room temperature for 15 min as described above, and then assay buffer was replaced with the corresponding incubation solution.

After the incubation, the slides were rinsed by dipping them into ice-cold assay buffer for 5 min and then into ice-cold distilled water for 0.5 min. Afterwards, the slices were dried in air stream and exposed against phosphor screens (Packard Instruments Co) for 24-72 h along with a calibration curve prepared by spotting 1 µL aliquots of [¹¹¹In]In-PSMA-617 solutions with known concentrations (0.1-30 nM) on a silica TLC strip.

The screens were then read by a Cyclone Plus storage phosphor system (Packard Instruments Co). Quantification of readings was done with Optiquant software (version 3.00, Packard Instruments Co). Namely, elliptic regions of interest (ROIs) encompassing the midbrain and cerebellar nuclei were drawn inside the brain slice images, while circular ROIs were drawn around calibration curve spots, and average exposure was calculated for both ROI categories. Exposure values were then recalculated into fmol [¹¹¹In]In-PSMA-617 per µL tissue (equivalent to nM) by fitting a linear function onto the calibration curve values. The fmol/µL values were then used for non-linear regression in GraphPad Prism (v9.4.1) to calculate Bₘₐₓ and K_{D} of [¹¹¹In]In-PSMA-617 in the saturation assay and IC50 of PSMA derivatives in the competitions assays. IC50 values were automatically recalculated into Kᵢ values by GraphPad Prism using the Cheng-Prusoff equation. The experiment was performed with tetrazines having a stable atom in their structure (Table 2).

**Table 2.**

| **Compound** | **Lipophilic linker** | **Amino acid linker (N-Linker-C)** | **Tetrazine** | **Kᵢ (nM)** |
|---|---|---|---|---|
| **PSMA-617** | 2-naphtylalanine | - | - | 0.89 |
| **28** | 3-I-L-Phenylalanine | R-E-R-E | **6** | 1.25 |
| **33** | 3-I-L-Phenylalanine | R-E-R-E-R-E | **6** | 1.11 |
| **40** | 3-I-L-Phenylalanine | R-E-S-S | **6** | 0.44 |
| **45** | 3-I-L-Phenylalanine | S-S-S-S | **6** | 0.97 |

The values for all the tested compounds were in line with results published for PSMA-617 suggesting that the affinity for the target is retained.

### Example 17: General procedure for internalization assay

Poly-L-Lysine (300 µL of a 0.01% w/v in H₂O) is added to each well of a 24-well plate (Greiner Bio). The plate is left at room temperature for 20-30 minutes. Afterwards, the poly-L-lysine solution is removed, and the wells washed with 500 µL of PBS. Afterwards, 1 mL of LNCaP cells suspension is placed in each well to obtain 10⁵ cells/well. The 24-well plates are left in the incubator overnight until the start of the internalisation experiment. Subsequently, the previously plated cells were incubated at 37 °C with the selected ¹⁸ F-labelled PSMA derivative in 250 µL growth medium for 45 min (c = 32 nM). For the determination of specific uptake, 2-PMPA was added into half of the wells in an excess concentration of 500 µM/well. After incubation, cellular uptake was interrupted by removing the medium and washing the cells with ice-cold PBS (3 × 1 mL). The surface bound radioactivity was removed by washing with 50 mM glycine (pH 2.8, 2 × 500 µL). Finally, the internalized fraction was determined by lysis of the cells with 0.3 M NaOH (1 × 500 µL). Fractions collected from the surface bound activity and lysates were collected and measured in a gamma counter. All counts were normalized to the starting activity and specific surface binding as well as specific internalization were determined by subtracting the non-specific binding obtained from the wells treated with 2-PMPA. Results were calculated and expressed as %AA/10⁵ cells (% applied activity/105 cells, n = 4). [⁶⁸Ga]-PSMA617 was selected as a reference to compare internalization values. The experiment was conducted three times on different compounds. The data are shown below in Table 5. PSMA-617 was used as a reference and internal control for all three experiments. The internalization of the control PSMA-617 are very similar and differs only marginally between the three tests confirming the reproducibility of the test. In order to take this slight difference into account and provide a value that can be directly compared between the three tests, the surface-to-internalized ratio is also shown.

**Table 5**

| | | **Surface** | **Internalized** | **Surface-to-internalized ratio** |
|---|---|---|---|---|
| | | Mean ± SD | Mean ± SD | Mean |
| **PSMA-617** | control | 1.67 ± 0.24 | 0.37 ± 0.09 | 4.52 |
| | block | 0.27 ± 0.03 | 0.08 ± 0.03 | 3.17 |
| **33** | control | 1.31 ± 0.10 | 0.49 ± 0.07 | 2.66 |
| | block | 0.22 ± 0.10 | 0.11 ± 0.05 | 2.11 |
| **40** | control | 0.98 ± 0.26 | 6.36 ± 0.13 | 2.72 |
| | block | 0.14 ± 0.04 | 0.05 ± 0.01 | 2.80 |
| **45** | control | 3.49 ± 0.11 | 1.37 ± 0.13 | 2.55 |
| | block | 1.07 ± 0.15 | 0.32 ± 0.21 | 3.36 |

The data shows that the compounds presented here have a better internalization compared to PSMA-617. The higher internalization could have a big impact on the therapeutic effect of such molecules.

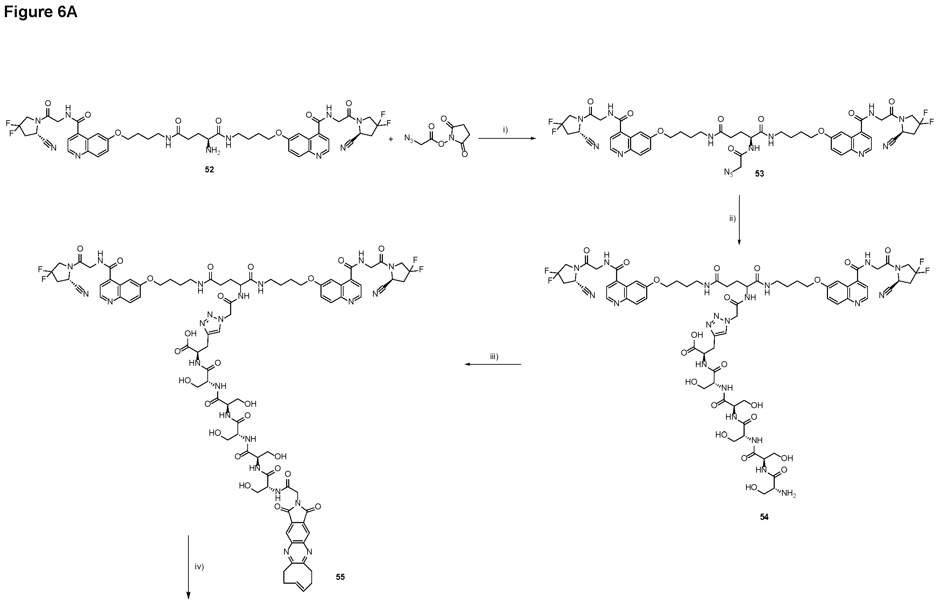

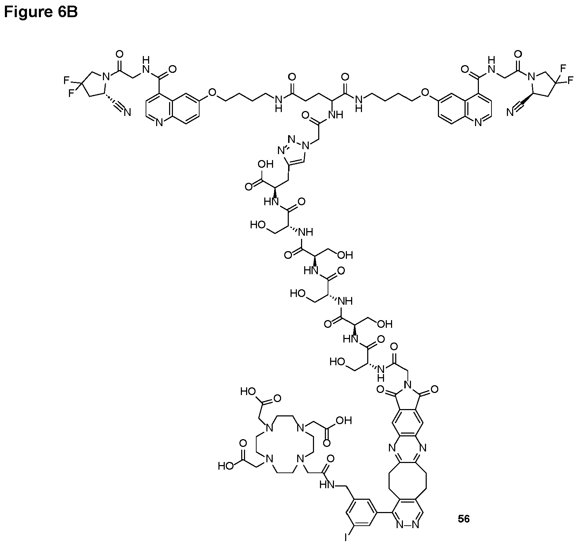

### Example 18: Synthesis of compound 56 (Figure 6A and 6B)

Figure 6A and 6B show the synthesis of compound **56.** The following reagents and conditions were used in the steps i - iii: i) DIPEA, DMF, rt, 5 h; ii) **51,** CuSO₄·H₂O, sodium ascorbate, BPDS, DMF, H₂O, rt, 12 h; iii) T4CO-NHS, N-methyl morpholine, DMF, rt, 12 h, iv) **6,** ACN, H₂O, TFA, rt, 5 h.

### Synthesis of A5Ser (Compound 51, Scheme 11)

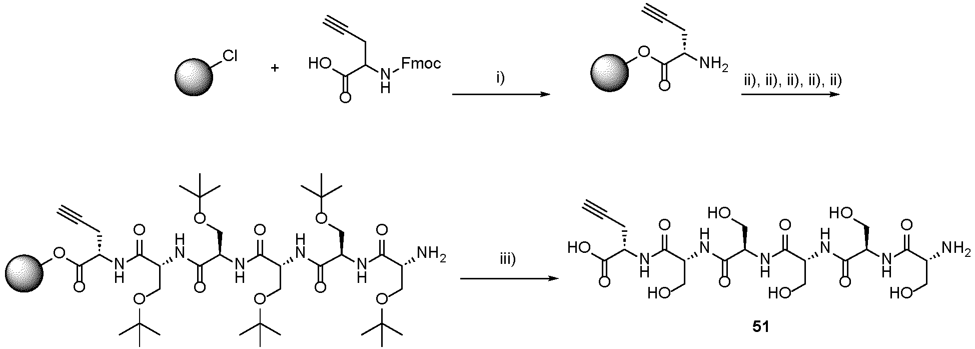

Scheme 11 shows the synthesis of compound **51.** The grey dot in the intermediate structures symbolizes a resin. The following reagents and conditions were used in the steps i - iii: i) a) DIPEA, CH2Cl2, rt, 2 h, b) 50% piperidine in DMF, rt, 20 min.; ii) Fmoc-tBu-D-Ser-OH, HATU, DIPEA, DMF, rt, 2 h, b) 50% piperidine in DMF, rt, 20 min.; iii) TFA, TIPS, H2O, rt, 2 h.

Chlorotrityl resin (1.28 mmol, 1.2 g) was stirred in a round bottom flask in CH2Cl2 (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH2Cl2 (10 mL). The resin was capped by reacting two times for 20 minutes with 5 mL of a mixture of CH2Cl2, MeOH, DIPEA (17:2:1). The Fmoc-protecting group was then cleaved with a mixture of DMF and piperidine (1:1). The product reacted with 2 equiv. of Fmoc-D-Ser(tBu)-OH activated with 1.95 equivalent of HATU and 2 equivalent of DIPEA. Deprotection of the Fmoc group with a mixture of DMF and piperidine (1:1) gave the unprotected immobilized peptide. The D-Ser coupling was repeated four more times to afford the final protected peptide. The product was cleaved from the resin and deprotected with trifluoroacetic acid (TFA), triisopropylsilane (TIPS) and water (95:2.5:2.5). Preparative HPLC afforded 0.14 g of the desired peptide. MS (ESI+): m/z (%) = 547.3 (100, [M-H]-), calculated for C20H30N6O12: 546.2 [M].

### Synthesis of compound 53 (Figure 6A)

To a solution of 52 (0.049 mmol, 0.05 g) in DMF (2 mL) was added DIPEA (0.12 mmol, 0.021 mL) and 2-azidoacetic NHS ester (0.054 mmol, 0.011 g). The resulting mixture was stirred for 4 hours. The volatiles were then removed under reduced pressure and the residue purified by reversed phase chromatography to give 0.02 g (43%) of the desired product as a white solid. Rf =0.15 (CH₂Cl₂/MeOH, 90/10); ¹H NMR (600 MHz, MeOD) δ 8.90 - 8.84 (m, 2H), 8.06 (t, *J* = 2.3 Hz, 2H), 8.03 (d, *J* = 9.3 Hz, 2H), 7.78 (dd, *J* = 5.0, 2.1 Hz, 2H), 7.61 (dt, *J* = 9.3, 3.0 Hz, 2H), 5.16 (dt, *J* = 9.4, 2.6 Hz, 2H), 4.40 - 4.20 (m, 12H), 4.19 - 4.09 (m, 2H), 3.91 (s, 2H), 3.26 (t, *J* = 7.0 Hz, 2H), 2.99 - 2.75 (m, 4H), 2.25 (t, *J* = 7.4 Hz, 2H), 2.08 (dtd, *J* = 13.0, 7.7, 5.1 Hz, 1H), 1.90 (pd, *J* = 7.8, 2.9 Hz, 6H), 1.74 (qd, *J* = 12.5, 7.1 Hz, 5H); ¹³C NMR (151 MHz, MeOD) δ 174.73, 173.35, 170.15, 169.43, 169.38, 169.34, 161.37, 161.12, 160.61, 160.58, 146.83, 146.72, 145.43, 145.37, 129.22, 128.34, 127.65, 127.55, 127.32, 127.25, 125.92, 120.66, 118.30, 118.28, 105.74, 69.95, 69.91, 57.48 (dt, *J* = 43.0, 21.5 Hz), 54.41, 53.17, 52.95, 52.78, 52.74, 45.97, 45.94, 42.97, 42.96, 40.17, 40.15, 33.10, 29.22, 27.43, 27.34, 26.98, 26.87, 17.28 (dt, *J* = 38.4, 19.1 Hz); MS (ESI+): m/z (%) = 538.3 (100, [M+2H]²⁺), 1057.4 (30, [M+H]⁺), calculated for C₄₉H₅₂F₄N₁₄O₉: 1056.4 [M].

### Synthesis of compound 54 (Figure 6A)

An aqueous solution of CuSO₄·5H₂O (100 mg/mL; 0.0089 mmol) was mixed with an aqueous solution of sodium ascorbate (300 mg/mL, 0.036 mmol), when the color of the mixture turned yellow a solution of BPDS (25 mg/mL, 0.0089 mmol) in water was added. The resulting blue/green mixture was added to a solution of the peptide (0.032 g, 0.048 mmol) in DMF (0.5 mL). Afterward, **53** (0.047 g, 0.044 mmol) dissolved in DMF (1 mL) was stirred at room temperature for 4 h. Full click was observed. The compound was diluted in water (12 mL) and purified by reversed flash HPLC to give 0.027 g of the compound as a white solid. MS (ESI+): m/z (%) = 402.5 (40, [M+4H]⁴⁺), 536.3 (100, [M+3H]³⁺), 803.7 (80, [M+4H]⁴⁺), calculated for C₆₉H₈₅F₄N₂₀O₂₁: 1605.6 [M].

### Synthesis of compound 55 (Figure 6A)

To a solution of **54** (0.03 g, 0.013 mmol) and T4CO-NHS (0.007 g, 0.015 mmol) in dry DMF (2 mL) under argon, was added 4-methylmorpholine (0.012 mL, 0.11 mmol). The reaction was stirred at room temperature for 12 h. The mixture was then diluted with 10 mL of 1%TFA in H₂O and purified by preparative HPLC. The collected fractions were freeze-dried to give 0.007 g (24%) of the desired compound as a white solid. MS (ESI+): m/z (%) = 642.4 (60, [M+2H]²⁺), 962.9 (100, [M+2H]²⁺), 1925.6 (100, [M+H]⁺), calculated for C₈₇H₉₇F₄N₂₃O₂₄: 1923.7 [M].

### Synthesis of compound 56 (Figure 6B)

To a solution of **55** (0.005 g, 0.0026 mmol) in ACN (2 mL) and 1% TFA in H₂O (2 mL) was added 6 (0.002 g, 0.0027 mmol) in 1%TFA in H₂O (1 mL). The reaction was stirred at room temperature for 12 h. The mixture was directly purified by preparative HPLC. The collected fractions were freeze-dried to give 0.004 g (59%) of the desired compound as a white solid. MS (ESI+): m/z (%) = 649.4 (100, [M+4H]⁴⁺), 865.4 (70, [M+3H]³⁺), 1297.9 (40, [M+2H]²⁺), calculated for C₁₁₂H₁₂₉F₄IN₃₀O₃₁: 2592.8 [M].

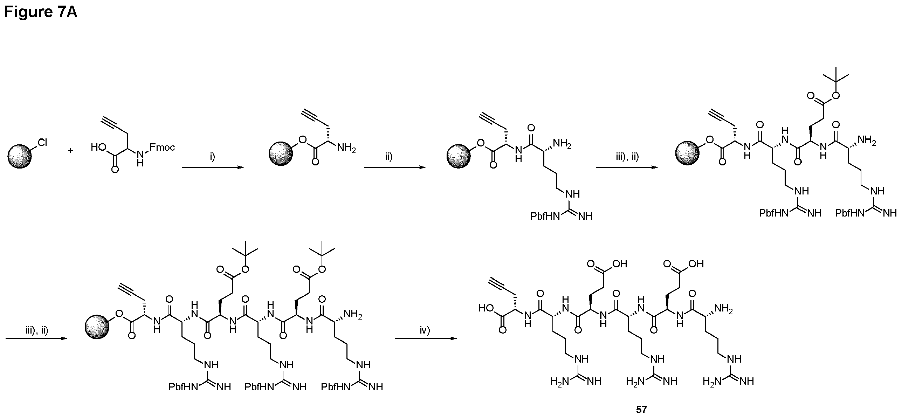

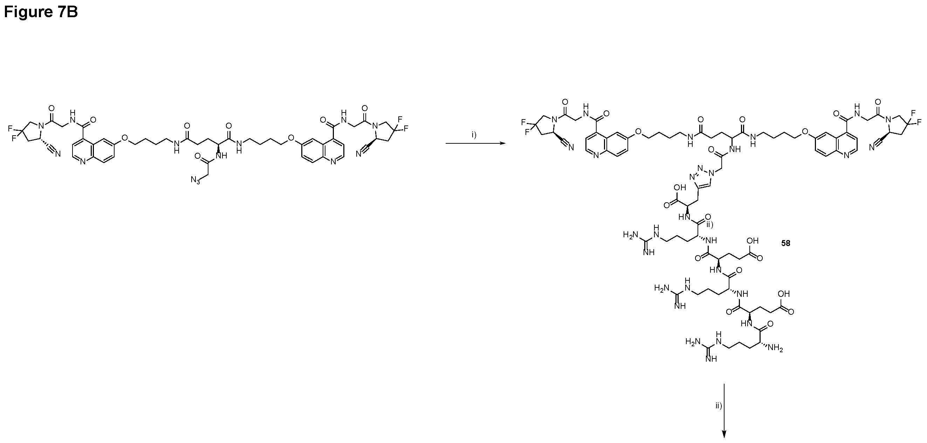

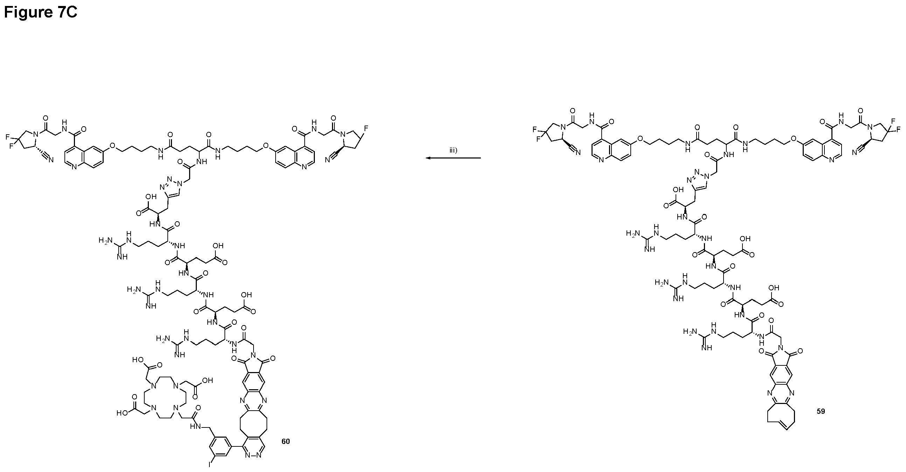

### Example 19: Synthesis of compound 60 (Figure 7A, 7B, and 7C)

Figure 7A, 7B, and 7C is a scheme showing the synthesis of compound **60.** The following reagents and conditions were used in the steps i - iii: i) **57,** CuSO₄·H₂O, sodium ascorbate, BPDS, DMF, H₂O, rt, 12 h; ii) T4CO-NHS, N-methyl morpholine, DMF, rt, 12 h, iii) H-DOTA-Itz, ACN, H₂O, TFA, rt, 5 h.

### Synthesis of 6A (Compound 57, Figure 7A)

Figure 7A is a scheme showing the synthesis of compound **57.** The grey dot in the intermediate structures symbolizes a resin. The following reagents and conditions were used in the steps i - iv: : i) a) DIPEA, CH₂Cl₂, rt, 2 h, b) 50% piperidine in DMF, rt, 20 min.; ii) Fmoc-D-Arg(Pbf)-OH, HATU, DIPEA, DMF, rt, 2 h, b) 50% piperidine in DMF, rt, 20 min.; iii) Fmoc-D-Glu-OH, HATU, DIPEA, DMF, rt, 2 h, b) 50% piperidine in DMF, rt, 20 min.; iv) TFA, TIPS, H₂O, rt, 2 h.

Chlorotrityl resin (1.28 mmol, 1.2 g) was stirred in a round bottom flask in CH₂Cl₂ (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH₂Cl₂ (10 mL). The resin was capped by reacting two times for 20 minutes with 5 mL of a mixture of CH₂Cl₂, MeOH, DIPEA (17:2:1). The Fmoc-protecting group was then cleaved with a mixture of DMF and piperidine (1:1). The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave the desired product. Subsequently, 4 equiv. of Fmoc-D-Glu-OH were activated with 3.92 equiv. of HATU and 4 equiv. of DIPEA in DMF. The solution was added to the resin-immobilized peptide and shaken for 1 hour. The Fmoc-protecting group was cleaved with a mixture of DMF and piperidine. The product reacted with 4 equiv. of Fmoc-D-Arg(Pbf)-OH activated with 3.92 equiv. of HATU and 4 equiv. of DIPEA. Deprotection of the Fmoc group with a mixture of DMF and piperidine (1:1) gave deprotected immobilized resin. The D-Glu and D-Arg coupling was repeated one more time to afford the final protected peptide. The product was cleaved from the resin and deprotected with trifluoroacetic acid (TFA), triisopropylsilane (TIPS) and water (95:2.5:2.5). Preparative HPLC afforded 0.15 g of the desired peptide. MS (ESI+): m/z (%) = 280.9 (100, [M+3H]³⁺), 420.8 (90, [M+2H]²⁺), 840.6 (30, [M+H]⁺), calculated for C₃₃H₅₇N₁₅O₁₁: 839.4 [M].

### Synthesis of compound 58 (Figure 7B)

An aqueous solution of CuSO₄·5H₂O (100 mg/mL; 0.01 mL; 0.0038 mmol) was mixed with an aqueous solution of sodium ascorbate (300 mg/mL, 0.01 mL; 0.015 mmol), when the color of the mixture turned yellow a solution of BPDS (25 mg/mL, 0.01 mL, 0.0038 mmol) in water was added. The resulting blue/green mixture was added to a solution of 9 (0.024 g, 0.021 mmol) in DMF (0.5 mL). Afterward, **53** (0.02 g, 0.019 mmol) dissolved in DMF (1 mL) was stirred at room temperature for 4 h. Full click was observed. The compound was diluted in water (12 mL) and purified by reversed flash HPLC to give 0.028 g of the compound as a white solid. MS (ESI+): m/z (%) = 380.3 (80, [M+5H]⁵⁺), 475.2 (100, [M+4H]⁴⁺), 633.2 (90, [M+3H]³⁺), 949.0 (50, [M+2H]²⁺), 1896.8 (5, [M+H]⁺), calculated for C₈₂H₁₀₉F₄N₂₉O₂₀: 1895.9 [M].

### Synthesis of compound 59 (Figure 7C)

To a solution of **58** (0.03 g, 0.013 mmol) and T4CO-NHS (0.07 g, 0.015 mmol) in dry DMF (2 mL) under argon, was added 4-methylmorpholine (0.012 mL, 0.11 mmol). The reaction was stirred at room temperature for 12 h. The mixture was then diluted with 10 mL of 1%TFA in H₂O and purified by preparative HPLC. The collected fractions were freeze-dried to give 0.007 g (21%) of the desired compound as a white solid. MS (ESI+): m/z (%) = 444.2 (100, [M+5H]⁵⁺), 555.2 (30, [M+4H]⁴⁺), 739.8 (30, [M+3H]³⁺), 1108.8 (20, [M+2H]²⁺), calculated for C₁₀₀H₁₂₂F₄N₃₂O₂₃: 2214.9 [M].

### Synthesis of compound 60 (Figure 7C)

To a solution of **59** (0.006 g, 0.0023 mmol) in ACN (2 mL) and 1% TFA in H₂O (2 mL) was added **6** (0.002 g, 0.0026 mmol) in 1%TFA in H₂O (1 mL). The reaction was stirred at room temperature for 12 h. The mixture was directly purified by preparative HPLC. The collected fractions were freeze-dried to give 0.005 g (71%) of the desired compound as a white solid. MS (ESI+): m/z (%) = 482.0 (100, [M+6H]⁶⁺), 578.4 (40, [M+5H]⁵⁺), 722.7 (30, [M+4H]⁴⁺), 962.8 (50, [M+3H]³⁺), calculated for C₁₂₅H₁₅₄F₄I N₃₉O₃₀: 2884.1[M].

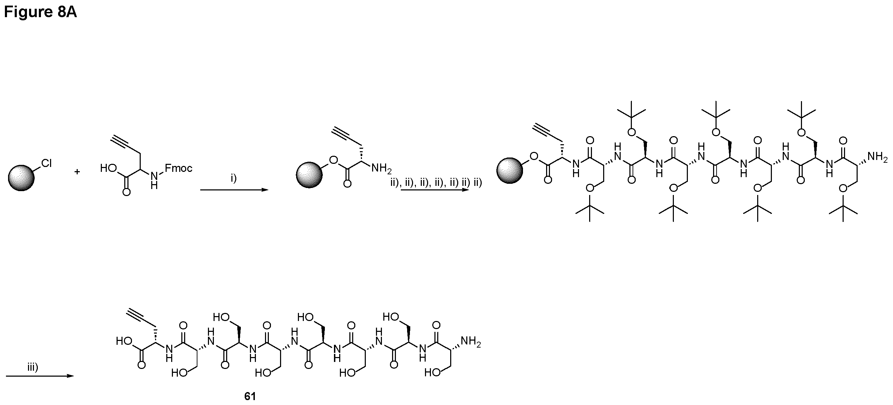

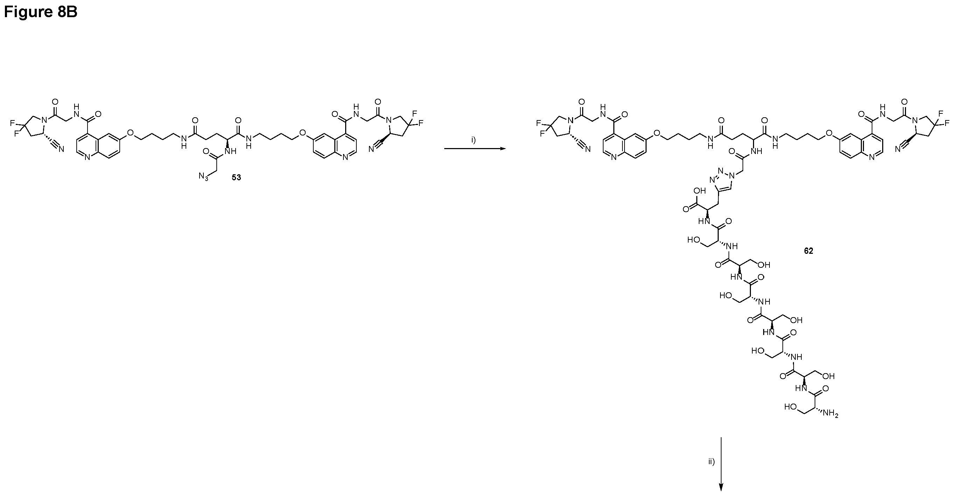

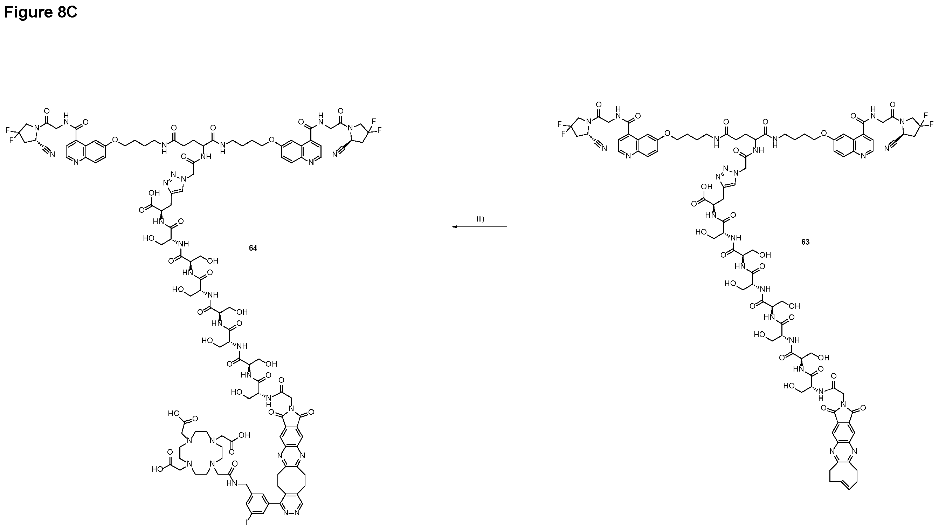

### Example 20: Synthesis of compound 64 (Figure 8A, 8B, and 8C)

Figure 8A, 8B, and 8C is a scheme showing the synthesis of compound **64.** The following reagents and conditions were used in the steps i - iii: i) **61,** CuSO₄·H₂O, sodium ascorbate, BPDS, DMF, H₂O, rt, 12 h; ii) T4CO-NHS, N-methyl morpholine, DMF, rt, 12 h, iii) H-DOTA-ITz, ACN, H₂O, TFA, rt, 5 h.

### Synthesis of A7Ser (Compound 61, Figure 8A)

Figure 8A is a scheme showing the synthesis of compound **13.** The grey dot in the intermediate structures symbolizes a resin. The following reagents and conditions were used in the steps i - iii: i) a) DIPEA, CH₂Cl₂, rt, 2 h, b) 50% piperidine in DMF, rt, 20 min.; ii) Fmoc-tBu-D-Ser-OH, HATU, DIPEA, DMF, rt, 2 h, b) 50% piperidine in DMF, rt, 20 min.; iii) TFA, TIPS, H₂O, rt, 2 h.

Chlorotrityl resin (1.28 mmol, 1.2 g) was stirred in a round bottom flask in CH₂Cl₂ (20 mL) for 2 hours. Subsequently, Fmoc-propargyl-glycine-OH (1.53 mmol, 0.51 g) and DIPEA (6.14 mmol, 1.08 mL) were added, and the reaction stirred for 12 hours at room temperature. The resin was then transferred to a reactor and washed twice with CH₂Cl₂ (10 mL). The resin was capped by reacting two times for 20 minutes with 5 mL of a mixture of CH₂Cl₂, MeOH, DIPEA (17:2:1). The Fmoc-protecting group was then cleaved with a mixture of DMF and piperidine (1:1). The product reacted with 2 equiv. of Fmoc-D-Ser(tBu)-OH activated with 1.95 equivalent of HATU and 2 equivalent of DIPEA. Deprotection of the Fmoc group with a mixture of DMF and piperidine (1:1) gave the unprotected immobilized peptide. The D-Ser coupling was repeated six more times to afford the final protected peptide. The product was cleaved from the resin and deprotected with trifluoroacetic acid (TFA), triisopropylsilane (TIPS) and water (95:2.5:2.5). Preparative HPLC afforded 0.12 g of the desired peptide. MS (ESI+): m/z (%) = 721.2 (100, [M-H]⁻), calculated for C₂₀H₃₀N₆O₁₂: 722.3 [M].

### Synthesis of compound 62 (Figure 8B)

An aqueous solution of CuSO₄·5H₂O (100 mg/mL; 0.01 mL; 0.0066 mmol) was mixed with an aqueous solution of sodium ascorbate (300 mg/mL, 0.03 mL; 0.053 mmol), when the color of the mixture turned yellow a solution of BPDS (25 mg/mL, 0.3 mL, 0.0133 mmol) in water was added. The resulting blue/green mixture was added to a solution of **61** (0.061 g, 0.073 mmol) in DMF (0.5 mL). Afterward, **53** (0.07 g, 0.066 mmol) dissolved in DMF (1 mL) was stirred at room temperature for 4 h. Full click was observed. The compound was diluted in water (12 mL) and purified by reversed flash HPLC to give 0.015 g of the compound as a white solid. MS (ESI+): m/z (%) = 594.1 (100, [M+3H]³⁺), 890.5 (60, [M+2H]²⁺), 1779.7 (5, [M+H]⁺), calculated for C₇₅H₉₄F₄N₂₂O₂₅: 1778.7 [M].

### Synthesis of compound 63 (Figure 8C)

To a solution of **62** (0.02 g, 0.011 mmol) and T4CO-NHS (0.06 g, 0.013 mmol) in dry DMF (2 mL) under argon, was added 4-methylmorpholine (0.006 mL, 0.053 mmol). The reaction was stirred at room temperature for 12 h. The mixture was then diluted with 10 mL of 1%TFA in H₂O and purified by preparative HPLC. The collected fractions were freeze-dried to give 0.005 g (22%) of the desired compound as a white solid. MS (ESI+): m/z (%) = 700.7 (60, [M+3H]³⁺), 1050.2 (100, [M+2H]²⁺), calculated for C₉₃H₁₀₇F₄N₂₅O₂₈: 2097.8 [M].

### Synthesis of compound 64 (Figure 8C)

To a solution of **63** (0.003 g, 0.0014 mmol) in ACN (2 mL) and 1% TFA in H₂O (2 mL) was added **6** (0.001 g, 0.0014 mmol) in 1%TFA in H₂O (1 mL). The reaction was stirred at room temperature for 12 h. The mixture was directly purified by preparative HPLC. The collected fractions were freeze-dried to give 0.001 g (30%) of the desired compound as a white solid. MS (ESI+): m/z (%) = 554.8 (45, [M+5H]⁵⁺), 693.2 (20, [M+4H]⁴⁺), 923.7 (100, [M+3H]³⁺), calculated for C₁₁₈H₁₃₉F₄I N₃₂O₃₅: 2776.9[M].

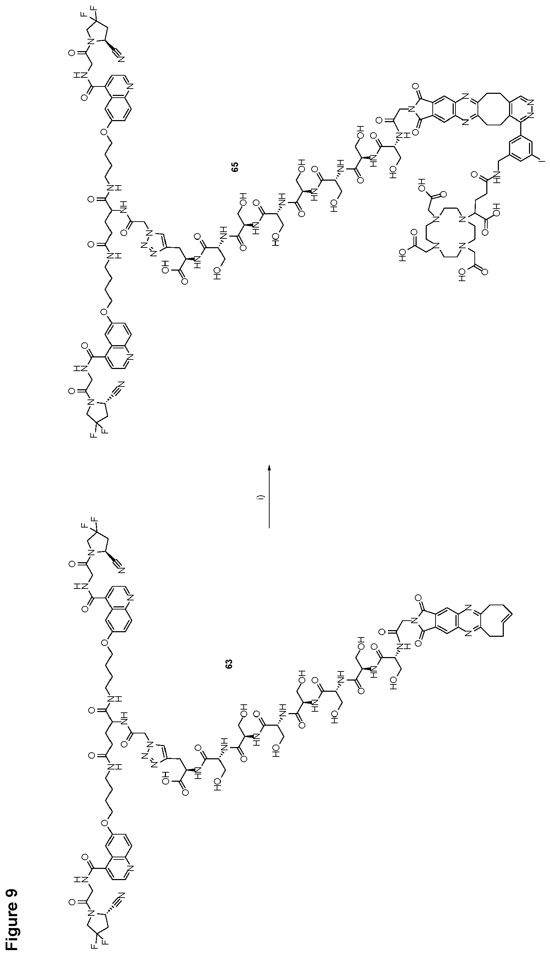

### Example 21: Synthesis compound 65 (Figure 9)

Figure 9 is a scheme showing the synthesis of compound **65.** The following reagents and conditions were used in step i: i) **6,** ACN, H₂O, TFA, rt, 5 h.

### Synthesis of compound 65 (Figure 9)

To a solution of **63** (0.003 g, 0.0014 mmol) in ACN (2 mL) and 1% TFA in H₂O (2 mL) was added **6** (0.001 g, 0.0014 mmol) in 1%TFA in H₂O (1 mL). The reaction was stirred at room temperature for 12 h. The mixture was directly purified by preparative HPLC. The collected fractions were freeze-dried to give 0.002 g (59%) of the desired compound as a white solid. MS (ESI+): m/z (%) = 569.3 (20, [M+5H]⁵⁺), 711.2 (40, [M+4H]⁴⁺), 947.5 (100, [M+3H]³⁺), calculated for C₁₂₁H₁₄₃F₄I N₃₂O₃₇: 2838.9[M].

## Claims

1. 1,2,4,5-tetrazine compound of formula (I): 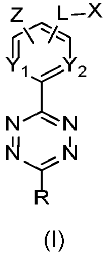 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO2NH(CH₂)ₙNH-, -NHSO₂(CH₂)ₙNH-, - CONH(CH₂)ₙNH-, -NHCO(CH₂)ₙNH-, -(OCH₂CH₂)n(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)ₙ(CH₂)ₘNH-, -SO₂NHCH₂(OCH₂CH₂)ₙ(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)ₘNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH₂)ₘNHCO-, - NHCOCH₂CH₂(OCH₂CH₂)ₙ(CH₂)ₘNHCO-,
where n and m are independently selected from the group consisting of 0-25;
Z is a moiety selected from:
Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, ¹⁸F, ¹⁹F, ⁷⁶Br, ⁷⁷Br, ⁷⁹Br, ⁸⁰Br, ^{80m}Br, ⁸¹Br, ¹²³I, ¹²⁴I,¹²⁵I, ¹²⁶I, ¹²⁷I, ¹³¹I, ²¹⁰At or ²¹¹At;
X is a chelator selected from:
1,4,7,10-tetraazacyclododecane-*N,N',N',N"*-tetraacetic acid (DOTA), 2-(4,7,10-tris(2-(tert-butoxy)-2-oxoethyl)-1,4,7,10-tetraazacyclododecan-1-yl)acetic acid (*t*Bu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((l2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
and wherein X is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr, ⁹⁴Tc, ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho, ¹⁷⁵Lu, ¹⁷⁷Lu, ¹⁸⁵Re, ¹⁸⁶Re, ¹⁸⁸Re, ²⁰¹Tl, ²⁰³Tl, ²⁰⁵Tl, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.

2. 1,2,4,5-tetrazine compound according to claim 1, wherein Z is a radionuclide halogen selected from: ¹⁸F, ⁷⁶Br, ⁷⁷Br, ^{80m}Br, ¹²³I, ¹²⁴I,¹²⁵I, ¹²⁶I, ¹³¹I, ²¹⁰At,²¹¹At, and wherein X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi.

3. 1,2,4,5-tetrazine compound according to claim 2, wherein Z is a radionuclide halogen selected from: ¹²³I, ¹³¹I, ²¹⁰At, ²¹¹At, and wherein X is unlabeled or X is labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho, ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi.

4. 1,2,4,5-tetrazine compound according to claim 2 selected from:

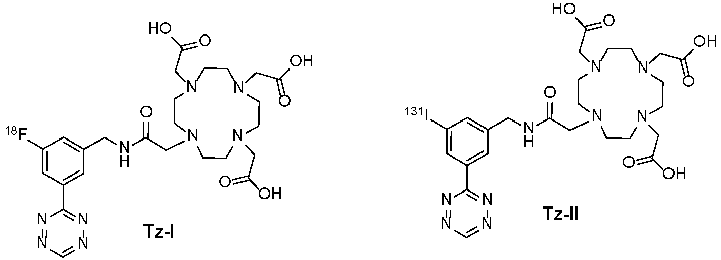

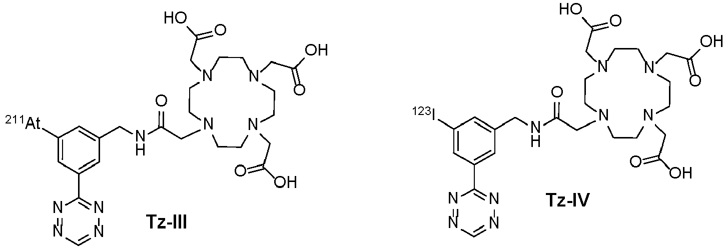

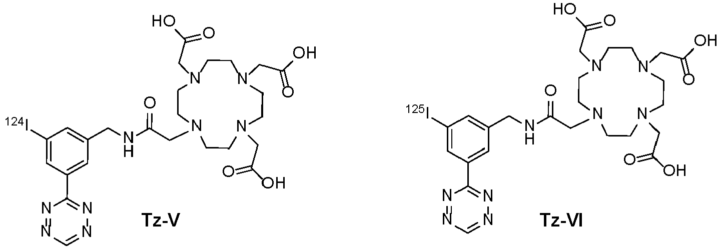

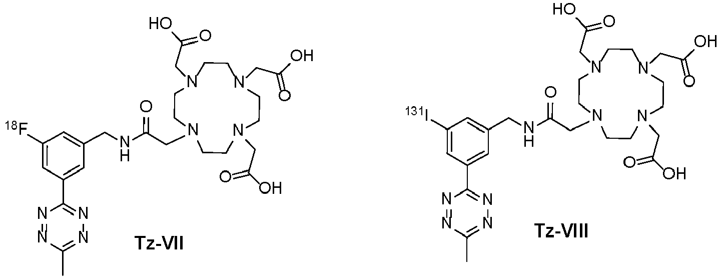

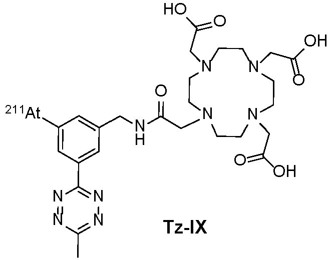

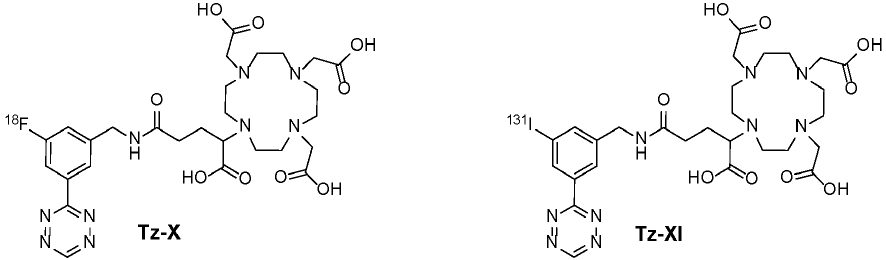

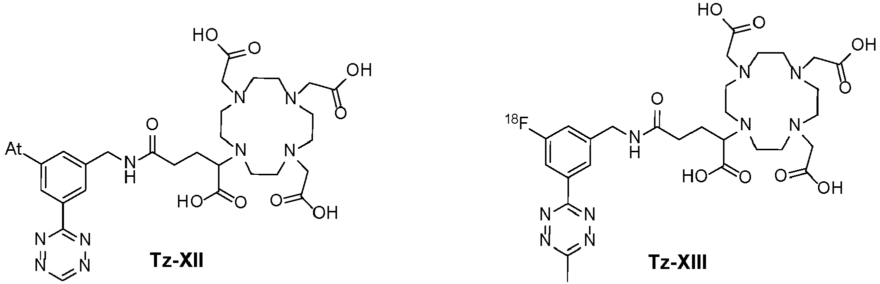

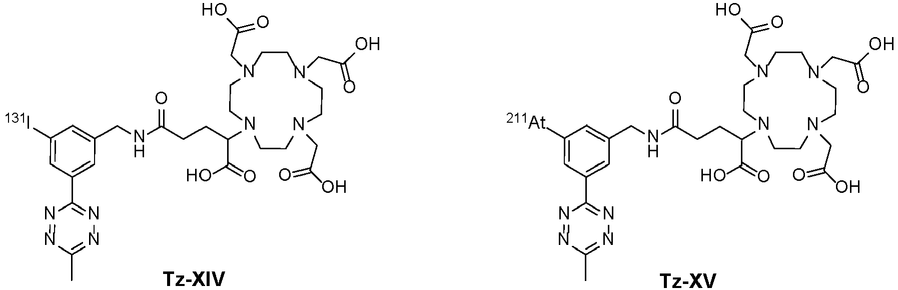

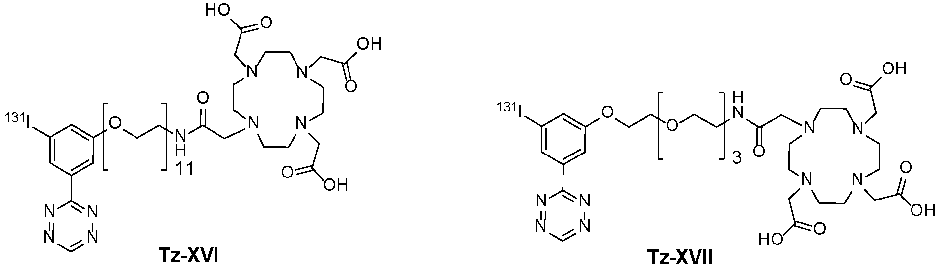

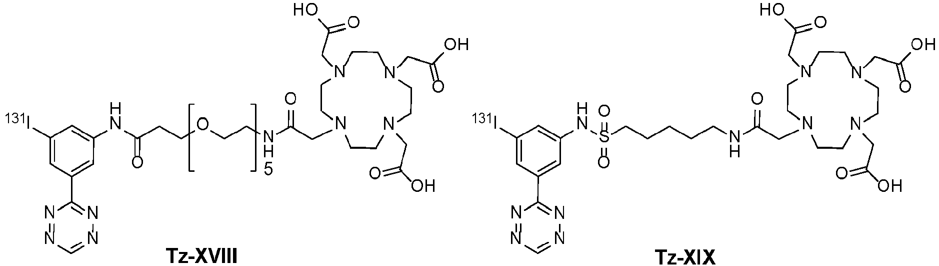

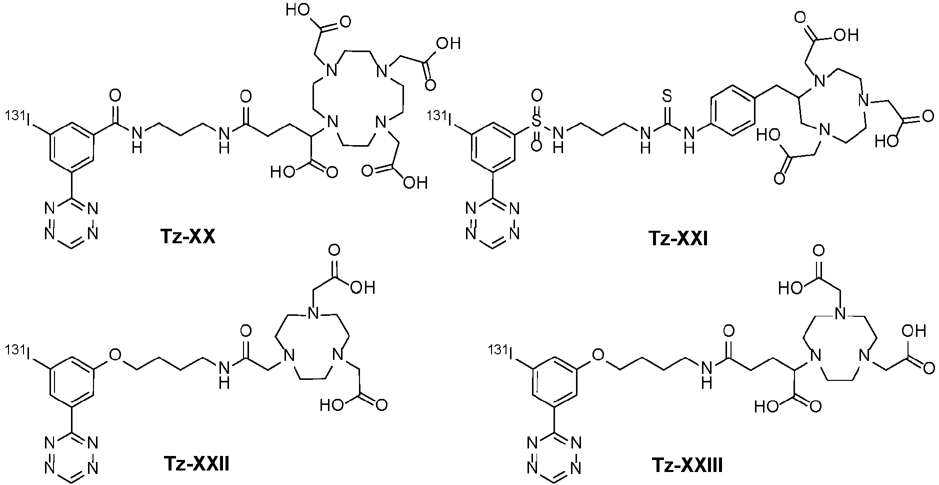

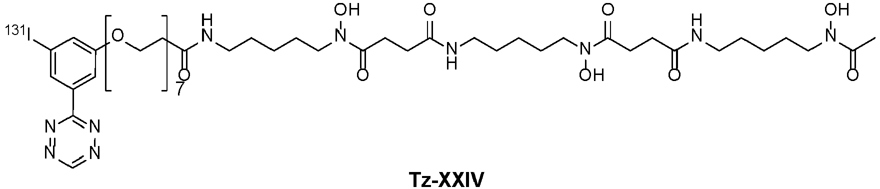

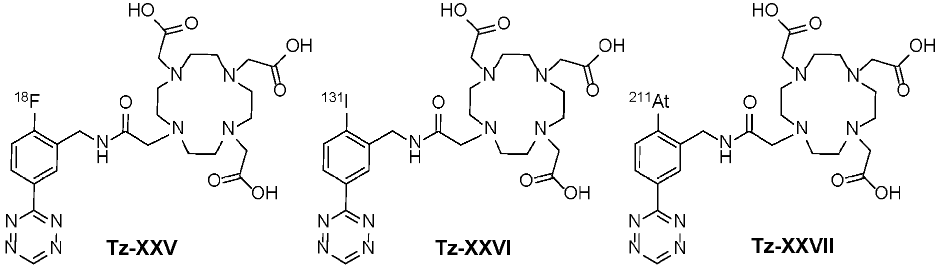

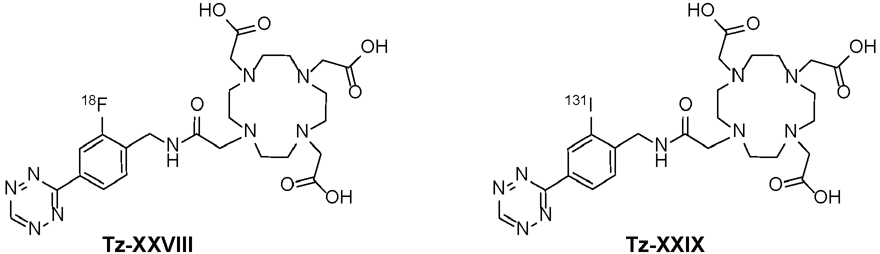

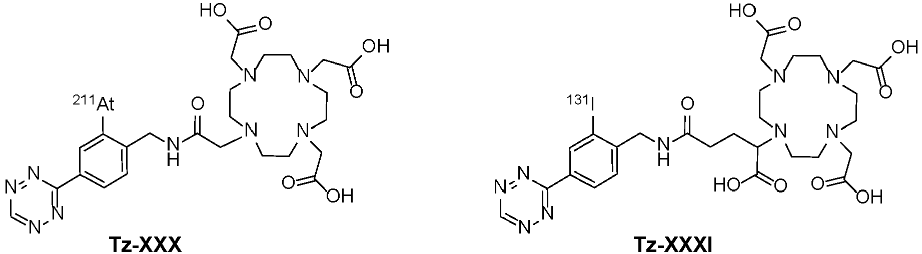

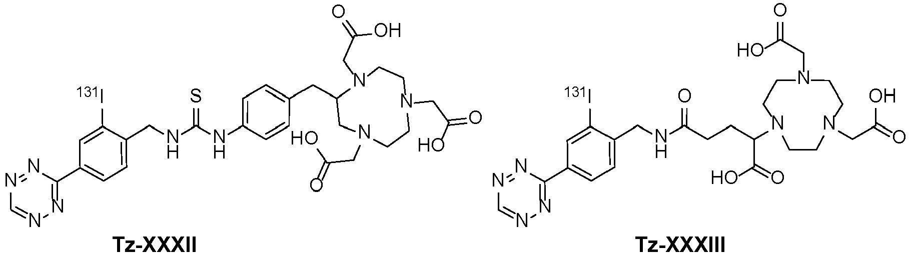

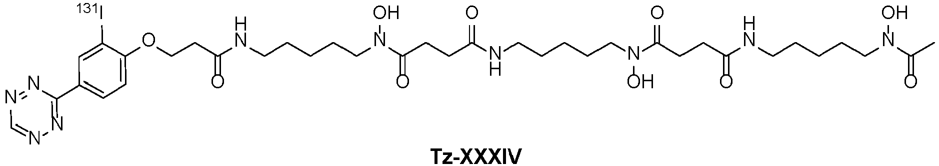

5. 1,2,4,5-tetrazine compound according to claim 1, wherein Z is selected from: ¹⁹F, ⁷⁹Br, ⁸¹Br, ¹²⁷I, and wherein X is labeled with a radionuclide metal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In, ¹¹³In, ^{114m}In, ^{115m}In, ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹Tl, ²⁰³Pb, ²¹²Pb, ²⁰⁹Bi, ²¹²Bi, ²¹³Bi, ²²³Ra, ²²⁵Ac, ²²⁷Th, ²³²Th.

6. 1,2,4,5-tetrazine compound according to claim 5, wherein X is labeled with a radionuclide metal selected from: ⁶⁴Cu, ⁶⁷Cu, ⁶⁸Ga, ⁹⁰Y, ⁸⁹Zr, ^{99m}Tc, ¹¹¹In, ¹⁷⁷Lu, ²⁰³Pb, ²¹²Pb, ²²⁵Ac.

7. 1,2,4,5-tetrazine compound according to claim 5 selected from:

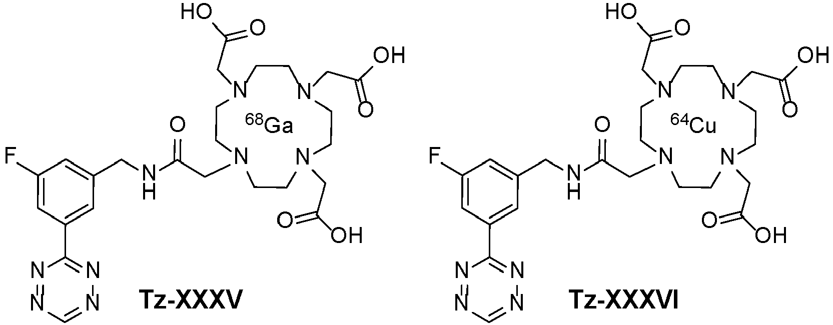

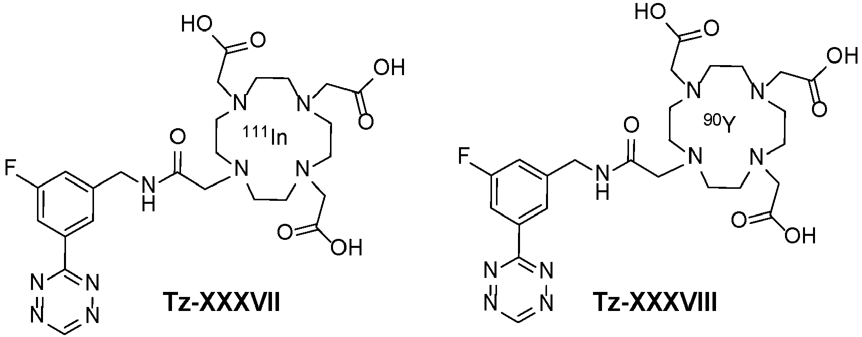

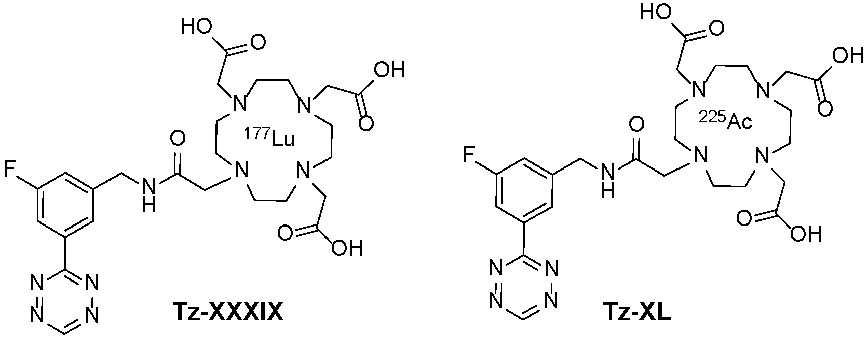

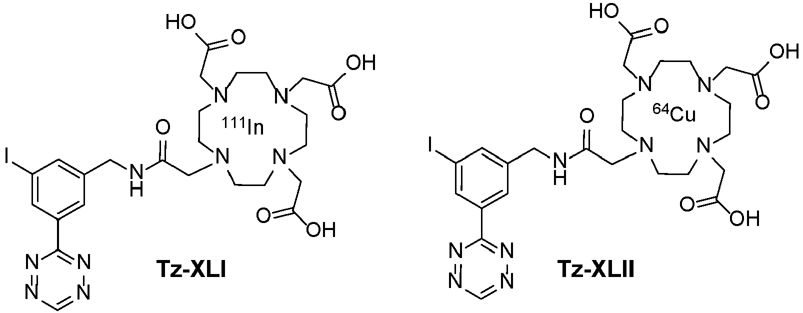

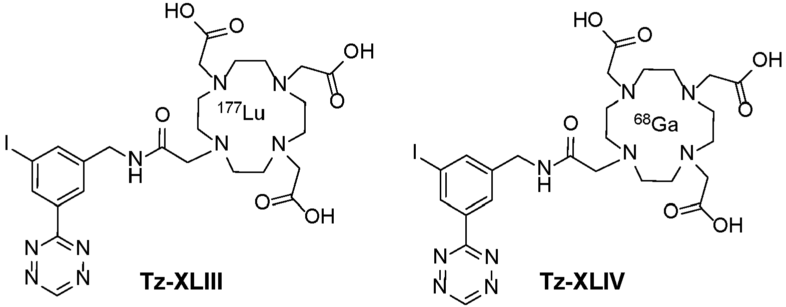

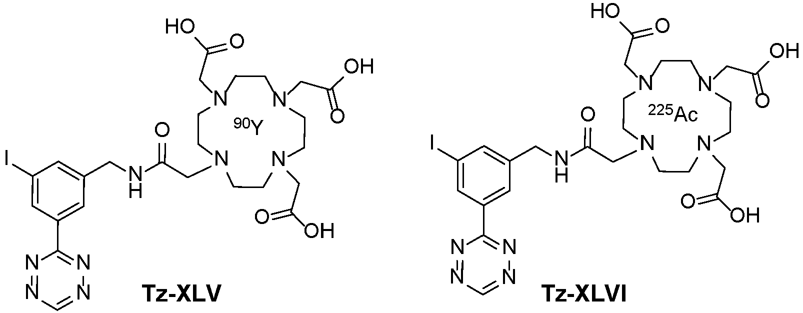

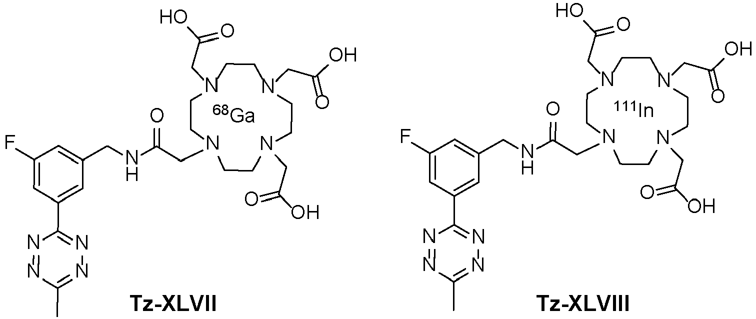

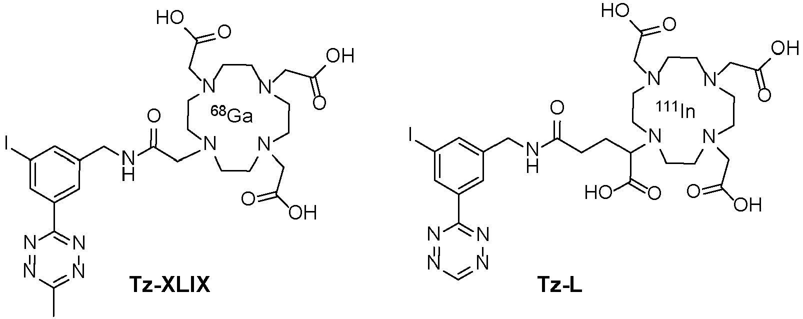

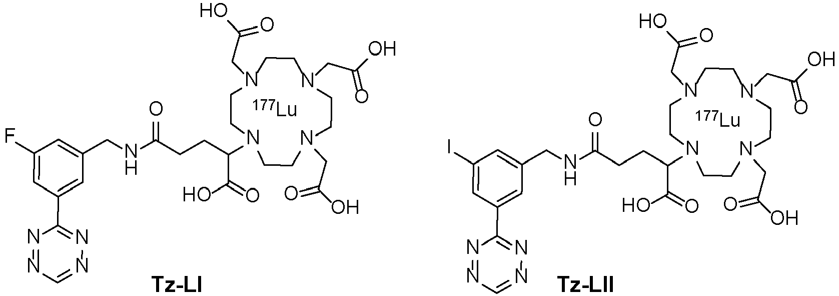

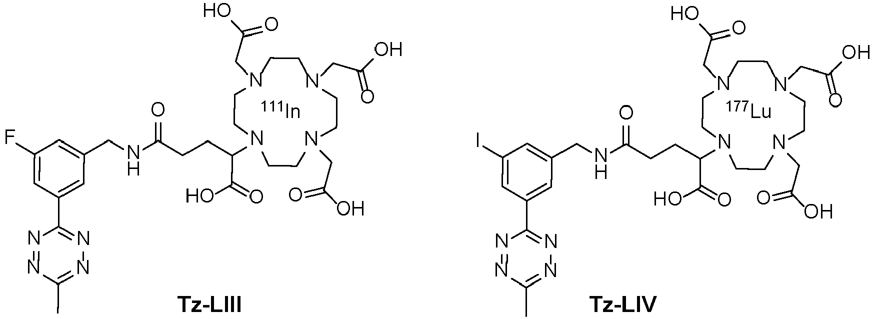

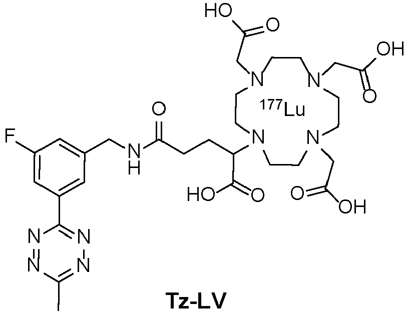

8. 1,2,4,5-tetrazine compound according to claim 1, wherein Z is selected from: Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, and wherein X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho ¹⁷⁵Lu, ¹⁸⁵Re, ²⁰³Tl, ²⁰⁵Tl, ²⁰⁶Pb, ²⁰⁷Pb, ²⁰⁸Pb, ²⁰⁹Bi.

9. 1,2,4,5-tetrazine compound according to claim 8, selected from:

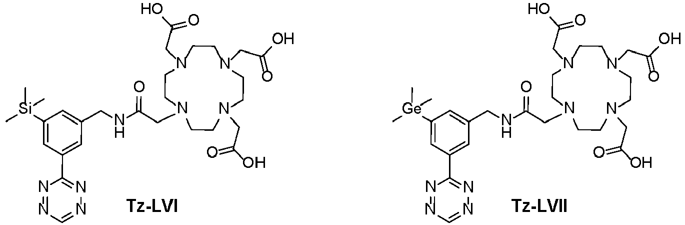

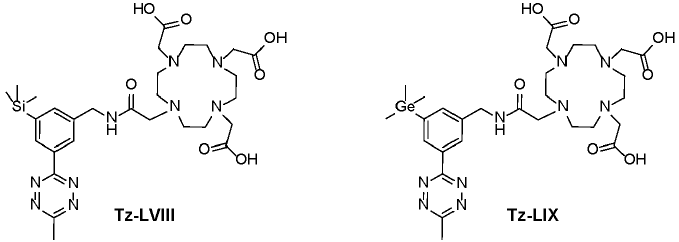

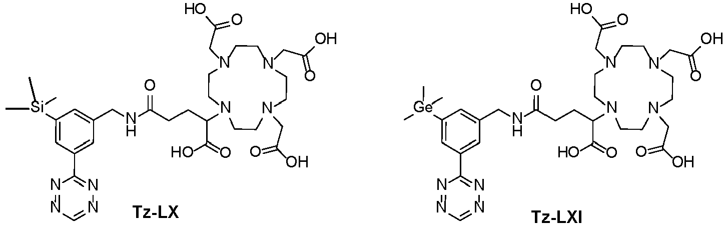

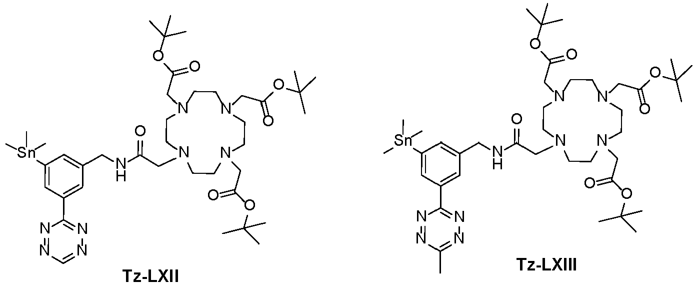

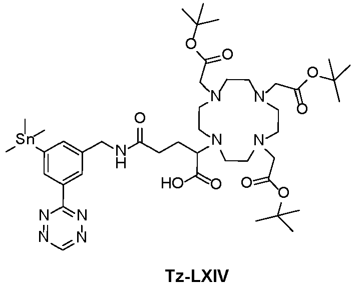

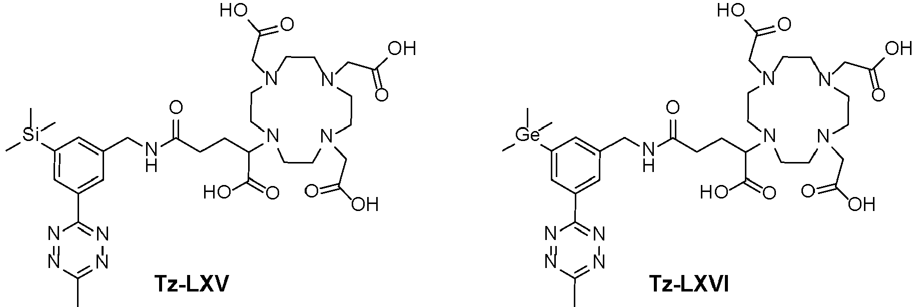

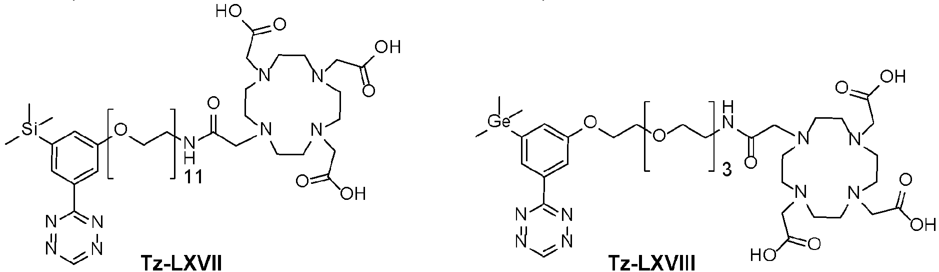

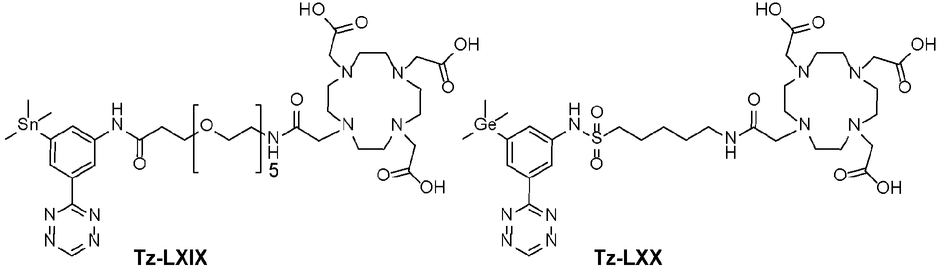

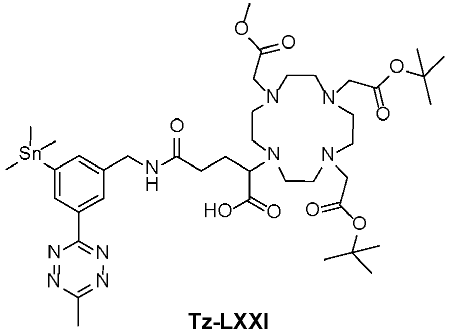

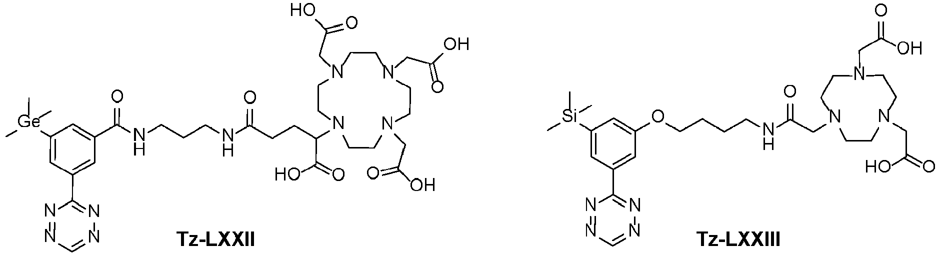

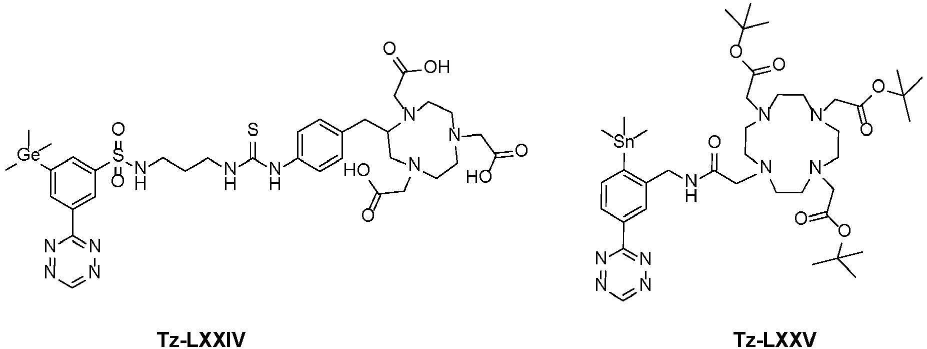

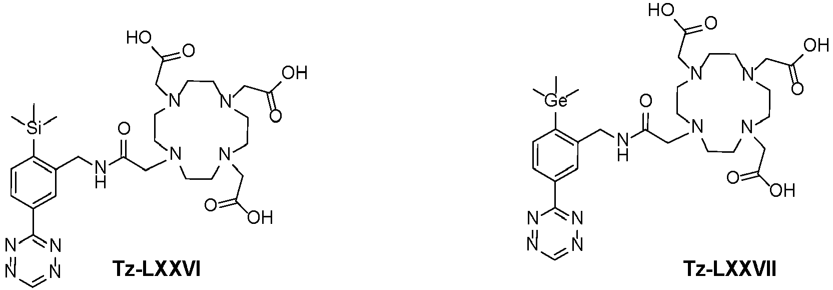

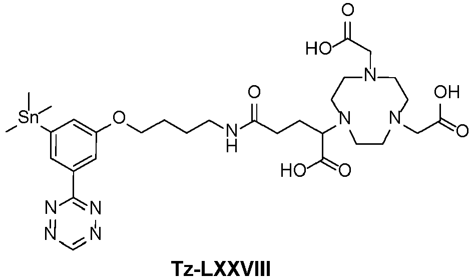

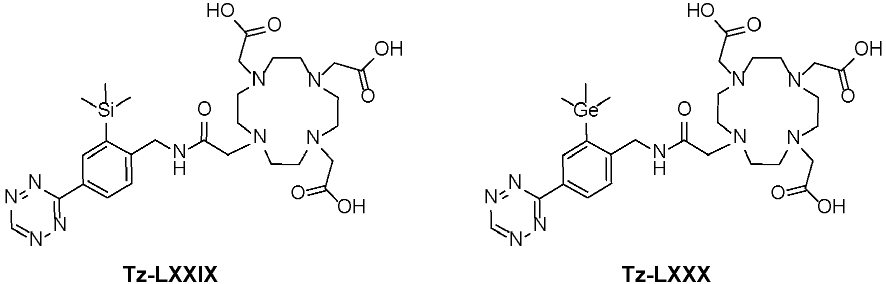

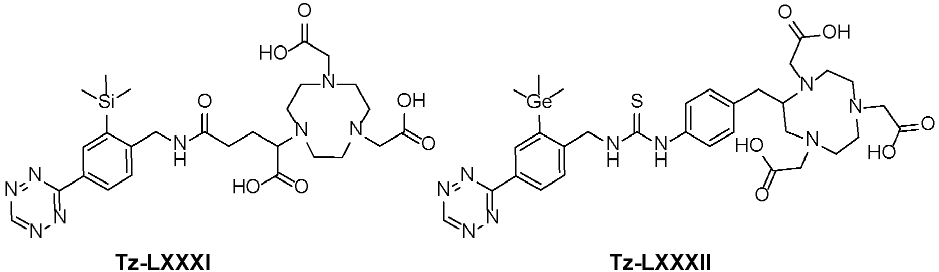

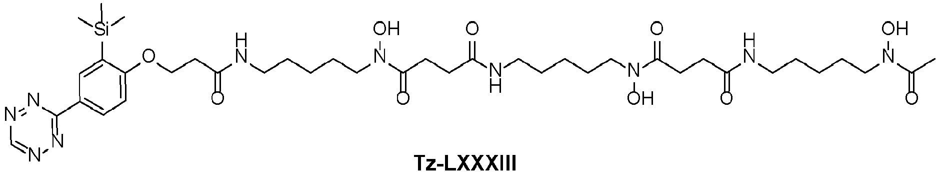

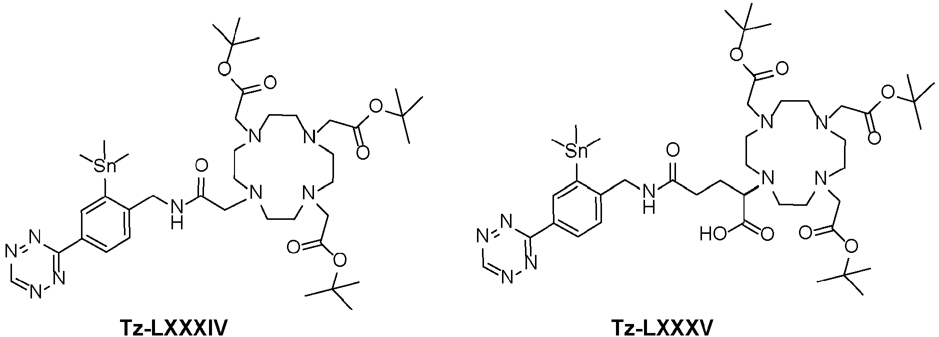

10. 1,2,4,5-tetrazine of formula (I): 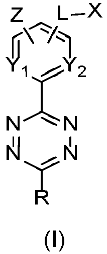 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)nNH-, - CONH(CH₂)ₙNH-, -NHCO(CH₂)ₙNH-, -(OCH₂CH₂)n(CH₂)ₘNH-,-(CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-,-SCH₂(OCH₂CH₂)n(CH₂)ₘNH-, -SO₂NHCH₂(OCH₂CH₂)n(CH₂)ₘNH-,-NHSO₂CH₂(OCH₂CH₂)ₙ(CH₂)ₘNH-, -CONHCH₂(OCH₂CH₂)ₙ(CH₂)ₘNH-,-NHCOCH₂CH₂(OCH₂CH₂)ₙ(CH₂)mNH-, -CH₂CH₂(OCH₂CH₂)(CH₂)ₘNH-,-OCH₂CH₂(OCH₂CH₂)ₙ(CH₂)ₘNH-, -SCH₂CH₂(OCH₂CH₂)ₙ(CH₂)ₘNH-,-SO₂NHCH₂CH₂(OCH₂CH₂)ₙ(CH₂)ₘNHCO-, -NHSO₂CH₂CH₂(OCH₂CH₂)ₙ(CH₂)ₘNHCO-, -CONHCH₂CH₂(OCH₂CH₂)ₙ(CH2)mNHCO-, -NHCOCH₂CH₂(OCH₂CH₂)ₙ(CH2)mNHCO-,
where n and m are independently selected from the group consisting of 0-25;
X is a chelator selected from:
1,4,7,10-tetraazacyclododecane-*N,N',N*',*N"-*tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((l2-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans-*cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is a radionuclide halogen selected from: ¹⁸F, ⁷⁶Br, ⁷⁷Br, ^{80m}Br, ¹²³I, ¹²⁴I,¹²⁵I, ¹²⁶I, ¹³¹I, ²¹⁰At,²¹¹At, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho ¹⁷⁵Lu, ¹⁸⁵Re, ²⁰³TI, ²⁰⁵TI, ²⁰⁶Pb, ²⁰⁷Pb, ²⁰⁸Pb, ²⁰⁹Bi;
for use in pre-targeting based therapy, diagnostics, imaging, or theranostics.

11. 1,2,4,5-tetrazine of formula (I): 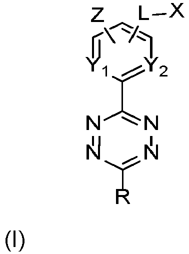 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)nNH-, - CONH(CH₂)ₙNH-, -NHCO(CH₂)ₙNH-, -(OCH₂CH₂)n(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)n(CH₂)ₘNH-, -SO₂NHCH₂(OCH₂CH₂)n(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)mNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH2)mNHCO-, - NHCOCH₂CH₂(OCH₂CH₂)ₙ(CH2)mNHCO-,
where n and m are independently selected from the group consisting of 0-25;
X is a chelator selected from:
1,4,7,10-tetraazacyclododecane-*N,N;N;N*"-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((12-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (tBu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N-*hydroxysuccinamide (DFO), diethylenetriaminepentaacetic acid (DTPA), *trans-*cyclohexyl-diethylenetriaminepentaacetic acid (CHX-DTPA), 1-oxa-4,7,10-triazacyclododecane-4,7,10-triacetic acid (OXO-Do3A), p-isothiocyanatobenzyl-DTPA (SCN-BZ-DTPA), 1-(p-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
Z is selected from: ¹⁹F, ⁷⁹Br, ⁸¹Br, ¹²⁷I, and
X is labeled with a radionuclide metal selected from: ⁴³Sc, ⁴⁴Sc, ⁴⁷Sc, ⁴⁵Ti, ⁵¹Cr, ⁵⁵Co, ^{58m}Co, ⁶⁰Cu, ⁶¹Cu, ⁶⁴Cu, ⁶⁷Cu, ⁶⁷Ga, ⁶⁸Ga, ⁷²As, ⁸⁹Sr, ⁸⁶Y, ⁹⁰Y, ⁸⁹Zr, ⁹⁶Zr, ⁹⁴Tc, ^{99m}Tc, ^{103m}Rh, ^{193m}Pt, ^{195m}Pt, ¹⁹⁸Pt, ¹¹¹In ¹¹³In, ^{114m}In, ^{115m}In ¹¹⁹Sb, ¹³⁵La, ¹³⁸La, ¹⁴⁹Tb, ¹⁵²Tb, ¹⁵⁹Tb, ¹⁶¹Tb, ¹⁶¹Dy, ¹⁶⁶Er, ¹⁶⁶Ho, ¹⁷⁷Lu, ¹⁸⁵Re, ²⁰¹TI, ²⁰³Pb, ²¹²Pb, ²⁰⁹Bi, ²¹²Bi, ²¹³Bi , ²²³Ra, ²²⁵Ac, ²²⁷Th, ²³²Th,
for use in pre-targeting based therapy, diagnostics, imaging, or theranostics.

12. Use of compound of formula (I): 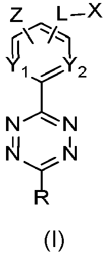 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)nNH-, - CONH(CH₂)ₙNH-, -NHCO(CH₂)ₙNH-, -(OCH₂CH₂)n(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)n(CH₂)ₘNH-, -SO₂NHCH₂(OCH₂CH₂)n(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)mNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH2)mNHCO-, - NHCOCH₂CH₂(OCH₂CH₂)ₙ(CH2)mNHCO-,
where n and m are independently selected from the group consisting of 0-25;
Z is selected from: Si(Me)₃, Si(Et)₃, Si(Pr)₃, Si(Bu)₃, Ge(Me)₃, Ge(Et)₃, Ge(Pr)₃, Ge(Bu)₃, Sn(Me)₃, Sn(Et)₃, Sn(Pr)₃, Sn(Bu)₃, and
X is unlabeled or labeled with a metal selected from: ⁴⁵Sc, ⁴⁶Ti, ⁴⁷Ti, ⁴⁸Ti, ⁴⁹Ti, ⁵⁰Ti, ⁵²Cr, ⁵³Cr, ⁵⁴Cr, ⁵⁴Fe, ⁵⁶Fe, ⁵⁷Fe, ⁵⁸Fe, ⁵⁹Co, ⁶³Cu, ⁶⁵Cu, ⁶⁹Ga, ⁷¹Ga, ⁷⁵As, ⁸⁴Sr, ⁸⁶Sr, ⁸⁷Sr, ⁸⁸Sr, ⁸⁹Y, ⁹⁰Zr, ⁹¹Zr, ⁹²Zr, ⁹⁴Zr, ¹⁰³Rh, ¹⁹²Pt, ¹⁹⁴Pt, ¹⁹⁵Pt, ¹⁹⁶Pt, ¹²¹Sb, ¹²³Sb, ¹³⁹La, ¹⁵⁴Gd, ¹⁵⁵Gd, ¹⁵⁶Gd, ¹⁵⁷Gd, ¹⁵⁸Gd, ¹⁶⁰Gd, ¹⁵⁶Dy, ¹⁵⁸Dy, ¹⁶⁰Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁷Er, ¹⁶⁸Er, ¹⁷⁰Er, ¹⁶⁵Ho ¹⁷⁵Lu, ¹⁸⁵Re, ²⁰³TI, ²⁰⁵TI, ²⁰⁶Pb, ²⁰⁷Pb, ²⁰⁸Pb, ²⁰⁹Bi,
as a precursor for radiolabeling.

13. Use according to claim 12 for providing a radiolabeled 1 ,2,4,5-tetrazine according to any of claims 2 to 4.

14. Use of a 1,2,4,5-tetrazine compound of formula (I): 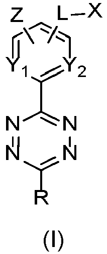 wherein:
R is H or Methyl;
Y₁ is -N- or -CH-;
Y₂ is -N- or -CH-;
L is selected from;
-(CH₂)ₙNH-, -O(CH₂)ₙNH-, -S(CH₂)ₙNH-, -SO₂NH(CH₂)ₙNH-, -NHSO₂(CH₂)nNH-, - CONH(CH₂)ₙNH-, -NHCO(CH₂)ₙNH-, -(OCH₂CH₂)n(CH₂)ₘNH-, - (CH₂)ₙ(OCH₂CH₂)ₘNH-, -O(CH₂)ₙ(OCH₂CH₂)ₘNH-, -S(CH₂)ₙ(OCH₂CH₂)ₘNH-, - SO₂NH(CH₂)ₙ(OCH₂CH₂)ₘNH-, -NHSO₂(CH₂)ₙ(OCH₂CH₂)ₘNH-, - CONH(CH₂)ₙ(OCH₂CH₂)ₘNH-, -NHCO(CH₂)ₙ(OCH₂CH₂)ₘNH-, - CH₂(OCH₂CH₂)(CH₂)ₘNH-, -OCH₂(OCH₂CH₂)ₙ(CH₂)ₘNH-, - SCH₂(OCH₂CH₂)n(CH₂)ₘNH-, -SO₂NHCH₂(OCH₂CH₂)n(CH₂)ₘNH-, - NHSO₂CH₂(OCH₂CH₂)ₙ(CH₂)ₘNH-, -CONHCH₂(OCH₂CH₂)ₙ(CH₂)ₘNH-, - NHCOCH₂CH₂(OCH₂CH₂)ₙ(CH₂)mNH-, -CH₂CH₂(OCH₂CH₂)(CH₂)ₘNH-, - OCH₂CH₂(OCH₂CH₂)ₙ(CH₂)ₘNH-, -SCH₂CH₂(OCH₂CH₂)ₙ(CH₂)ₘNH-, - SO₂NHCH₂CH₂(OCH₂CH₂)ₙ(CH₂)ₘNHCO-, - NHSO₂CH₂CH₂(OCH₂CH₂)ₙ(CH₂)ₘNHCO-, - CONHCH₂CH₂(OCH₂CH₂)ₙ(CH2)mNHCO-, - NHCOCH₂CH₂(OCH₂CH₂)ₙ(CH2)mNHCO-,
where n and m are independently selected from the group consisting of 0-25;
Z is a moiety selected from:
¹⁸F, ¹⁹F, ⁷⁶Br, ⁷⁷Br, ⁷⁹Br, ^{80g}r, ^{80m}Br, ⁸¹Br, ¹²³I, ¹²⁴I,¹²⁵I, ¹²⁶I, ¹²⁷I, ¹³¹I, ²¹⁰At or ²¹¹At;
X is a chelator selected from:
1,4,7,10-tetraazacyclododecane-*N,N',N',N*"-tetraacetic acid (DOTA), 2-(4,7,10-tris(2-(tert-butoxy)-2-oxoethyl)-1,4,7,10-tetraazacyclododecan-1-yl)acetic acid (tBu-DOTA), *N,N'*-bis(2-hydroxy-5-(carboxyethyl)benzyl)ethylenediamine *N,N'-*diacetic acid (HBED-CC), 14,7-triazacyclononane-1,4,7-triacetic acid (NOTA), 2,2',2"-(2-(4-((12-azaneyl)methanethioamido)benzyl)-1,4,7-triazonane-1,4,7-triyl)triacetic acid (benzyl-NOTA), 2-(4.7-bis(carboxymethyl)-1,4,7-triazonan-1-yl)pentanedioic acid (NODAGA), 2-(4,7,10-tris(carboxymethyl)-1,4,7,10-tetraazacyclododecan-1- yl)pentanedioic acid (DOTAGA), 2-(4,7,10-tris(2-(*tert-*butoxy)-2-oxoethyl)-1,4,7,10-tetraazacyclododecan-1-yl)pentanedioic acid (*t*Bu-DOTAGA), 14,7-triazacyclononane phosphinic acid (TRAP), 14,7-triazacyclononane-1-methyl(2-carboxyethyl)phosphinic acid-4,7-bis(methyl(2-hydroxymethyl)phosphinic acid (NOPO), 3,6,9,15-tetraazabicyclo9.3.1.pentadeca-1 (15),11,13-triene-3,6,9- triacetic acid (PCTA), *N*'-(5-acetyl (hydroxy)aminopentyl-*N*-(5-(4-(5- aminopentyl)(hydroxy)amino-4-oxobutanoyl)amino)pentyl-*N*-hydroxysuccinamide (DFO), diethylenetriaminepentaacetic acid (DTPA), *trans*-cyclohexyl-diethylenetriaminepentaacetic acid (CHX-DTPA), 1-oxa-4,7,10-triazacyclododecane-4,7,10-triacetic acid (OXO-Do3A), *p*-isothiocyanatobenzyl-DTPA (SCN-BZ-DTPA), 1-(*p*-isothiocyanatobenzyl)-3-methyl-DTPA (1B3M), 2-(p-isothiocyanatobenzyl)-4-methyl-DTPA (1M3B), and 1-(2)-methyl-4-isocyanatobenzyl-DTPA (MX-DTPA);
and wherein X is optionally labeled with a metal selected from:
⁴³Sc, ⁴⁴Sc, ⁴⁵Sc, ⁴⁷Sc, ⁴⁵Ti, ⁴⁶Ti, ⁴⁷Ti, ⁴⁸Ti, ⁴⁹Ti, ⁵⁰Ti, ⁵¹Cr, ⁵²Cr, ⁵³Cr, ⁵⁴Cr, ⁵⁴Fe, ⁵⁶Fe, ⁵⁷Fe, ⁵⁸Fe, ⁵⁵Co, ^{58m}Co, ⁵⁹Co, ⁶⁰Cu, ⁶¹Cu, ⁶³Cu, ⁶⁴Cu, ⁶⁵Cu, ⁶⁷Cu, ⁶⁷Ga, ⁶⁸Ga, ⁶⁹Ga, ⁷¹Ga, ⁷²As, ⁷⁵As, ⁸⁴Sr, ⁸⁶Sr, ⁸⁷Sr, ⁸⁸Sr, ⁸⁹Sr, ⁸⁶Y, ⁸⁹Y, ⁹⁰Y, ⁸⁹Zr, ⁹⁰Zr, ⁹¹Zr, ⁹²Zr, ⁹⁴Zr, ⁹⁶Zr ,⁹⁴Tc , ^{99m}Tc, ¹⁰³Rh, ^{103m}Rh, ^{193m}Pt, ^{195m}Pt, ¹⁹²Pt, ¹⁹⁴Pt, ¹⁹⁵Pt, ¹⁹⁶Pt, ¹⁹⁸Pt, ¹¹¹In, ¹¹³In, ^{114m}In, ^{115m}In, ¹¹⁹Sb, ¹²¹Sb, ¹²³Sb, ¹³⁵La, ¹³⁸La, ¹³⁹La, ¹⁴⁹Tb, ¹⁵²Tb, ¹⁵⁹Tb, ¹⁶¹Tb, ¹⁵⁴Gd, ¹⁵⁵Gd, ¹⁵⁶Gd, ¹⁵⁷Gd, ¹⁵⁸Gd, ¹⁶⁰Gd ¹⁵⁶Dy, ¹⁵⁸Dy, ¹⁶⁰Dy, ¹⁶¹Dy, ¹⁶²Dy, ¹⁶³Dy, ¹⁶⁴Dy, ¹⁶⁵Dy, ¹⁶²Er, ¹⁶⁴Er, ¹⁶⁵Er, ¹⁶⁶Er, ¹⁶⁷Er, ¹⁶⁸Er, ¹⁷⁰Er, ¹⁶⁵Ho, ¹⁶⁶Ho ¹⁷⁵Lu, ¹⁷⁷Lu, ¹⁸⁵Re, ¹⁸⁶Re, ¹⁸⁸Re, ²⁰¹TI, ²⁰³TI, ²⁰⁵TI, ²⁰³Pb, ²⁰⁶Pb, ²⁰⁷Pb, ²⁰⁸Pb, ²¹²Pb, ²⁰⁹Bi, ²¹²Bi, ²¹³Bi, ²²³Ra, ²²⁵Ac, ²²⁷Th, ²³²Th.
as reagent in tetrazine ligation.
